# EUROPEAN PATENT SPECIFICATION

(11) **EP 1 199 565 B1**
(45) Date of publication and mention of the grant of the patent: **08.10.2008**
(21) Application number: 00937283.0
(22) Date of filing: 16.06.2000
(51) Int. Cl.: G01N 33/53, A61K 45/00, A61K 39/395, C12N 15/06, C12N 15/12, C07K 16/28

(54) **DIAGNOSTICS AND REMEDIES FOR DISEASES WITH PARTICIPATION OF MACROCYTES/MACROPHAGES**
DIAGNOSTIKUM UND HEILMITTEL FÜR KRANKHEITEN UNTER TEILNAHME VON MAKROZYTEN/MAKROPHAGEN
MOYENS DE DIAGNOSTIC ET DE TRAITEMENT DE MALADIES UTILISANT DES MACROCYTES/MACROPHAGES

(30) Priority: 17.06.1999 JP 17170999
(43) Date of publication of application: 24.04.2002
(60) Divisional application: 07024517.0
(73) Proprietor: KYOWA HAKKO KOGYO CO., LTD., Chiyoda-ku, Tokyo 100-8185 (JP)
(72) Inventor: SHITARA, Kenya, Kyowa Hakko Koggo Co.Ltd, Machida-shi, Tokyo 194-8533 (JP); SHIBUYA, Masabumi, Kawaguchi-shi, Saitama 333-0866 (JP)
(74) Representative: Kinzebach, Werner
(86) International application number: PCT/JP2000/003957
(87) International publication number: WO 2000/079275

(56) References cited:
- EP-A- 0 882 799
- WO-A1-98/22626
- CLAUSS M ET AL: "THE VASCULAR ENDOTHELIAL GROWTH FACTOR RECEPTOR FLT-1 MEDIATES BIOLOGICAL ACTIVITIES" JOURNAL OF BIOLOGICAL CHEMISTRY, AMERICAN SOCIETY OF BIOLOGICAL CHEMISTS, BALTIMORE, MD, US, vol. 271, no. 30, 26 July 1996 (1996-07-26), pages 17629-17634, XP000918913 ISSN: 0021-9258
- SAWANO A ET AL: "THE PHOSPHORYLATED 1169 - TYROSINE CONTAINING REGION OF FLT-1 KINASE (VEGFR-1) IS A MAJOR BINDING SITE FOR PLCGAMMA" BIOCHEMICAL AND BIOPHYSICAL RESEARCH COMMUNICATIONS, ACADEMIC PRESS INC. ORLANDO, FL, US, vol. 238, 1997, pages 487-491, XP002949764 ISSN: 0006-291X
- BARLEON BERNHARD ET AL: "Migration of human monocytes in response to vascular endothelial growth factor (VEGF) is mediated via the VEGF receptor flt-1" BLOOD, vol. 87, no. 8, 1996, pages 3336-3343, XP002297912 ISSN: 0006-4971
- HIRATSUKA SACHIE ET AL: "Flt-1 lacking the tyrosine kinase domain is sufficient for normal development and angiogenesis in mice" PROCEEDINGS OF THE NATIONAL ACADEMY OF SCIENCES OF THE UNITED STATES OF AMERICA, vol. 95, no. 16, 4 August 1998 (1998-08-04), pages 9349-9354, XP002297913 ISSN: 0027-8424
- CLAUSS M: "Functions of the VEGF Receptor-1 (FLT-1) in the Vasculature." TRENDS IN CARDIOVASCULAR MEDICINE. AUG 1998, vol. 8, no. 6, August 1998 (1998-08), pages 241-245, XP002298088 ISSN: 1050-1738
- SAWANO ASAKO ET AL: "Flt-1, vascular endothelial growth factor receptor 1, is a novel cell surface marker for the lineage of monocyte-macrophages in humans" BLOOD, vol. 97, no. 3, 1 February 2001 (2001-02-01), pages 785-791, XP002297914 ISSN: 0006-4971
- DATABASE BIOSIS, ACC. NO. 199799785097 SAWANO ASAKO ET AL.: 'The phosporylated 1169-tyrosine contaaining region of F1t-1 kinase is a major binding site for PLC-gamma' & J. BIOCHEM. BIOPHYS. RES. COMMUN., vol. 238, no. 2, 1997, pages 487 - 491
- DATABASE BIOSIS, ACC. NO. 199799777801 WALTENBERGER J. ET AL.: 'Vascular endothelial growth factor stimulates monocyte activation and chemotaxis via the receptor tyrosine kinase F1t-1' & EUROPEAN HEART J., vol. 18, 1997, page 4
- DATABASE BIOSIS, ACC. NO. 199699135536 CLAUSS MATTHIAS ET AL.: 'The vascular endothelial growth factor receptor F1t-1 mediate biological activities' & J. BIOL. CHEM., vol. 271, no. 30, 1996, pages 17629 - 17634
- DATABASE BIOSIS, ACC. NO. 199698771458 BARLEON BERNHARD ET AL.: 'Migration of human monocytes in response to vascular endothelial growth factor is mediated via the VEGF receptor F1t-1' & BLOOD, vol. 87, no. 8, 1996, pages 3336 - 3343
- BROWN ET AL: "Overexpression of vascular permeability factor (VPF/VEGF) and its endothelial cell receptors in delayed hypersensitivity skin reactions" JOURNAL OF IMMUNOLOGY, vol. 154, no. 6, 1995, pages 2801-2807, XP008073887

## Description

### TECHNICAL FIELD

The present invention relates to uses of anti-Flt-1 antibodies.

### BACKGROUND ART

Angiogenesis plays an important role in the formation of the circulatory organ system and construction of many tissues in the embryonal period of vertebrates and also closely relates to the formation of corpus luteum during sexual cycle, transient proliferation of uterine endometrium, formation of placenta and the like in mature individuals (females). With regard to pathological states, angiogenesis closely relates to the proliferation or metastasis of solid tumors and formation or acceleration of morbid states of diabetic retinopathy and rheumatoid arthritis (J. Biol. Chem., 267, 10931 (1992)). Vascular permeability factor (VPF)/vascular endothelial growth factor (VEGF) are known as the most important factors involved in angiogenesis induction, such as during the developmental and pathological conditions described above (Advances in Cancer Research, 67, 281 (1995)).

Among diseases accompanied by angiogenesis, it has been reported that VEGF closely relates to the proliferation or metastasis of solid tumors and formation of morbid states of diabetic retinopathy and rheumatoid arthritis (Advances in Cancer Research, 67, 281 (1995); Endocrine Reviews, 18, 184 (1997)).

Two kinds of human VEGF receptor have been reported, namely a first receptor Flt-1 (fms-like tyrosine kinase) (Oncogene, 5, 519 (1990); Science, 255, 989 (1992)) and a second receptor KDR (kinase insert domain-containing receptor) (WO 92/14748, Priority Feb. 22, 1991; Biochem. Biophys. Res. Comm., 187, 1579 (1992)), which belong to the receptor-type tyrosine kinase family. A mouse-type homologue of the human-type VEGF receptor KDR is named Flk-1 (Proc. Natl. Acad. Science, USA, 88, 9026 (1991); WO 94/11499, Priority Nov. 13, 1992; Cell, 72, 835 (19,93)). In the Flt-1 and KDR/Flk-1, the extracellular domain is composed of seven immunoglobulin-like domains, and the intracellular domain is composed of a membrane protein of 180 to 200 kilodalton in molecular weight which has a tyrosine kinase region. VEGF specifically binds to Flt-1 and KDR/Flk-1 at KD values of 20 pM and 75 pM, respectively. EP-A 0 882 799 describes anti-Flt-1 antibodies which inhibit binding to VEGF. It has been reported that Flt-1 and KDR/Flk-1 are expressed specifically in vascular endothelial cells (Proc. Natl. Acad. Science, USA, 90, 7533 (1993); Proc. Natl. Acad. Science, USA, 90, 8915 (1993)).

With regard to the expression of Flt-1 in various diseases, it has been reported that the expression of flt-1 mRNA is more highly increased in tumor vascular endothelial cells of human glioblastoma tissues (Nature, 359, 845 (1992)) and tumor vascular endothelial cells of human digestive organ cancer tissues (Cancer Research, 53, 4727 (1993)) than vascular endothelial cells in normal tissues. In addition, it has been reported that the expression of flt-1 mRNA is also observed in vascular endothelial cells of joints of patients with rheumatoid arthritis by *in situ* hybridization (Journal Experimental Medicine, 180, 341 (1994)). These results strongly suggest that a VEGF-VEGF receptor-Flt-1 system plays an important role in tumor angiogenesis. Although it has been reported that VEGF binds to Flt-1 and the intracellular domain is auto-phosphorylated (Science, 255, 989 (1992)), detailed functions are unknown. However, flt-1 knock out mouse in which the flt-1 gene had been destroyed, the animal died after a fetal age of 8.5 to 9.5 days due to abnormal blood vessel construction caused by abnormal morphology of vascular endothelial cells during the blood island formation in the early stage of development and subsequent angiogenesis, it has been assumed that Flt-1 functions in a manner essential for the hollow tube formation of vascular endothelial cells in angiogenesis *(*Nature, 376, 66 (1995)).

VEGF is a growth factor having high selectivity for blood endothelial cells, and it has been reported that it has activity of acting upon mouse monocyte to thereby accelerate migration of the monocyte (Journal of Experimental Medicine, 172, 1535 (1990)). Furthermore, it has been reported that VEGF also shows activity of accelerating migration of a monocyte prepared from human peripheral blood (Blood, 81, 2767 (1993)), and that the migration accelerating activity is inhibited by a VEGF neutralizing antibody (Blood, 87, 3336 (1996)). When expression of VEGF receptors in the monocyte prepared from human peripheral blood was analyzed at mRNA level by Northern blotting or RT-PCR method, Flt-1 was expressed but KDR was not expressed, so that it is considered that Flt-1 is a receptor related to migration of a monocyte **(**Journal of Biological Chemistry, 221, 17629 (1996)).

It has been reported that expression of Flt-1 at mRNA level increased when human monocyte was activated with lipopolysaccharide (Blood, 87, 3336 (1996)).

Monocytes occupy 5 to 6% of the total leukocytes in peripheral blood of a healthy person. Monocytes in blood are large cells having a diameter of 10 to 20 µm, and circulate in peripheral blood by its active migrating ability. On the other hand, they migrate into various organs and tissues through a blood vessel wall and become macrophage, which is larger and more differentiated than monocyte, therein. Physiologically, monocytes and macrophages have a defense function by phagocytizing and digesting microorganisms, disrupted cells and the like, and are generalized as a monocyte-macrophage group due to their common origin, differentiation, cellular structure, abundant lysosome and phagocytosis (Macrophage, edited by Toru Tokunaga, Kodansha (1986), New Special Pathology, edited by Kokichi Kikuchi and Takashi Yoshiki, Nanzando (1996)). The monocyte-macrophage group infiltrates into the affected part of diseases, such as inflammatory disease, delayed type hypersensitivity, malignant tumor, arteriosclerosis and the like, and relates to the development of these diseases (Macrophage, edited by Toru Tokunaga, Kodansha (1986), New Special Pathology, edited by Kokichi Kikuchi and Takashi Yoshiki, Nanzando (1996)).

In rheumatoid arthritis which is an inflammatory disease, synovial membrane which is a lesion is thickened and macrophage is increased in comparison with healthy people. Synovial membrane macrophage in rheumatic synovial membrane (hereinafter, the synovial membrane macrophage in rheumatic synovial membrane is referred to as "RA synovial membrane macrophage") is mainly derived from migrated monocytes in blood. The RA synovial membrane macrophage produces various types of cytokine, proteolytic enzymes, active oxygen, nitrogen monoxide, prostaglandin E2 and the like to thereby induce activation and disorder of peripheral cells and causes denaturation of extracellular matrix. Examples of the inflammation-accelerating cytokine produced by RA synovial membrane macrophage include interleukin-1β (hereinafter, interleukin is referred to as "IL"), IL-6, IL-8, IL-10, IL-15, MCP-1 (monocyte chemotactic protein), MIP-1α (macrophage inflammatory protein), TNF-α (tumor necrosis factor), VEGF (vascular endothelial growth factor), PDGF (platelet-derived growth factor) and the like (Clinical Immunology, 30, 214 (1998)).

In arteriosclerosis, macrophages are present in a focal region from the initial stage of lesion formation, and relate to the development of the morbid state. The monocytes in blood bind to blood endothelial cells in the initial stage of lesion region, migrate into a blood vessel wall under the endothelium, and differentiate into macrophages. The macrophages gathered on the blood vessel wall become foam cells by incorporating denatured LDL into the cells and thereby form an arteriosclerotic lesion. The macrophage in the lesion produces various cytokines, such as VEGF, PDGF, FGF (fibroblast growth factor), IL-1 and the like, which accelerate the morbid state (Recent Medicine, 53, 88 (1998)).

As described above, it has been reported that the VEGF receptor Flt-1 is expressed at mRNA level in monocytes prepared from peripheral blood, and that migration of the monocytes is accelerated VEGF-dependently. However, it its not clear that Flt-1 is expressed on the cell surface of monocytes and the monocytes migrate VEGF-dependently via Flt-1. Also, it is not known about a substance which inhibits the signal transduction via Flt-1 capable of inhibiting VEGP-dependent migration of monocytes.

### DISCLOSURE OF THE INVENTION

The invention relates to the use of an anti-Flt-1 antibody in the manufacture of a medicament for treating delayed type hypersensitivity.

The invention further relates to the use of an anti-Flt-1 antibody in the manufacture of an agent for diagnosing a delayed type hypersensitivity wherein the diagnosis comprises detecting monocyte-macrophage infiltrates into the affected part of the living body.

The present inventors have found for the first time that an antibody (hereinafter sometimes referred to as "anti-Flt-1 antibody") for a VEGF receptor Flt-1 (hereinafter sometimes referred sometimes to as "Flt-1") reacts with a monocyte in healthy human peripheral blood, that the anti-Flt-1 antibody does not react with a blood stem cell in human cord blood but the anti-Flt-1 antibody reacts with a monocyte differentiated from a blood stem cell, and that the anti-Flt-1 antibody inhibits migration of a monocyte induced by VEGF, thus accomplishing the present invention.

The present invention relates to the following (1) to (10) :
(1) The use of an antibody against a human VEGF receptor Flt-1 for providing an agent for diagnosing a delayed type hypersensitivity wherein the diagnosis composes detecting monocyte-macrophage infiltrates into the affected part of the living body.
(2) The use of an antibody against a human VEGF receptor Flt-1 which inhibits signal transduction via a human VEGF receptor Flt-1 for providing an agent for therapy of a delayed type hypersensitivity.
(3) The use according to (1) or (2), wherein the antibody against a human VEGF receptor Flt-1 is a polyclonal antibody or a monoclonal antibody.
(4) The use According to (3), wherein the monoclonal antibody is an antibody selected from an antibody produced by a hybridoms, a humanized antibody and antibody fragments thereof.
(5) The use according to (4), wherein the antibody produced by a hybridoma, is an antibody selected from the group consisting of KM1730, KM1731, KM1732, KM1748, and KM1750.
(6) The use according to (4), wherein the humanized antibody is an antibody selected from a human chimeric antibody and a human complementarity determining region-grafted antibody.
(7) The use according to (6), wherein the human chimeric antibody is KM2532 or KM2550.
(8) The use according to (6), wherein the human complementarity determining region-grafted antibody is an antibody selected from the group consisting of KM8550, KM8551, KM8552, KM8553, KM8554, and KM8555.
(9) The use according to (4), wherein the antibody fragment is an antibody fragment selected from the group consisting of Fab, Fab', F(ab')₂, a single chain antibody, a disulfide-stabilized Fv and a peptide comprising a complementarity determining region.
(10) The use according to any one of (2) to (9), wherein the antibody is an antibody fused with a radioisotope, a protein or a low molecular weight agent by a chemical or genetic engineering means.

Thus, the present invention describes a diagnostic agent for delayed type hypersensitivity (a disease related to a monocyte and/or a macrophage), which comprises, as an active ingredient, an anti-Flt-1 antibody (which is a substance having binding activity to a human VEGF receptor Flt-1).

The present invention thus also describes a therapeutic agent for delayed type hypersensitivity, which comprises, as an active ingredient, an anti-Flt-1 antibody which inhibits signal transduction via a human VEGF receptor Flt-1.

The antibody which inhibits signal transduction via a human VEGF receptor Flt-1 is an antibody which inhibits binding of VEGF to an Flt-1 receptor or an antibody which inhibits signal transduction from an Flt-1 receptor.

A hematopoietic stem cell is a cell having both of the pluripotency by which it can be differentiated into all blood cells and the self regenerative ability to replicate itself. The hematopoietic stem cell differentiates into a bone marrow stem cell or a lymphoid stem cell. From the bone marrow stem cell, cells of lineages of erythrocyte, lineages of granulocyte, lineages of monocyte-macrophage, lineages of eosinophil, lineages of basophil and lineages of bone marrow megakaryocyte-platelet are differentiated. Lineages of T cell and lineages of B cell are differentiated from the lymphoid stem cell. Regarding the monocytes and macrophages, monocytes occupy 5 to 6% of the total leukocytes in peripheral blood of a healthy person. Monocytes in blood are large cells having a diameter of 10 to 20 µm, and circulate in peripheral blood by its active migrating ability. On the other hand, they migrate into various organs and tissues through a blood vessel wall, and become macrophages, which are larger and more differentiated than monocytes, therein. Physiologically, both monocytes and macrophages have a defense function by phagocytizing and digesting microorganisms, disrupted cells and the like in the living body, and are generalized as a monocyte-macrophage group due to their common origin, differentiation, cellular structure, abundant lysosome and phagocytosis (Macrophage, edited by Toru Tokunaga, Kodansha (1986), New Special Pathology, edited by Kokichi Kikuchi and Takashi Yoshiki, Nanzando (1996)). The monocyte-macrophage group infiltrates into the affected part of diseases, such as inflammatory disease, delayed type hypersensitivity, malignant tumor, arteriosclerosis and the like, and relates to the development of these diseases (Macrophage, edited by Toru Tokunaga, Kodansha (1986), New Special Pathology, edited by Kokichi Kikuchi and Takashi Yoshiki, Nanzando (1996)).

The anti-Flt-1 antibody used in the present invention can also be used for detecting differentiation from a hematopoietic stem cell to a monocyte and/or a macrophage as it has binding activity to a protein which is not expressed in a hematopoietic stem cell but expressed in a monocyte and/ or a macrophage, i.e., Flt-1. An antibody fragment or an analogue of an anti-Flt-1 antibody can also be used.

As the diagnosing agent for delayed type hypersensitivity, any anti-Flt 1 antibody can be used, as long as it has binding activity to Flt-1.

As the therapeutic agent for delayed type hypersensitivity, any anti-fit 1 antibody can be used, as long as it inhibits signal transduction via Flt-1. As the antibody which inhibits signal transduction, any antibody can be used, as long as it inhibits the function of Flt-1. Examples include an antibody which inhibits binding of human VEGF to Flt-1, an antibody which inhibits signal transduction from Flt-1.

Examples of the substance which inhibits binding of human VEGF to Flt-1 include a neutralizing antibody for Flt-1, an antibody fragment thereof, and an analogue thereof.

According to the present invention a delayed type hypersensitivity is a disease related to a monocyte and/or a macrophage. Examples of the delayed type hypersensitivity include contact dermatitis and the like.

The anti-Flt-1 antibody used in the present invention can be obtained as a polyclonal or monoclonal antibody using conventionally known means (Antibodies-A Laboratory Manual, Cold Spring Harbor Laboratory, 1988 (hereinafter referred to as *"Antibodies-A Laboratory Manua*l")).

As the anti-Flt-1 antibody used in the present invention, any of polyclonal and monoclonal antibodies can be used, but a monoclonal antibody is preferred.

The monoclonal antibody includes an antibody produced by a hybridoma, and a gene recombinant antibody produced by a transformant transformed with an expression vector containing an antibody gene, antibody fragments, single chain antibodies and disulfide stabilized antibodies.

Examples of the antibody produced by a hybridoma include an anti-human VEGF receptor Flt-1 monoclonal antibody obtained by preparing a human VEGF receptor Flt-1 protein as an antigen, inducing a plasma cell having the antigen specificity from an animal immunized with the antigen, fusing the cell with a inyeloma cell to thereby prepare a hybridoma, culturing the hybridoma or administering the hybridoma to a mammal to cause ascites tumor, and then separating and purifying the culture or ascitic fluid.

The gene recombinant antibody used in the present invention includes those in which the above monoclonal antibody of the present invention is modified using gene recombination techniques. Examples include a humanized antibody and the like. Among these antibodies, an antibody having low antigenicity and prolonged blood half-life is preferred as a therapeutic agent.

The humanized antibody used in the present invention includes a human chimeric antibody and a human complementary determining region (hereinafter referred to as "CDR")-grafted antibody.

The human chimeric antibody means an antibody containing an antibody variable region heavy chain (hereinafter referred to as "VH"), a variable region light chain (hereinafter referred to as "VL") of an animal other than human, a constant region heavy chain (hereinafter referred to as "CH") of a human antibody, and a constant region light chain (hereinafter referred to as "CL") of a human antibody.

The human chimeric antibody used in the present invention can be produced by preparing cDNAs encoding VH and VL from a hybridoma capable of producing a monoclonal antibody which binds to Flt-1, inserting each of them into an expression vector for animal cell containing genes encoding human antibody CH and human antibody CL to thereby construct a human chimeric antibody expression vector, and then introducing the vector into an animal cell to thereby express the antibody.

A human CDR-grafted antibody is an antibody in which CDR sequences of VH and VL of a human antibody are replaced by respective CDR sequences of an antibody of an animal other than human.

The human CDR-grafted antibody used in the present invention can be produced by constructing cDNAs encoding V regions in which the VH and VL CDR sequences of an optional human antibody are replaced by the VH and VL CDR sequences of an antibody of an animal other than human, respectively, which binds to the human VEGF receptor Flt-1, inserting each of them into an expression vector for animal cell containing a genes encoding human antibody CH and human antibody CL to thereby construct a human CDR-grafted antibody expression vector, and then introducing the vector into an animal cell to express the antibody.

The human antibody moiety of the human chimeric antibody and human CDR-grafted antibody used in the present invention may belong to any immunoglobulin (Ig) class, but an IgG-type is preferred, and any C region of immunoglobulin belonging to the IgG-type (e.g., IgG1, IgG2, IgG3, IgG4 or the like) can be used.

An antibody fragment used in the present invention includes Fab (abbreviation of "fragment of antigen binding"), Fab', F(ab')₂, a single chain antibody (single chain Fv; hereinafter referred to as "scFv"), a disulfide stabilized antibody (disulfide stabilized Fv; hereinafter referred to as "dsFv"), and a peptide comprising CDR, which are antibody fragments having the affinity for Flt-1.

An Fab is a fragment of about 50,000 molecular weight having an antigen binding activity, which is constituted by about half of the N-terminal side of H chain obtained by digesting the upper side of two disulfide bonds cross-linking two H chains in the hinge regions of IgG with papain, and the L chain.

The Fab used in the present invention can be obtained by treating the anti-human VEGF receptor Flt-1 antibody with papain. Alternatively, the Fab can be produced by inserting DNA encoding a Fab fragment of the anti-human VEGF receptor Flt-1 antibody into an expression vector for animal cells, and then expressing the antibody by introducing the vector into an animal cell.

An Fab' is a fragment of about 50,000 in molecular weight having an antigen binding activity, which is obtained by cutting the disulfide bonds between the hinges of the above F(ab')₂.

The Fab' used in the present invention can be obtained by treating the anti-Flt-1 antibody with a reducing agent, dithiothreitol. Alternatively, the Fab' can be produced by inserting DNA encoding a Fab' fragment of the anti-Flt-1 antibody into an expression vector for animal cells, and then expressing the fragment by introducing the vector into an animal cell.

An F(ab')₂ is a fragment of about 100,000 molecular weight having an antigen binding activity, which is constituted by two bound Fab regions at the hinge part which are obtained by digesting the lower side of two disulfide bonds in the hinge regions of IgG with trypsin.

The F(ab')₂ used in the present invention can be obtained by treating the anti-Flt-1 antibody with trypsin. Alternatively, the F(ab')₂ can be produced by inserting DNA encoding a F(ab')₂ fragment of the anti-Flt-1 antibody into an expression vector for animal cells, and then expressing the fragment by introducing the vector into an animal cell.

A single chain antibody (scFv) is a VH-P-VL or VL-P-VH polypeptide obtained by linking a VH chain and a VL chain using an appropriate peptide linker (hereinafter referred to as "P"). As the VH and VL contained in the scFv used in the present invention, any anti-Flt-1 monoclonal antibody can be used.

The single chain antibody used in the present invention can be produced by obtaining cDNA encoding VH and VL from a hybridoma capable of producing an antibody which binds to the Flt-1, constructing a single chain antibody expression vector therefrom and then expressing it by introducing the vector into *Escherichia coli,* yeast, or an animal cell.

A disulfide stabilized antibody (dsfv) is an antibody in which polypeptides prepared by replacing an amino acid residue in each of VH and VL with a cysteine residue are linked via a disulfide bond. The amino acid residue to be replaced by a cysteine residue can be selected based on the three-dimensional structure estimation of the antibody in accordance with the method shown by Reiter et al. (Protein Engineering, 1, 697 (1994)). As the VH or VL contained in the disulfide stabilized antibody used in the present invention, any anti-Flt-1 monoclonal antibody can be used.

The disulfide stabilized antibody used in the present invention can be produced by obtaining cDNA encoding VH and VL from a hybridoma capable of producing an antibody which reacts with the human VEGF receptor Flt-1, inserting the cDNA into an appropriate expression vector and then expressing the antibody by introducing the expression vector into *Escherichia coli,* yeast, or an animal cell.

A peptide comprising CDR is constituted by including at least one region of the H chain or L chain CDR. Plural CDRs can be bound directly or via an appropriate peptide linker.

The peptide comprising CDR of the present invention can be produced by obtaining cDNAs encoding the VH and VL of an antibody which specifically reacts with a human VEGF receptor Flt-1, constructing DNA encoding CDR, inserting the DNA into an expression vector for prokaryote or an expression vector for eukaryote, and then by introducing the expression vector into a prokaryote or eukaryote to express the peptide comprising CDR.

The peptide comprising CDR can also be produced by a chemical synthesis method, such as Fmoc method (fluorenylmethoxycarbonyl method), tBoc method (t-butyloxycarbonyl method), or the like.

The antibody of the present invention include antibody analogues in which a radioisotope, a protein or a low molecular weight compound is fused to the antibody produced by a hybridoma, the humanized antibody, the human antibody or antibody fragments thereof of the present invention.

As the antibody analogues, a fused antibody in which a radioisotope, a protein, a low molecular weight agent or the like is fused to the above antibody or antibody fragment used in the present invention by a chemical or genetic engineering means can also be used.

The fused antibody used in the present invention can be produced by chemically fusing an antibody which binds to the human VEGF receptor Flt-1 to a radioisotope, a protein, a low molecular weight agent or the like. Also, the antibody fused to a protein can be produced by linking cDNA encoding the antibody to cDNA encoding the protein, inserting it into an appropriate expression vector and then expressing it by introducing the expression vector into *Escherichia coli,* yeast, or an animal cell.

### 1. Production method of anti-human VEGF receptor Flt-1 monoclonal antibody

### (1) Preparation of antigen

Examples of the antigen necessary for preparing the anti-human VEGF receptor Flt-1 monoclonal antibody include cells in which the human VEGF receptor Flt-1 has been expressed on the cell surface or a cell membrane fraction thereof, a soluble human VEGF receptor Flt-1 protein having an extracellular region of different amino acid length, a fusion protein of the protein with the Fc region of the antibody, and the like.

The cells capable of expressing the human VEGF receptor Flt-1 on the cell surface include NIH3T3-Flt-1 cell (Cell Growth & Differentiation, 1, 213 (1996)). As a method for expressing it as a soluble human VEGF receptor Flt-1 protein having different extracellular regions or a fused protein of the protein with the Fc region of the antibody, the full length or a partial fragment of the human VEGF receptor Flt-1 can be produced inside the cells or in a culture supernatant as such or as a fusion protein, by constructing a recombinant vector in which cDNA encoding the full length or a partial fragment of the human VEGF receptor Flt-1 (Cell Growth & Differentiation, 1, 213 (1996)) is inserted into the downstream site of the promoter of an appropriate vector, introducing the vector into a host cell, and then culturing the thus obtained human VEGF receptor Flt-1-expressing cells in an appropriate medium. Alternatively, it can also be prepared by synthesizing a polypeptide having a partial sequence of the above protein using an amino acid synthesizer.

Any host such as bacteria, yeast, animal cells, insect cells and the like can be used, so long as they can express the gene of interest. Examples of the bacteria include bacteria belonging to the genera *Escherichia, Bacillus* and the like such as *Escherichia coli, Bacillus subtilis* and the like. Examples of the yeast include *Saccharomyces cerevisiae, Schizosaccharomyces pombe* and the like. Examples of the animal cells include namalwa cell as a human cell, COS cell as a monkey cell, CHO cell as a Chinese hamster cell and the like. Examples of the insect cells include Sf9 and Sf21 (manufactured by Pharmingen), High Five (manufactured by Invitrogen) and the like.

As the vector for introducing the DNA of the present invention, any vector can be used, so long as the DNA can be introduced therein and expressed in a host cell.

When a bacterium such as *Escherichia coli* is used as the host, the expression vector may be preferably constructed with a promoter, a ribosome binding sequence, the DNA of the present invention, a transcription termination sequence and optionally a promoter controlling sequence. Examples include commercially available pGEX (manufactured by Pharmacia), pET System (manufactured by Novagen) and the like.

As the method for introducing the recombinant vector into a bacterium, any one of the known methods for introducing DNA into bacteria, such as a method in which a calcium ion is used (Proc. Natl. Acad. Sci., USA, 69, 2110 (1972)), a protoplast method (Japanese Published Unexamined Patent Application No. 2483942/88) and the like, can be used.

When yeast is used as the host, YEp13 (ATCC 37115), YEp24 (ATCC 37051), YCp50 (ATCC 37419) or the like may be used as the expression vector.

As the method for introducing the recombinant vector into yeast, any one of the known methods for introducing DNA into yeast, such as an electroporation method (Methods. Enzymol., 194, 182-187 (1990)), a spheroplast method (Proc. Natl. Acad. Sci., USA, 84, 1929 (1978)), a lithium acetate method (J. Bacteriol., 153, 163 (1983)) and the like, can be used.

When animal cells are used as the host, pAGE107 (Japanese Published Unexamined Patent Application No. 22979/91; Cytotechnology, 3, 133 (1990)), pAGE103 (J. Biochem., 101, 1307 (1987)) and the like, may be used as the expression vector.

Any promoter can be used, so long as it functions in an animal cell. Examples include the promoter of IE (immediate early) gene of cytomegalovirus (CMV), the SV40 promoter, the metallothionein promoter and the like. Also; the enhancer of the IE gene of human CMV may be used together with the promoter.

As the method for the introducing the recombinant vector into an animal cell, any one of the known methods for introducing DNA into an animal cell, such as an electroporation method (Cytotechnology, 3, 133 (1990)), a calcium phosphate method (Japanese Published Unexamined Patent Application No. 227075/90), a lipofection method (Proc. Natl. Acad. Sci., USA, 84, 7413 (1987)) and the like, can be used.

When insect cells are used as the host, the protein can be expressed by the known method described, for example, in Current Protocols in Molecular Biology, Supplement 1-34 and *Baculovirus Expression Vectors,* A laboratory manual. That is, a protein-expressing insect cell is obtained by simultaneously introducing the recombinant gene-introducing vector and baculovirus described below to obtain a recombinant virus in the insect cell culture supernatant and then infecting the insect cell with the thus obtained recombinant virus.

The gene introducing vector includes pVL1392, pVL1393, pBlueBacIII (all manufactured by Invitrogen) and the like.

The baculovirus includes *Autographa californica* nuclear polyhedrosis virus with which insects of the family *Barathra* are infected.

The method for simultaneously introducing the above recombinant gene introducing vector and the above baculovirus into an insect cell for the preparation of the recombinant virus includes a calcium phosphate method (Japanese Published Unexamined Patent. Application No. 227075/90), a lipofection method (Proc. Natl. Acad. Sci., USA, 84, 7413 (1987)) and the like.

Alternatively, the protein can be produced by preparing a recombinant baculovirus making use of BaculoGold Starter Kit manufactured by Pharmigen or the like and then infecting the above insect cell such as Sf9, Sf21, High Five or the like with the recombinant virus (Bio/Technology, 6, 47 (1988)).

As the gene expression method, secretion production, fusion protein expression and the like have been developed in addition to the direct expression, and any of such methods can be used. For example, it can be carried out in accordance with the method described in (Molecular Cloning, 2nd edition, Cold Spring Harbor Lab. Press, New York (1989); hereinafter referred to as *"*Molecular Cloning, 2nd edition").

The full length or a partial fragment of the human VEGF receptor Flt-1 can be produced as such or as a fusion protein thereof, by culturing the thus obtained transformant in a medium to produce and accumulate the protein of the present invention in the resulting culture, and then recovering the product from the culture.

The above method for culturing the transformant in a medium is carried out in accordance with a usual method which is used in culturing the host cell.

As the medium for use in culturing the transformant obtained using a microorganism such as *Escherichia coli,* yeast or the like as the host, any of a natural medium and a synthetic medium can be used, so long as it contains a carbon source, a nitrogen source, an inorganic salt and the like which can be assimilated by the microorganism and the transformant is efficiently cultured therein *(*Molecular Cloning, 2nd edition). Culturing is carried out generally under aerobic conditions such as shaking culture, submerged agitation aeration culture or the like at 15 to 40°C for 16 to 96 hours. During culturing, the pH is maintained at 3.0 to 9.0. The pH is adjusted using an inorganic or organic acid, an alkali solution, urea, calcium carbonate, ammonia and the like. Antibiotics such as ampicillin, tetracycline and the like can be optionally added to the medium during culturing.

As the medium for use in culturing a transformant obtained using animal cells as the host, usually used RPMI 1640 medium, Eagle's MEM medium or any one of these media further supplemented with fetal calf serum can be used. Culturing is carried out generally at 35 to 37°C for 3 to 7 days in the presence of 5% CO₂, and antibiotics such as kanamycin, penicillin and the like can be optionally added to the medium during culturing.

As the medium for use in culturing a transformant obtained using insect cells as the host, usually used TNM-FH medium (manufactured by Pharmingen), Sf900IISFM (manufactured by Life Technologies), ExCell400 or ExCell405 (both manufactured by JRH Biosciences) or the like can be used. Culturing is carried out generally at 25 to 30°C for 1 to 4 days, and antibiotics such as gentamicin and the like can be optionally added to the medium during culturing.

Also, if it is possible in culturing animal cells and insect cells, it is preferred to use a serum-free medium in order to facilitate purification of the full length or a partial fragment of the human VEGF receptor Flt-1 as such or as a fusion protein.

When the complete length or a partial fragment of the human VEGF receptor Flt-1 is accumulated inside the host cells as such or as a fusion protein, the cells after completion of culturing are centrifuged, suspended in an aqueous buffer and disrupted using an ultrasonic oscillator method, a French press method or the like, and then the protein is recovered from the supernatant after centrifugation.

Also, when an insoluble material is formed inside the cells, the insoluble material is solubilized using a protein denaturing agent and then a higher-order structure of the protein can be formed by diluting or dialyzing the protein in or against a solution which contains no protein denaturing agent or contains the protein denaturing agent in such a low concentration that the protein is not denatured.

When the full length or a partial fragment of the human VEGF receptor Flt-1 is secreted outside the cells as such or as a fusion protein, the expressed protein can be recovered from the culture supernatant.

The isolation and purification can be carried out by employing separation means, such as solvent extraction, fractional precipitation with organic solvents, salting out, dialysis, centrifugation, ultrafiltration, ion exchange chromatography, gel filtration chromatography, hydrophobic chromatography, affinity chromatography, reverse phase chromatography, crystallization, electrophoresis and the like, alone or in combination.

Also, the polypeptide having a partial sequence can also be produced by a chemical synthesis method such as a Fmoc method (fluorenylmethoxycarbonyl method), a tBoc method (t-butyloxycarbonyl method) or the like. It can also be produced using a peptide synthesizer available from Sowa Boeki (manufactured by Advanced chemTech), Perkin-Elmer Japan (manufactured by Perkin-Elmer, USA), Aroka (manufactured by Protein Technology Instrument, USA), Krabow (manufactured by Synthecell-vega, USA), Japan PerSeptive Ltd. (manufactured by PerSeptive, USA), Shimadzu Corp. or like.

Moreover, the polypeptide having a partial sequence of the above protein can also be synthesized using an amino acid synthesizer.

### (2) Immunization of animal and preparation of antibody-producing cell

An animal is immunized using the thus obtained protein as the antigen. As the immunization method, the antigen may be administered as such to an animal subcutaneously, intravenously or intraperitoneally, but it is preferred to administer the antigen by binding a carrier protein having a high antigenicity thereto or together with an appropriate adjuvant.

Examples of the carrier protein include Fissurellidae hemocyanin, keyhole limpet hemocyanin, bovine serum albumin, bovine thyroglobulin and the like. Examples of the adjuvant include complete Freund's adjuvant, a combination of aluminum hydroxide with pertussis vaccine and the like.

The animal to be immunized includes non-human mammals such as rabbit, goat, mouse, rat, hamster and the like.

After the first administration, the antigen may be administered every 1 to 2 weeks for 3 to 10 times. A dose of the antigen is preferably from 50 to 100 µg per animal. A blood sample is collected from the fundus of the eye or caudal vein of the immunized animal 3 to 7 days after each administration and tested, for example, by an enzyme immunoassay (*Enzyme-linked Immunosorbent Assay (ELISA),* published by Igaku Shoin, (1976)) on the reactivity of the serum with the antigen.

A non-human animal showing a sufficient antibody titer in its serum is used as the supply source of antibody-producing cells.

Three to seven days after final administration of the antigen, lymphocytes are collected from the immunized animal in accordance with the known method (*Antibodies, A Laboratory Manual*) to fuse the lymphocytes with myeloma cells.

The polyclonal antibody can be prepared by separating and purifying the serum.

The monoclonal antibody can be obtained by preparing a hybridoma through fusion of an antibody-producing cell with a myeloma cells derived from a non-human mammal, culturing the hybridoma, or administering the cells to a mammal to cause ascites tumor, and then separating and purifying the culture or ascitic fluid.

The antibody-producing cell is collected from the spleen cells, lymph node, peripheral blood or the like of the antigen-administered non-human mammal.

### (3) Preparation of myeloma cell

As the myeloma cell, any myeloma cell capable of growing in vitro can be used. Examples include established cells obtained from mouse, such as 8-azaguanine-resistant mouse (BALB/c) myeloma cell lines P3-X63Ag8-U1 (P3-U1) (Eur. J. Immunol, 6, 511 (1976)), SP2/O-Ag14 (SP-2) (Nature, 276, 269 (1978)), P3-X63-Ag8653 (653) (J. Immunol., 123, 1548 (1979)), P3-X63-Ag8 (X63) *(*Nature, 256, 495 (1975)) and the like. These cell lines are cultured and subcultured in accordance with the known method (*Antibodies, A Laboratory Manual*) and 2×10⁷ or more of the cells are secured until cell fusion.

### (4) Cell fusion and selection of monoclonal antibody

The antibody-producing cells and myeloma cells obtained in the above are washed, mixed with a cell aggregating medium such as polyethylene glycol-1000 (PEG-1000) or the like to fuse the cells and then suspended in a medium. The cells are washed with MEM medium, PBS (1.83 g of disodium hydrogen phosphate 0.21 g of potassium dihydrogen phosphate, 7.65 g of sodium chloride, 1 liter of distilled water, pH 7.2) or the like. In order to obtain the fused cells of interest selectively, HAT medium {normal medium (a medium prepared by adding glutamine (1.5 mM), 2-mercaptoethanol (5×10⁻⁵ M), gentamicin (10 µg/ml) and fetal calf serum (FCS) (10%, manufactured by CSL) to RPMI-1640 medium) further supplemented with hypoxanthine (10⁻⁴ M), thymidine (1.5×10⁻⁵ M) and aminopterin (4×10⁻⁷ M)} is used as the medium for suspending the fused cells.

After culturing, a portion of the culture supernatant is sampled and tested by an enzyme immunoassay to select a sample which reacts with the antigen protein but does not react with non-antigen proteins. Thereafter, cloning is carried out by limiting dilution analysis, and a hybridoma which shows a stable and high antibody titer by the following enzyme immunoassay is selected as the hybridoma capable of producing the monoclonal antibody.

### Enzyme immunoassay:

The antigen protein, a cell expressing the antigen protein or the like is coated on a plate and allowed to react with a hybridoma culture supernatant or a purified antibody obtained by the above method as a first antibody.

After the first antibody reaction, the plate is washed and a second antibody is added.

The second antibody is an antibody which can recognize the first antibody and is labeled with biotin, an enzyme, a chemiluminescent substance, a radioactive compound or the like. Specifically, when mouse is used in the preparation of hybridoma, an antibody which can recognize mouse immunoglobulin is used as the second antibody.

After the reaction, a reaction suitable for the second antibody-labeled substance is carried out to select a hybridoma capable of producing a monoclonal antibody which specifically reacts with the antibody.

Specific examples of the anti-Flt-1 monoclonal antibody include monoclonal antibody KM1732 belonging to the IgG1 subclass which is produced by hybridoma KM1732 (FERM BP-5698), monoclonal antibody KM1730 belonging to the IgG2b subclass which is produced by hybridoma KM1730 (FERM BP-5697), monoclonal antibody KM1731 belonging to the IgG2a subclass which is produced by hybridoma KM1731 (FERM BP-5718), monoclonal antibody KM1748 belonging to the IgG2b subclass which is produced by hybridoma KM1748 (FERM BP-5699), monoclonal antibody KM1750 which belonging to the IgG2b subclass which is produced by hybridoma KM1750 (FERM BP-5700) described in WO 98/22616 and the like.

Among the above-described monoclonal antibodies produced by hybridomas, KM1732, KM1748 and KM1750 have neutralizing activity against Flt-1, and are therefore preferred as an inhibitor of migration of a monocyte and/or a macrophage or as a therapeutic agent for diseases related to a monocyte and/or a macrophage.

### (5) Preparation of monoclonal antibody

The monoclonal antibody can be prepared by separating and purifying it from a culture obtained by culturing a hybridoma cell or from an ascitic fluid of an 8- to 10-week-old mouse or nude mouse in which ascites tumor was induced by treating the animal with pristane (by intraperitoneal administration of 0.5 ml of 2,6,10,14-tetramethylpentadecane (pristane), followed by feeding for 2 weeks) and then intraperitoneally administering a monoclonal antibody-producing hybridoma cell.

The method for separating and purifying the monoclonal antibody includes centrifugation, salting out with 40 to 50% saturated ammonium sulfate, caprylic acid precipitation and chromatography using a DEAE-Sepharose column, an anion exchange column, a protein A or G column, a gel filtration column or the like, which may be used alone or in combination. By this method, a purified monoclonal antibody can be obtained by recovering an IgG or IgM fraction.

The subclass of the purified monoclonal antibody can be determined using a monoclonal antibody typing kit or the like. The amount of the protein can be calculated by the Lowry method or based on the absorbance at 280 nm.

The subclass of the antibody is an isotype in a class. Examples in mouse include IgG1, IgG2a, IgG2b and IgG3, and examples in human include IgG1, IgG2, IgG3 and IgG4. Particularly since mouse IgG1 and IgG2a and human IgGl-types have complement-dependent cytotoxic activity (hereinafter "CDS activity") and antibody-dependent cellular cytotoxic activity (hereinafter "ADCC activity"), they are useful in applying to medical treatments.

### 2. Production method of gene recombinant antibody (1)

### Production method of anti-human VEGF receptor Flt-1 humanized antibody:

### (1) Construction of humanized antibody expression vector

A vector for humanized antibody expression necessary for producing a humanized antibody from an antibody of an animal other than human is constructed. The humanized antibody expression vector is an expression vector for animal cell into which genes encoding the human antibody C regions CH and CL have been incorporated, which is constructed by inserting respective genes encoding CH and CL of a human antibody into an expression vector for animal cell.

As the human antibody C region, the C region of an optional human antibody can be used, such as Cγ1 and Cγ4 in human antibody H chain and Cκ in human antibody L chain. As the gene encoding a human antibody C region, a chromosomal DNA composed of exon and intron can be used, and a cDNA can also be used. As the expression vector for animal cell, any vector can be used, so long as a gene encoding a human antibody C region can be incorporated and expressed.

Examples include pAGE107 (Cytotechnology, 3, 133 (1990)), pAGE103 (J. Biochem., 101, 1307 (1987)), pHSG274 (Gene, 21, 223 (1984)), pKCR (Proc. Natl. Acad. Sci. USA, 78, 1527 (1981)), pSG1βd2-4 (Cytotechnology, 4, 173 (1990)) and the like. The promoter and enhancer used in the expression vector for animal cell include, SV40 early promoter and enhancer (J. Biochem., 101, 1307 (1987)), Moloney mouse leukemia virus LTR promoter and enhancer (Biochem. Biophys. Res. Comun., 149, 960 (1987)), immunoglobulin H chain promoter *(*Cell, 41, 479 (1985)) and enhancer (Cell, 33, 717 (1983)) and the like.

As the humanized antibody expression vector, any of a type in which the antibody H chain and L chain are present on separate vectors and a type in which they are present on the same vector (tandem-type) can be used, but a tandem-type humanized antibody expression vector is preferred from the viewpoint that the humanized antibody expression vector can be easily constructed, its introduction into an animal cell is easy, and expression amounts of the antibody H chain and L chain in the animal cell can be balanced (J. Immunol. Methods, 167, 271 (1994)).

### (2) Preparation of cDNA encoding VH and VL of antibody of animal other than human

cDNA encoding the VH and VL of an antibody of an animal other than human, such as a mouse anti-human VEGF receptor Flt-1 monoclonal antibody, is obtained, for example, as follows.

cDNA is synthesized by extracting mRNA from an anti-human VEGF receptor Flt-1 monoclonal antibody-producing cell, such as a mouse anti-human VEGF receptor Flt-1 antibody-producing hybridoma or the like. The thus synthesized cDNA is inserted into a phage vector, plasmid vector or the like to prepare a cDNA library. A recombinant phage or recombinant plasmid containing cDNA encoding VH and a recombinant phage or recombinant plasmid containing cDNA encoding VL are isolated from the library using part of C region and part of V region, respectively, of an antibody of an animal other than human, such as a mouse antibody, as the probe. Full nucleotide sequences of the antibody VH and VL of interest on the recombinant phage or recombinant plasmid are determined, and full amino acid sequences of the VH and VL are deduced from the nucleotide sequences.

### (3) Construction of human chimeric antibody expression vector

A human chimeric antibody expression vector can be constructed by inserting cDNA encoding VH and VL of an antibody of an animal other than human into the upstream of a gene encoding CH and CL of a human antibody on the humanized antibody expression vector constructed in the above 2(1). For example, a human chimeric antibody expression vector can be produced by arranging a restriction enzyme recognizing sequence for use in the cloning of cDNA encoding VH and VL of an antibody of an animal other than human, in advance upstream of a gene encoding CH and CL of a chimeric antibody expression vector, and inserting cDNA encoding V regions of an antibody of an animal other than human into this cloning site via a synthetic DNA described below. The synthetic DNA contains a 3'-terminal nucleotide sequence of the V region of an antibody of an animal other than human and a 5'-terminal nucleotide sequence of the C region of a human antibody and can be produced using a DNA synthesizer in such a manner that it has appropriate restriction enzyme sites on both termini.

### (4) Identification of CDR sequence of antibody of animal other than human

The VH and VL which form the antigen binding region of an antibody are composed of four frame work regions (hereinafter referred to as "FR region") whose sequences are relatively preserved and three complementarity determining regions (CDR) which connects them and is rich in sequence changes (*Sequences of Proteins of Immunological Interest,* US Dep. Health and Human Services, 1991: hereinafter referred to as *"Sequences of Proteins of Immunological Interest"*). Each CDR amino acid sequence (CDR sequence) can be identified by comparing it with amino acid sequences of V regions of known antibodies (*Sequences of Proteins of Immunological Interest*).

### (5) Construction of cDNA encoding V regions of human CDR-grafted antibody

The cDNA encoding the VH and VL of human CDR-grafted antibody can be obtained as follows.

First, the amino acid sequence of FR of the V region of a human antibody for grafting CDR of the V region of an antibody of an animal other than human is selected for both VH and VL. As the amino acid sequence of FR of the V region of a human antibody, any amino acid sequence can be used, so long as it is an amino acid sequence of FR of the V region derived from a human antibody.

For example, amino acid sequences of FR of V regions of human antibodies registered at Protein Data Bank and an amino acid sequence common in each subgroup of FR of V regions of human antibodies (*Sequences of Proteins of Immunological Interest*) can be cited, but in order to create a human CDR-grafted antibody having sufficient activity, it is preferred that it has a high homology, preferably a homology of 65% or more, with the intended amino acid sequence of V regions of an antibody of an animal other than human. Next, a DNA sequence encoding the thus selected amino acid sequence of FR of the V region of human antibody is linked to a DNA sequence encoding the intended amino acid sequence of CDR of the V region of an antibody of an animal other than human, and DNA sequences encoding amino acid sequences of VH and VL, respectively are designed. In order to obtain the DNA sequences designed for constructing a CDR-grafted antibody variable region gene, several synthetic DNA fragments are designed for each chain in such a manner that the complete DNA sequence is covered, and a polymerase chain reaction (hereinafter referred to as "PCR") is carried out using these fragments. Based on the reaction efficiency in the PCR and the length of DNA which can be synthesized, preferably 6 synthetic DNA fragments are designed for each chain. After the reaction, the thus amplified fragments are subcloned in an appropriate vector and their nucleotide sequences are determined to thereby obtain a plasmid containing cDNA encoding the amino acid sequence of the V region of each chain of the human CDR-grafted antibody of the interest. Alternatively, cDNA encoding the amino acid sequence of the V region of each chain of the human CDR-grafted antibody of the interest can also be constructed by synthesizing complete sense and antisense sequences using a synthetic DNA fragment of about 100 bases, and annealing and linking them.

### (6) Modification of amino acid sequence of V region of human CDR-grafted antibody

It is known that when only the CDR of the v region of an antibody of an animal other than human of interest is simply grafted between FR's of the V region of a human antibody, the activity of the human CDR-grafted antibody is reduced in comparison with the activity of the original antibody of an animal other than human (BIO/TECHNOLOGY, 9, 266 (1991)). Accordingly, attempts have been made to increase the activity by a method in which, among amino acid sequences of FR's of V regions of a human antibody, an amino acid residue directly relating to the binding to the antigen, an amino acid residue interacting with an amino acid residue of CDR or an amino acid residue having a possibility of relating to the maintenance of three-dimensional structure of the antibody or the like is changed to an amino acid residue found in the original antibody of an animal other than human. In order to identify these amino acid residues efficiently, construction and analysis of the three-dimensional structure of antibodies have been carried out using an X-ray crystal analysis, computer modeling or the like. However, since a method for producing a human CDR-grafted antibody applicable to every antibody has not yet been established, it is necessary under the present situation to carry out various trial and error investigations on individual antibodies.

The selected amino acid sequence of the FR of V region of a human antibody can be modified by carrying out the PCR described in the above 2(5) using various mutation-introduced primers. The amplified fragments after the PCR are subcloned in an appropriate vector and their nucleotide sequences are determined to obtain a vector containing cDNA introduced with the mutation of interest (hereinafter referred to as "amino acid sequence-modified vector").

Also, in the modification of an amino acid sequence within a narrow range, it can be carried out by a PCR mutation introducing method using mutation-introduced primers consisting of 20 to 35 nucleotides. Specifically, a sense mutated primer and antisense mutated primer consisting of 20 to 35 nucleotides containing a DNA sequence encoding the amino acid residues after modification are synthesized, and two step PCR is carried out using a plasmid containing cDNA encoding the V region amino acid sequence to be modified as the template. The finally amplified fragments are subcloned in an appropriate vector and their nucleotide sequences are determined to obtain an amino acid sequence-modified vector containing cDNA introduced with the mutation of interest.

### (7) Construction of human CDR-grafted antibody expression vector

A human CDR-grafted antibody expression vector can be constructed by inserting the cDNA encoding human CDR-grafted antibody VH and VL obtained in the above 2(5) and 2(6) into the upstream of the gene encoding human antibody CH and CL in the humanized antibody expression vector of the above 2(1). For example, appropriate restriction enzyme recognizing sequences are introduced into the of 5'-end and 3'-end termini of the synthetic DNA in PCR for constructing cDNA encoding amino acid sequences of the VH and VL of human CDR-grafted antibody so that they can be inserted into the upstream of the gene encoding the desired human antibody C region in such a manner that they can be expressed in a suitable form.

### (8) Transient expression and activity evaluation of humanized antibody

In order to evaluate the activity of various humanized antibodies efficiently, their activities can be measured by carrying out transient expression of humanized antibodies (Methods in Nucleic Acids Res., CRC Press, p. 283, 1991), by introducing the human chimeric antibody expression vector of the above 2(3) and the human CDR-grafted antibody expression vector of the above 2(7) or a modified vector thereof into COS-7 cell (ATCC CRL 1651).

The method for introducing an expression vector into COS-7 cell includes a DEAE-dextran method (Methods in Nucleic Acids Res., CRC Press, p. 283, 1991), a lipofection method (Proc. Natl. Acad. Sci., 84, 7413 (1987)) and the like.

After introduction of the vector, the activity of the humanized antibody in a culture supernatant can be measured by the enzyme immunoassay (ELISA) described in the above 1(4) or the like.

### (9) Stable expression and activity evaluation of humanized antibody

A transformant capable of producing a humanized antibody stably can be obtained by introducing the human chimeric antibody expression vector of the above 2(3) and the human CDR-grafted antibody expression vector of the above 2(7) into an appropriate host cell.

The method for introducing an expression vector into a host cell includes an electroporation method (Japanese Published Unexamined Patent Application No. 257891/90, Cytotechnology, 3. 133 (1990)) and the like.

As the host cell into which a humanized antibody expression vector is introduced, any cell can be used, so long as it is a host cell which can express the humanized antibody. Examples include mouse SP2/0-Ag14 cell (ATCC CRL 1581), mouse P3X63-Ag8.653 cell (ATCC CRL 1580), CHO cell from which dihydrofolate reductase gene (hereinafter referred to as "DHFR gene") has been deleted (Proc. Natl. Acad. Sci. USA, 11, 4216 (1980)), rat YB2/3HL.P2.G11.16Ag.20 cell (ATCC CRL 1662, hereinafter referred to as "YB2/0 cell") and the like.

After introduction of the vector, a transformant capable of stably producing the humanized antibody is selected using RPMI1640 medium containing G418 and FCS in accordance with the method disclosed in Japanese Published Unexamined Patent Application No. 257891/90. By culturing the thus obtained transformant in a medium, the humanized antibody can be produced and accumulated in the culture. The activity of the humanized antibody in the culture is measured by the method described in the above 1(4) or the like. Also, the produced amount of the humanized antibody can be increased in accordance with the method disclosed in Japanese Published Unexamined Patent Application No. 257891/90, making use of a DHFR gene amplification system.

The humanized antibody can be purified from a culture supernatant of the transformant using a protein A column (Antibodies, A Laboratory Manual, Cold Spring Harbor Laboratory, Chapter 8, 1988; hereinafter referred to as "*Antibodies*")*.* In addition to this, a purification method generally used for a protein can also be used. For example, its purification can be carried out through the combination of gel filtration, ion exchange chromatography, ultrafiltration and the like. The molecular weight of the H chain, L chain or entire antibody molecule of the purified humanized antibody is measured by polyacrylamide gel electrophoresis (SDS-PAGE) (Nature, 227, 680 (1970)), Western blotting method (Antibodies, Chapter 12, 1988) or the like.

The reactivity of the purified humanized antibody or the inhibition activity of the humanized antibody against VEGF can be measured by the method described in the above 1(4) or the like.

Examples of the humanized antibody against Flt-1 include human chimeric antibodies KM2532, 2550 and the like, and human CDR-grafted antibodies KM8550, KM8551, KM8552, KM8553, KM8554, KM8555, and the like, which were produced in Reference Examples.

As *Escherichia coli* into which a plasmid containing DNA encoding the variable region of the above antibody has been introduced, examples for human chimeric antibodies include *Escherichia coli* XL-1 Blue MRF'/KM1732HA2 (FERM BP-6354) containing the H chain variable region DNA of KM2532, *Escherichia coli* XL-1. Blue MRF'/KM1732L2-1 (FERM BP-6352) containing the L chain variable region DNA of KM2532, *Escherichia coli* XL-1 Blue MRF'/KM175OH2-1 (FERM BP-6353) containing the H chain variable region DNA of KM2550, *Escherichia coli* XL-1 Blue MRF'/KM1750L3-1 (FERM BP-6355) containing the L chain variable region DNA of KM2550, and the like.

As *Escherichia coli* into which a plasmid containing DNA encoding the variable region of the above antibody has been introduced, examples for human CDR-grafted antibodies include *Escherichia coli* DH5α/phKM1750HV0 (FERM BP-6719) containing the H chain variable region DNA of KM8550, KM8551 and KM8554, *Escherichia coli* DH5α/phHKM1750HV3 (FERM BP-6720) containing the H chain variable region DNA of KM8552, KM8553 and KM8555, *Escherichia coli* (FERM BP-6716) containing the L chain variable region DNA of KM8550 and KM8552, *Escherichia coli* (FERM BP-6717) containing the L chain variable region DNA of KM8551 and KM8553, *Escherichia coli* (FERM BP-6718) containing the H chain variable region DNA of KM8554 and KM8555, and the like.

### 3. Production method of gene recombinant antibody (2)

### (1) Method for producing antibody fragments Fab, Fab' and F(ab')₂

An antibody fragment is formed by treating the above antibody with an enzyme. The enzyme includes papain, trypsin and the like.

Alternatively, Fab, Fab' or F(ab')₂ can be produced by inserting DNA encoding the Fab, Fab' or F(ab')₂ fragment of the human VEGF receptor Flt-1 antibody into an expression vector for animal cell and introducing the vector into an animal cell to thereby express it.

The thus formed antibody fragment can be purified using the combination of gel filtration, ion exchange or affinity chromatography, ultrafiltration and the like. The molecular weight of the purified Fab, Fab' or F(ab')₂ is measured by polyacrylamide gel electrophoresis (SDS-PAGE) (Nature, 227, 680 (1970)), Western blotting (Antibodies, Chapter 12, 1988) or the like.

The reactivity of the purified Fab, Fab' or F(ab')₂ or the inhibition activity of the Fab, Fab' or F(ab')₂ against VEGF can be measured by the method described in the above 1(4) or the like.

### (2) Method for producing anti-human VEGF receptor Flt-1 single chain antibody

An expression vector for a single chain antibody of an antibody of an animal other than human or a single chain antibody of a human CDR-grafted antibody can be constructed by inserting the cDNA encoding VH and VL of the antibody of an animal other than human or the human CDR-grafted antibody described in the above 2(2), 2(5) and 2(6) into an expression vector for single chain antibody. As the expression vector for single chain antibody, any vector can be used, so long as the cDNA encoding VH and VL of the antibody of an animal other than human or the human CDR-grafted antibody can be incorporated and expressed.

Examples include pAGE107 (Cytotechnology, 3, 133 (1990)), pAGE103 (J. Biochem., 101, 1307 (1987)), pHSG274 (Gene, 27, 223 (1984)), pKCR (Proc. Natl. Acad. Sci. USA, 78, 1527 (1981)), pSGβd2-4 (Cytotechnology, 4, 173 (1990)) and the like. As the host for expressing the single chain antibody, a suitable cell can be selected from *E. coli,* yeast, animal cells and the like, but in that case, it is necessary to select an expression vector suitable for each host. Also, the single chain antibody can be secreted into the outside of the cell, transported into the periplasmic region or retained inside the cell, by inserting cDNA encoding an appropriate signal peptide into the expression vector.

A single chain antibody expression vector into which cDNA encoding the single chain antibody of interest is inserted can be constructed by inserting cDNA encoding a single chain antibody composed of VH-P-VL or VL-P-VH (P is a peptide linker) into the downstream of an appropriate promoter and a signal peptide region of the thus selected expression vector.

The cDNA encoding the single chain antibody can be obtained by linking cDNA encoding VH to cDNA encoding VL using a synthetic DNA fragment encoding a peptide linker having appropriate restriction enzyme recognizing sequences on both termini. It is important to optimize the linker peptide in such a manner that its addition does not obstruct binding of VH and VL to the antigen, and, for example, a peptide shown by Pantoliano et al. (Biochemistry, 30, 10117 (1991)) or a modified product thereof can be used.

### (3) Method for producing anti-human VEGF receptor Flt-1 disulfide stabilized antibody

A disulfide stabilized antibody can be produced by substituting a DNA sequence corresponding to an amino acid residue at an appropriate position of the cDNA encoding VH and VL of the antibody of an animal other than human or the cDNA encoding VH and VL of the human CDR-grafted antibody with a DNA sequence corresponding to a cysteine residue, followed by expression and purification, and then forming a disulfide bond. The amino acid residue can be substituted with a cysteine residue by the mutation introducing method using the PCR described in the above 2(5).

A disulfide stabilized antibody H chain expression vector and a disulfide stabilized antibody L chain expression vector can be constructed by inserting the thus obtained cDNA encoding the modified VH and modified VL into an appropriate expression vector. As the vector for disulfide stabilized antibody expression, any vector can be used, so long as it can incorporate and express the cDNA encoding the modified VH and modified VL. Example includes pAGE107 (Cytotechnology, 3, 133 (1990)), pAGE103 (J. Biochem., 101, 1307 (1987)), pHSG274 (Gene, 27, 223 (1984)), pKCR (Proc. Natl. Acad. Sci. USA, 78, 1527 (1981)), pSG1βd2-4 *(*Cytotechnology, 4, 173 (1990)) and the like. As the host for expressing the disulfide stabilized antibody L chain expression vector and disulfide stabilized antibody H chain expression vector used for forming a disulfide stabilized antibody, an appropriate cell can be selected from *E. coli,* yeast, animal cells and the like, but in that case, it is necessary to select an expression vector suitable for each host.

Also, the disulfide stabilized antibody can be secreted into the outside of the cell, transported into the periplasmic region or retained inside the cell, by inserting cDNA encoding an appropriate signal peptide into the expression vector.

### (4) Expression and activity evaluation of various antibodies

A transformant capable of producing the antibody fragment, single chain antibody, disulfide stabilized antibody H chain or disulfide stabilized antibody L chain of interest can be obtained by introducing the antibody fragment expression vector, single chain antibody expression vector, disulfide stabilized antibody H chain expression vector or disulfide stabilized antibody L chain expression vector constructed in the above (1) to (3) into a host cell by an electroporation method (Japanese Published Unexamined Patent Application No. 257891/90, Cytotechnology, 3, 133 (1990)) or the like. After introduction of the expression vector, expression of the antibody fragment, single chain antibody, disulfide stabilized antibody H chain or disulfide stabilized antibody L chain contained in a culture supernatant or the like can be confirmed by the method described in the above 1(4) or the like.

The single chain antibody, disulfide stabilized antibody H chain or disulfide stabilized antibody L chain can be recovered and purified by combining known techniques. For example, when the antibody fragment expression vector, single chain antibody, disulfide stabilized antibody H chain or disulfide stabilized antibody L chain is secreted into a culture medium, it can be concentrated by ultrafiltration, and then it can be recovered and purified by various chromatography or gel filtration. Also, when it is transported into a periplasmic region of the host cell, it can be concentrated by ultrafiltration after adding an osmotic pressure shock to the cell, and then it can be recovered and purified by various chromatography or gel filtration. When the antibody fragment expression vector, single chain antibody, disulfide stabilized antibody H chain or disulfide stabilized antibody L chain is insoluble and present as granules (inclusion body), centrifugation and washing for dissolving the cell and isolating granules are repeated, it is solubilized, for example with guanidine-hydrochloric acid, and then it can be recovered and purified by various chromatography or gel filtration.

The activity of the single chain antibody can be measured by the method described in the above 1(4) or the like.

The purified disulfide stabilized antibody H chain and disulfide stabilized antibody L chain are mixed and a disulfide bond is formed by a technique for leading to an active structure (refolding procedure, Molecular Immunology, 32, 249 (1995)), and then the disulfide stabilized antibody having the activity can be purified by antigen affinity chromatography, ion exchange chromatography or gel filtration. The activity of the disulfide stabilized antibody can be measured by the method described in the above 1(4) or the like.

### 4. Method for producing fused antibody

A fused antibody in which an antibody and a toxin protein are chemically fused can be prepared in accordance with, for example, Anticancer Research, 11, 2003 (1991); Nature Medicine, 3, 350 (1996). A fused antibody in which an antibody is fused to a protein such as toxin, cytokine or the like by genetic engineering techniques can be prepared in accordance with, for example, Proc. Natl. Acad. Science, USA, 93, 974 (1996), Proc. Natl. Acad. Science, USA, 93, 7826 (1996). A fused antibody in which an antibody is chemically fused to a low molecular weight anticancer agent can be prepared in accordance with, for example, Science, 261, 212 (1993). A fused antibody in which an antibody is chemically fused to a radioisotope can be prepared in accordance with, for example, Antibody Immunoconjugates and Radiopharmaceuticals, 3, 60 (1990); Anticancer Research, 11, 2003 (1991).

Since these analogues can accumulate a radioisotope, a protein (a cytokine, a toxin, an enzyme or the like), a low molecular weight agent or the like into the periphery of a target tissue according to the specificity of the antibody molecule, a diagnosis or treatment which is more effective and has less side effects can be expected from them.

### 5. Diagnosis method for disease related to monocyte and macrophage

Examples of diseases related to a monocyte and/or a macrophage include an inflammatory disease, a delayed type hypersensitivity, an arteriosclerosis, a malignant tumor, and the like. The diagnosis method using the antibody used in the present invention includes a method in which a human VEGF receptor Flt-1 existing in the cells or tissues of people to be tested is immunologically detected or determined as described below.

The method for immunologically detecting a monocyte and/or a macrophage using an antibody against the VEGF receptor Flt-1, includes a fluorescent antibody method, an enzyme-linked immunosorbent assay (ELISA), a radioimmunoassay (RIA), an immunohistochemical staining method (ABC method, CSA method or the like) such as immunological tissue staining, immunological cell staining or the like, Western blotting, an immunoprecipitation method, the above enzyme immunoassay, sandwich ELISA (Monoclonal Antibody Experimentation Manual (Kodan-sha Scientific, 1987), Second Series Biochemical Experimentation Course 5, Immunobiochemical Method (Tokyo Kagaku Dojin, 1986)) and the like.

The fluorescent antibody method can be carried out using the method described in Monoclonal Antibodies: Principles and practice, Third edition (Academic Press, 1996), Monoclonal Antibody Experimentation Manual (Kodansha Scientific, 1987) or the like. Specifically, this is a method in which a separated cell or tissue or the like is allowed to react with the antibody used in the present invention and then with an anti-immunoglobulin antibody or binding fragment labeled with a fluorescent material such as fluorescein isothiocyanate (FITC), phycoerythrin or the like, and then the fluorescence dye is measured using a flow cytometer.

The immunohistochemical staining method (ABC method, CSA method or the like) such as immunological tissue staining, immunological cell staining or the like can be carried out according to the method described in Monoclonal Antibodies: Principles and practice, Third edition (Academic Press, 1996), Monoclonal Antibody Experimentation Manual (Kodan-sha Scientific, 1987), and the like.

The enzyme-linked immunosorbent assay (ELISA) is a method in which a cell or disintegrated solution thereof, a tissue or disintegrated solution thereof, a cell culture supernatant, a serum, a pleural effusion, an ascites, an eye fluid or the like is allowed to react with the antibody and then with an anti-immunoglobulin antibody or binding fragment treated with an enzyme label such as peroxidase, biotin or the like, and then the colored dye is measured using a spectrophotometer.

The radioimmunoassay (RIA) is a method in which a cell or disintegrated solution thereof, a tissue or disintegrated solution thereof, separated sample such as a cell culture supernatant, a serum, a pleural effusion, an ascites, an eye fluid or the like is allowed to react with the antibody and then with an anti-immunoglobulin antibody or binding fragment treated with a radioactive ray label, and then the radioactivity is measured using a scintillation counter or the like.

The immunological tissue staining or immunological cell staining is a method in which a separated cell or tissue or the like is allowed to react with the antibody and then with an anti-immunoglobulin antibody or binding fragment labeled with a fluorescent material such as fluorescein isothiocyanate (FITC) or the like or an enzyme label such as peroxidase, biotin or the like, and then the sample is observed using a microscope.

The Western blotting is a method in which a cell or a disrupted solution thereof or a tissue or a disrupted solution thereof which is separated from the living body, a cell culturing supernatant, a serum or the like is fractionated by SDS-polyacrylamide gel electrophoresis (Antibodies-A Laboratory Manual, Cold Spring Harbor Laboratory, 1988) and then blotted on a nitrocellulose membrane, the membrane is allowed to react with the monoclonal antibody of the present invention or an antibody fragment thereof and then with an anti-mouse IgG antibody or binding fragment labeled with a fluorescent material such as FITC or the like or an enzyme label such as peroxidase, biotin or the like, and then the reaction result is confirmed.

The immunoprecipitation is a method in which a cell or a disrupted solution thereof or a tissue or a disrupted solution thereof which is separated from the living body, a cell culturing supernatant, a serum or the like is allowed to react with the monoclonal antibody of the present invention or an antibody fragment thereof and then mixed with a carrier capable of specifically binding to the immunoglobulin (e.g., protein G-Sepharose or the like) to thereby precipitate the antigen-antibody complex.
The sandwich ELISA is a method in which, among two monoclonal antibodies of the present invention or antibody fragments thereof having different antigen recognition sites, one of the monoclonal antibodies or an antibody fragments is adsorbed to a plate in advance, and another monoclonal antibody or antibody fragment is labeled with a fluorescent material such as FITC or the like or an enzyme such as peroxidase, biotin or the like. The antibody-adsorbed plate is allowed to react with a cell or a disrupted solution thereof or a tissue or a disrupted solution thereof which is separated from the living body, a cell culturing supernatant, a serum or the like and then with the labeled monoclonal antibody to carry out the reaction corresponding to the label.

### 6. Therapeutic agent and diagnostic agent for diseases related to a monocyte and/or a macrophage

Since the above anti-Flt-1 antibody, antibody fragment thereof and analogue thereof bind to Flt-1 on a monocyte and/or macrophage to thereby inhibit migration of a monocyte induced by VEGF and destroy cells which express a VEGF receptor Flt-1 on the cell surface through antibody effector activity such as ADCC, CDC or the like, they can be used as an inhibitor of migration of a monocyte and/or a macrophage and are useful for, for example, treatment of diseases related to a monocyte and/or a macrophage.

A medicament comprising the antibody used in the present invention can be administered as a therapeutic agent alone, but generally, it is preferred to provide it as a pharmaceutical preparation produced by an optional method well known in the technical field of manufacturing pharmacy, by mixing it with one or more pharmaceutically acceptable carriers.

It is preferred to use a route of administration which is most effective in a treatment. Examples include oral administration and parenteral administration such as buccal, airway, rectal, subcutaneous, intramuscular or intravenous administration. Intravenous administration is preferred in an antibody or peptide preparation.

The dosage form includes sprays, capsules, tablets, granules, syrups, emulsions, suppositories, injections, ointments, tapes and the like.

Liquid preparations such as emulsions and syrups can be produced using, as additives, water; saccharides such as sucrose, sorbitol, fructose, etc.; glycols such as polyethylene glycol, propylene glycol, etc.; oils such as sesame oil, olive oil, soybean oil, etc.; antiseptics such as p-hydroxybenzoic acid esters, etc.; flavors such as strawberry flavor, peppermint, etc.; and the like.

Capsules, tablets, powders, granules and the like can be produced using, as additives, fillers such as lactose, glucose, sucrose, mannitol, etc.; disintegrating agents such as starch, sodium alginate, etc.; lubricants such as magnesium stearate, talc, etc.; binders such as polyvinyl alcohol, hydroxypropylcellulose, gelatin etc.; surfactants such as fatty acid ester, etc.; plasticizers such as glycerine, etc.; and the like.

Examples of the pharmaceutical preparation suitable for parenteral administration include injections, suppositories, sprays and the like.

The injections are prepared using a carrier such as a salt solution, a glucose solution or a mixture thereof or the like.

The suppositories are prepared using a carrier such as cacao butter, hydrogenated fat, carboxylic acid or the like.

Also, sprays are prepared using the compound as such or using a carrier or the like which does not stimulate the buccal or airway mucous membrane of the patient and can facilitate absorption of the compound by dispersing it as fine particles.

Examples of the carrier include lactose, glycerol and the like. It is possible to produce pharmaceutical preparations such as aerosols, dry powders and the like. Depending on the properties of the antibody and the carrier to be used, in addition, the components exemplified as additives for oral preparations can also be added to these parenteral preparations.

Although the clinical dose or the frequency of administration varies depending on the intended therapeutic effect, administration method, treating period, age, body weight and the like, it is usually from 10 µg/kg to 8 mg/kg per day per adult.

The antibody used in the present invention can be used as a diagnostic agent or therapeutic agent for diseases related to a monocyte and/or a macrophage.

Also, among the antibodies used in the present invention, the antibody having neutralizing activity for human Flt-1 inhibits binding of human VEGF to Flt-1. That is, it inhibits Flt-1 self-phosphorylation and inhibits migration of a human monocyte VEGF-dependently. Accordingly, it can be used as a therapeutic agent for diseases which accompanies infiltration of a monocyte and/or a macrophage, such as inflammatory disease, delayed type hypersensitivity, arteriosclerosis, malignant tumor, and the like. Examples of the inflammatory disease include rheumatoid arthritis, juvenile rheumatoid arthritis, systemic sclerosis, autoimmune thyropathy, multiple sclerosis, Crohn disease, ulcerative colitis, and the like. Examples of the delayed type hypersensitivity include contact dermatitis, and the like. Examples of the arteriosclerosis include atherosclerosis and the like. Examples of the malignant tumor include melanoma, breast cancer, brain tumor, and the like.

Also, the method for measuring the activity of inhibiting migration of a monocyte and/or a macrophage using the antibody used in the present invention can be carried out in accordance with the method described in Blood, 87, 3336 (1996) or the like.

Examples of the present invention are shown below.

### BRIEF DESCRIPTION OF THE DRAWINGS

Fig. 1 shows a result of the measurement of forward scattering value and side-way scattering value of human peripheral blood mononuclear corpuscles using a flow cytometer. The gate of M indicates monocytes, and the gate of L indicates lymphocytes.
Fig. 2 shows a result of the examination of reactivity of the monocytes of gate M of Fig. 1 with anti-CD97 antibody and anti-CD11b antibody using a flow cytometer.
Fig. 3 shows a result of the examination of reactivity of the monocytes of gate M of Fig. 1 with anti-CD97 antibody and anti-CD11c antibody using a flow cytometer.
Fig. 4 shows a result of the examination of reactivity of the human lymphocyte of gate L of Fig. 1 with anti-Flt-1 antibody and anti-CD34 antibody.
Fig. 5 shows a result of the examination of reactivity of the human monocytes of gate M of Fig. 1 with anti-Flt-1 antibody and anti-CD34 antibody.
Fig. 6 shows a result of the examination of reactivity of the human monocytes of gate M of Fig. 1 with anti-Flt-1 antibody and anti-CD97 antibody.
Fig. 7 shows a result of the measurement, of forward scattering value and side-way scattering value of human cord venous blood mononuclear corpuscles using a flow cytometer. The gate of M indicates monocytes, and the gate of L indicates lymphocytes.
Fig. 8 shows a result of the examination of reactivity of the human lymphocyte of gate L of Fig. 7 with anti-CD97 antibody and anti-CD34 antibody.
Fig. 9 shows a result of the examination of reactivity of the human lymphocyte of gate L of Fig. 7 with anti-Flt-1 antibody and anti-CD97 antibody using a flow cytometer.
Fig. 10 shows a result of the measurement of forward scattering value and side-way scattering value of monocytes obtained by culturing CD34-positive cells in human cord venous blood, using a flow cytometer. The gate of M indicates monocytes, and the gate of L indicates lymphocytes.
Fig. 11 shows a result of the examination of reactivity of the human monocytes of gate M of Fig. 10 with anti-CD97 antibody and anti-CD34 antibody.
Fig. 12 shows a result of the examination of reactivity of the human monocytes of gate M of Fig. 10 with anti-Flt-1 antibody and anti-CD34 antibody.
Fig. 13 shows a result of the examination of effects of VEGF and anti-Flt-1 antibody on the migration ability of human monocytes.
Fig. 14 is a graph showing construction steps of plasmid pBS1732H.
Fig. 15 is a graph showing construction steps of plasmid pBS1732L.
Fig. 16 is a graph showing construction steps of plasmid pKANTEX1732H.
Fig. 17 is a graph showing construction steps of plasmid pKANTEX1732.
Fig. 18 is a graph showing construction steps of plasmid pBS1750H.
Fig. 19 is a graph showing construction steps of plasmid pBS1750L.
Fig. 20 is a graph showing construction steps of plasmid pKANTEX1750H.
Fig. 21 is a graph showing construction steps of plasmid pKANTEX1750.
Fig. 22 is a graph showing SDS-PAGE (a 4 to 15% gradient gel was used) electrophoresis patterns of purified anti-human VEGF receptor Flt-1 human chimeric antibodies KM2532 and KM2550. Lane 1: low molecular weight marker, lane 2: KM2532 under reducing conditions, lane 3: KM2550 under reducing conditions, lane 4: high molecular weight marker, lane 5: KM2532 under non-reducing conditions, lane 6: KM2550 under non-reducing conditions.

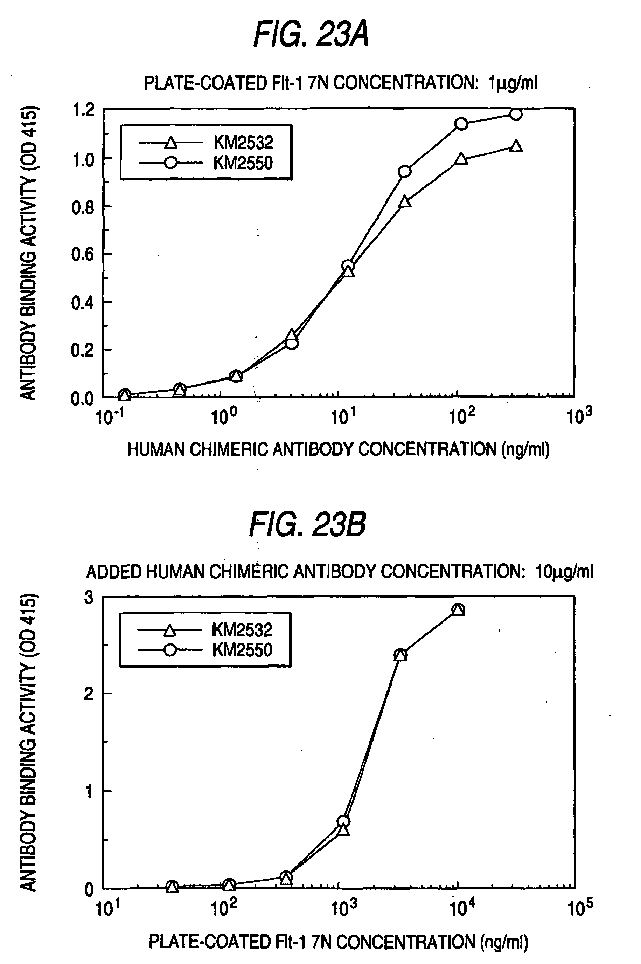

Fig. 23 is a graph showing the activity of purified anti-human VEGF receptor Flt-1 human chimeric antibodies KM2532 and KM2550 to bind to soluble human VEGF receptor Flt-1 7N. (A) shows the results when concentration of soluble human VEGF receptor Flt-1 7N to be adsorbed on the plate is fixed (1 µg/ml) and concentration of each human chimeric antibody to be added is varied. The binding activity with soluble human VEGF receptor Flt-1 7N is plotted as ordinate and the concentration of each human chimeric antibody as abscissa. "Δ" indicates binding activity of KM2532 and "O" indicates that of KM2550. (B) shows the results when binding activity of each human chimeric antibody at a fixed concentration (10 µg/ml) was measured by varying concentration of soluble human VEGF receptor Flt-1 7N to be adsorbed on the plate. The binding activity with soluble human VEGF receptor Flt-1 7N is plotted as ordinate, and-the concentration of soluble human VEGF receptor Flt-1 7N adsorbed on the plate as abscissa. "." indicates binding activity of KM2532 and "." indicates that of KM2550.
Fig. 24 is a graph showing the activity of purified anti-human VEGF receptor Flt-1 human chimeric antibodies KM2532 and KM2550 to inhibit binding between human VEGF and human VEGF receptor Flt-1. "O", "□", "●" and "■" shows the binding inhibition activities of KM1732, KM1750, KM2532 and KM2550, respectively.
Fig. 25 is a graph showing construction steps of plasmid phKM1732HV0.
Fig. 26 is a graph showing construction steps of plasmid phKM1750HV0.
Fig. 27 is a graph showing construction steps of plasmid phKM1732LV0.
Fig. 28 is a graph showing construction steps of plasmid phKM1750LV0(IV).
Fig. 29 is a graph showing construction steps of plasmid pKANTEX1732HV0.
Fig. 30 is a graph showing construction steps of plasmid pKANTEX1732HV0LV0.
Fig. 31 is a graph showing construction steps of plasmid pKANTEX1750HV0LV0(IV).
Fig. 32 is a graph showing the binding activity of anti-human VEGF receptor Flt-1 human monoclonal antibodies KM1732 and KM1750 and anti-human VEGF receptor Fit-1 human chimeric antibodies KM2532 and KM2550 upon soluble human VEGF receptor derivatives Flt-1 7N, Flt-1 3N, Flt-1 2N, Flt-1 7N.K2 and KDR-7N according to the enzyme immunoassay.
Fig. 33 is a graph showing the concentration-dependent binding activity of anti-human VEGF receptor Flt-1 human chimeric antibodies KM2532 and KM2550 upon soluble human VEGF receptor derivatives Flt-1 7N, Flt-1 3N, Flt-1 2N, Flt-1 7N.K2 and KDR-7N according to the enzyme immunoassay.
Fig. 34 is a schematic illustration of soluble human VEGF receptor derivatives.
Fig. 35 is a graph showing construction steps of plasmid phKM1750LV0 (I).
Fig. 36 is a graph showing construction steps of plasmid pKANTEX1750HV0LV0 (I).
Fig. 37 is a graph showing construction steps of plasmid phKM1750HV3.
Fig. 38 is a graph showing construction steps of plasmid phKM1750LV4.
Fig. 39 is a graph showing construction steps of plasmid pKANTEX1750HV3LV0 (I).
Fig. 40 is a graph showing construction steps of plasmid pKANTEX1750HV3LV0 (IV).
Fig. 41 is a graph showing construction steps of plasmid pKANTEX1750HV0LV4.
Fig. 42 is a graph showing construction steps of plasmid pKANTEX1750HV3LV4.

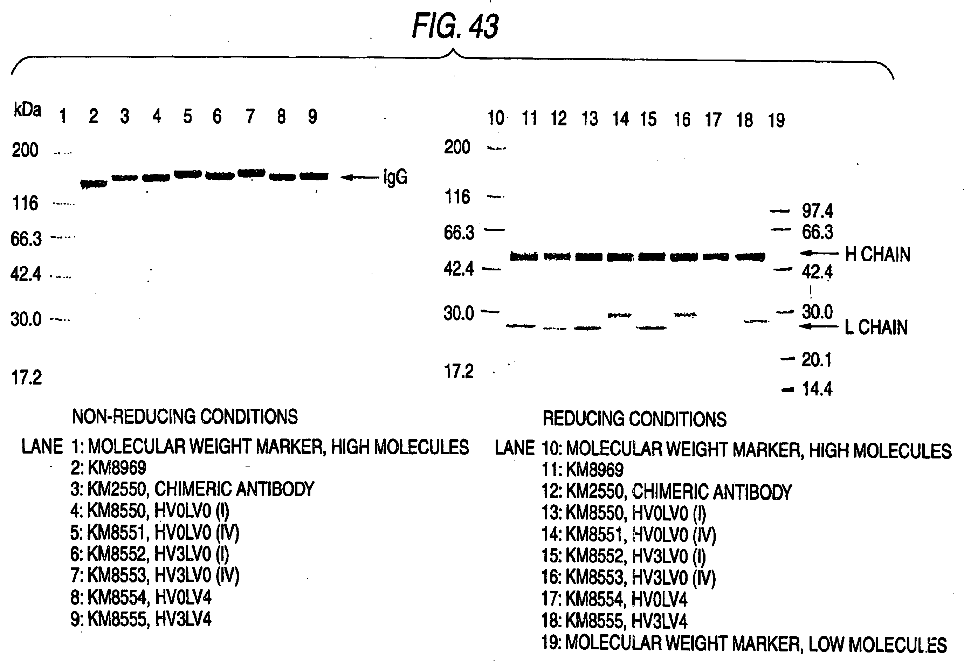

Fig. 43 is a graph showing SDS-PAGE (a 4 to 15% gradient gel was used) electrophoresis patterns of anti-human VEGF receptor Flt-1 human chimeric antibody KM2550 and human CDR-grafted antibodies KM8550, KM8551, KM8552, KM8553, KM8554 and KM8555. Lanes 1 to 9 show electrophoresis patterns under non-reducing conditions and lanes 10 to 19 shows electrophoresis patterns under reducing conditions. Lanes 1 and 10: high molecular weight marker, lane 19: low molecular weight marker, lanes 2 and 11: control antibody KM8969, lanes 3 and 12: KM2550, lanes 4 and 13: KM8550, lanes 5 and 14: KM8551, lanes 6 and 15: KM8552, lanes 7 and 16: KM8553, lanes 8 and 17: KM8554, and lanes 9 and 18: KM8555.
Fig. 44 is a graph showing the binding activity of anti-human VEGF receptor Flt-1 human chimeric antibody KM2550 and human CDR-grafted antibodies KM8550, KM8551, KM8552, KM8553, KM8554 and KM8555 upon soluble human VEGF receptor Flt-1 7N.
Fig. 45 is a graph showing the binding activity of anti-human VEGF receptor Flt-1 human chimeric antibody KM2550 and human CDR-grafted antibodies KM8550, KM8551, KM8552, KM8553, KM8554 and KM8555 upon soluble human VEGF receptor Flt-1 7N, Flt-1 3N, Flt-1 2N, Flt-1 7N.K2 and KDR 7N.
Fig. 46 is a graph- showing the activity of anti-human VEGF receptor Flt-1 human chimeric antibody KM2550 and human CDR-grafted antibodies KM8550, KM8551, KM8552, KM8553, KM8554 and KM8555 to inhibit the binding between human VEGF and human VEGF receptor Flt-1.
Fig. 47 is a graph showing amino acid sequences of the H chain variable regions of anti-human VEGF receptor Flt-1 human chimeric antibody and CDR-grafted antibody. In the drawing, KM1750mouse shows H chain variable region amino acid sequence of KM1750; KM1750HV0 shows an Amino acid sequence constituted by inserting CDR of H chain variable region of KM1750 into a human framework; and KM1750HV3 shows an amino acid sequence in which some of the amino acid sequence of the framework of KM1750HV0 are substituted with the amino acids of KM1750mouse.
Fig. 48 is a graph showing amino acid sequences of L chain variable region of anti-human VEGF receptor Flt-1 human chimeric antibody and CDR-grafted antibody. In the drawing, KM1750mouse shows an amino acid sequence of L chain V region of KM1750; KM1750LV0 (I) show an amino acid sequence constituted by inserting CDR of L chain variable region of KM1750 into a human framework; and KM1750LV4 shows an amino acid sequence in which some of the amino acid sequence of the framework of KM1750LV0 (I) are substituted with the amino acids of KM1750mouse.

### BEST MODE FOR CARRYING OUT THE INVENTION

### Example 1

### Reactivity of anti-human Flt-1 monoclonal antibody for human peripheral blood monocyte:

Reactivity of an anti-human Flt-1 monoclonal antibody KM1730 (WO 98/22616) for a human monocyte was confirmed in accordance with the following procedures using a fluorescent antibody staining method.

First, by the following procedure, a mononuclear corpuscle fraction comprising monocytes and lymphocytes was separated from human peripheral blood and its reactivity with anti-CD11b antibody, anti-CD11c antibody and anti-CD97 antibody (Leukocyte Typing VI, Kishimoto et al., Garland Publishing (1997)) was confirmed.

CD11b, CD11c and CD97 are expressed on monocytes *(*Leukocyte Typing VI, Kishimoto et al., Garland Publishing (1997)).

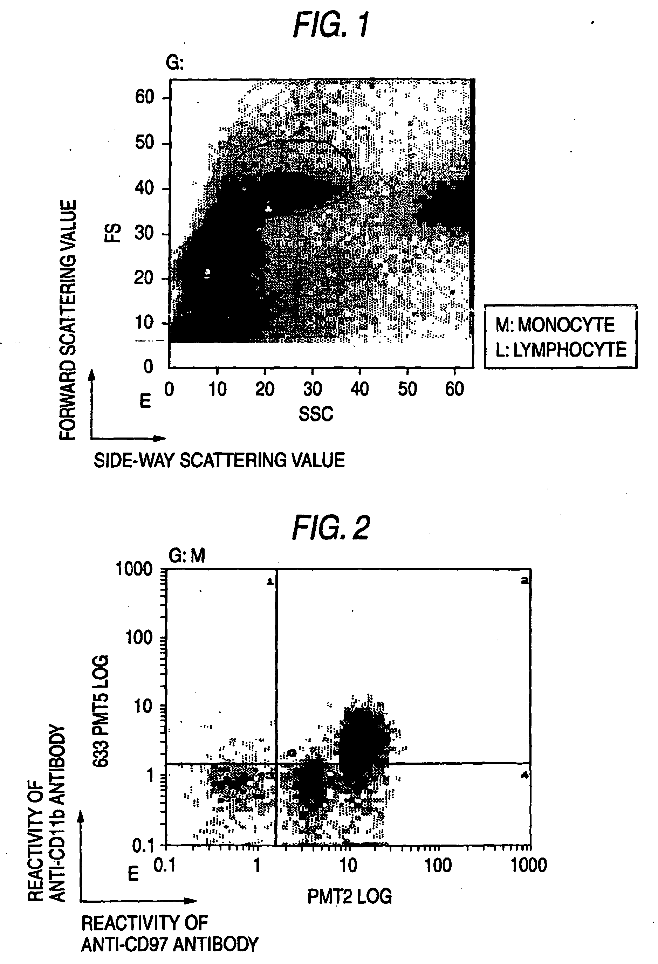

In accordance with the Ficoll/Paque density gradient centrifugation method described in The Journal of Immunology, 147, 2251 (1991), a mononuclear .corpuscle fraction comprising monocytes and lymphocytes was recovered from peripheral blood of a healthy people. The mononuclear corpuscle fraction was washed with PBS (9.6 mM phosphate buffer, pH 7.4, containing 137 mM NaCl and 2.7 mM KCl) and then suspended in 0.1% bovine serum albumin (hereinafter referred to as "BSA", manufactured by Sigma)-containing PBS (hereinafter referred to as "0.1% BSA-PBS"). The mononuclear corpuscles (3×10⁶) were suspended in 300 µl of 0.1% BSA-PBS and dispensed into 12 x 75 mm glass tubes (manufactured by Iwaki Glass), each of stock solutions of an FITC (fluorescein isothiocyanate)-labeled anti-CD97 antibody (manufactured by Pharmingen), a PE (phycoerythrin)-labeled anti-CD11c antibody (manufactured by Heckman-Coulter) and a biotin-labeled anti-CD11b antibody (manufactured by Beckman-Coulter) was dispensed in 10 µl into the glass tubes, and then the mononuclear corpuscles and antibodies were allowed to react at room temperature for 15 minutes. After the reaction, the mixture was centrifuged and then washed and suspended in 300 µl of 0.1% BSA-PBS, and the suspension was mixed with 10 µl of 50 times-diluted streptavidin-APC (manufactured by Becton Dickinson) to carry out the reaction at room temperature for 15 minutes. After the reaction, the mixture was centrifuged, washed and then analyzed using a flow cytometer (Epics Elite, manufactured by Coulter). The results are shown in Figs. 1, 2 and 3, respectively.

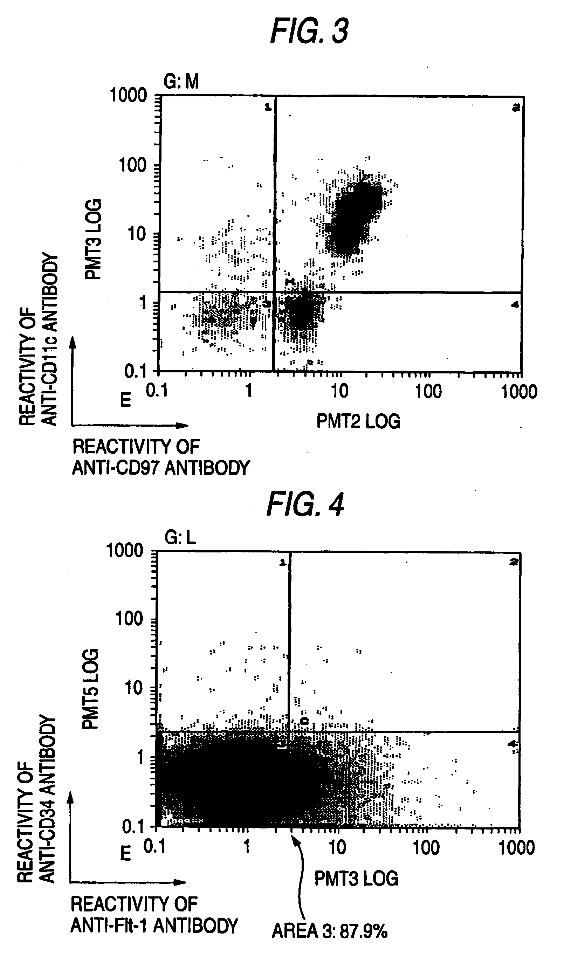

Fig. 1 shows a cytogram on the mononuclear corpuscle fraction, in which the forward scattering value is plotted as the ordinate and the side-way scattering value as the abscissa. The mononuclear corpuscle fraction contains monocytes indicated by M and lymphocytes indicated by L. By selecting cells of the specified region in the cytogram from the monocyte fraction indicated by M (hereinafter, the term "to select cells of the specified region in the cytogram" is described as "to put a gate"), reactivity of the monocytes with the anti-CD11b antibody, the anti-CD11c antibody and the anti-CD97 antibody was examined. The results are shown in Figs. 2 and 3. Since all of CD11b, CD11c and CD97 whose expression on monocytes has been reported *(*Leukocyte Typing VI: Kishimoto et al., Garland Publishing (1997)) showed the reaction, it was confirmed that the cells of gate M used in this example are monocyte.

Next, reactivity of an anti-Flt-1 antibody with monocytes and lymphocytes was examined by the following procedure.

CD34 is known as a marker expressed by blood stem cells *(*Leukocyte Typing VI, Kishimoto et al., Garland Publishing (1997)).

Regarding the reactivity of an anti-Flt-1 monoclonal antibody and an anti-CD34 antibody, 3×10⁶ cells of the mononuclear corpuscles were suspended in 300 µl of 0.1% BSA-PBS and dispensed into 12 x 75 mm glass tubes (manufactured by Iwaki Glass) and 1 mg/ml of the anti-human Flt-1 monoclonal antibody KM1730 was dispensed in 10 µl portions to carry out the reaction at room temperature for 15 minutes. After the reaction, the mixture was centrifuged and then washed and suspended in 300 µl of 0.1% BSA-PBS, and the suspension was mixed with 10 µl of 50 times-diluted PE-labeled goat anti-mouse IgG (manufactured by Dako) to carry out the reaction at room temperature for 15 minutes. After the reaction, the mixture was centrifuged and then washed and suspended in 300 µl of 0.1% BSA-PBS, and the suspension was mixed with 10 µl of a PC5 (abbreviation of PE-Cy5; PE: phycoerythrin, Cy5: trade name)-labeled anti-CD34 antibody (class III, manufactured by Beckman Coulter) to carry out the reaction at room temperature for 15 minutes. After the reaction, the mixture was centrifuged, washed and then analyzed using a flow cytometer (Epics Elite, manufactured by Coulter).

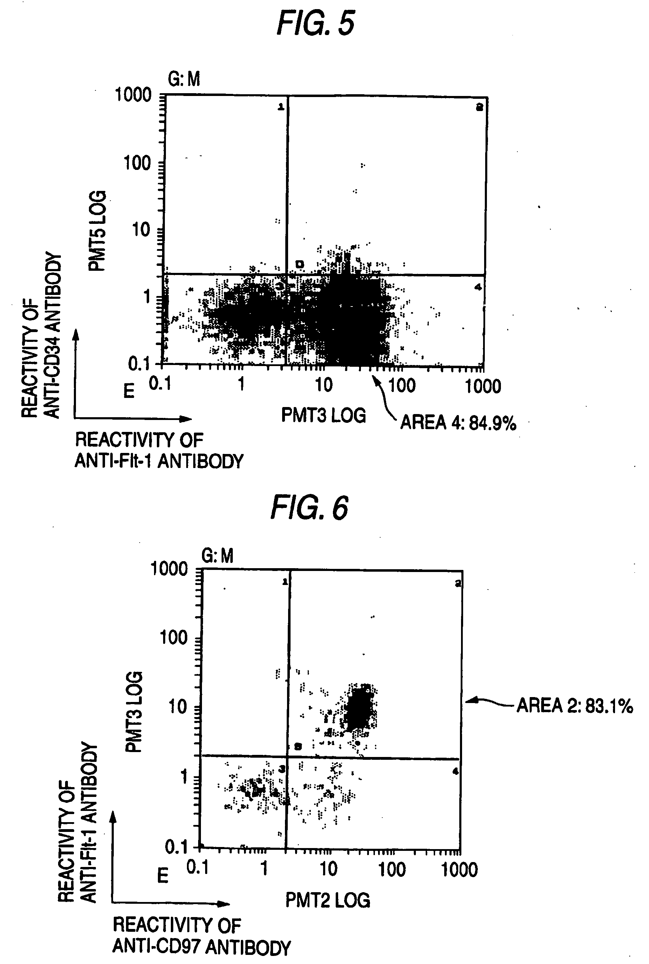

Figs. 4 and 5 show reactivity of the KM1730 and the anti-CD34 antibody with the lymphocytes of gate L and monocytes of gate M, respectively. In the drawings, area 1 indicates CD34-positive and Flt-1-negative cells, area 2 indicates CD34-positive and Flt-1-positive cells, area 3 indicates CD34-negative and Flt-1-negative cells, and area 4 indicates CD34-negative and Flt-1-positive cells. Regarding lymphocytes, cells in the area 3 with which both of the anti-Flt-1 antibody and the anti-CD34 antibody did not react occupied the majority of 87.9%. On the other hand, regarding monocytes, cells in the area 4 with which the anti-CD34 antibody did not react but the anti-Flt-1 antibody reacted occupied the majority of 84.9%. Accordingly, it was found that the Flt-1 antibody selectively react with monocytes among mononuclear corpuscles.

The reactivity of monocytes with an anti-Flt-1 monoclonal antibody and an anti-CD97 antibody was examined in the following manner. The mononuclear corpuscles (3 × 10⁶) were suspended in 300 µl of 0.1% BSA-PBS and dispensed into 12 x 75 mm glass tubes (manufactured by Iwaki Glass) and 1 mg/ml of the anti-human Flt-1 monoclonal antibody KM1730 was dispensed in 10 µl to carry out the reaction at room temperature for 15 minutes. After the reaction, the mixture was centrifuged and then washed and suspended in 300 µl of 0.1% BSA-PBS, and the suspension was mixed with 10 µl of 50 times-diluted PE-labeled goat anti-mouse IgG (manufactured by Dako) to carry out the reaction at room temperature for 15 minutes. After the reaction, the mixture was centrifuged and then washed and suspended in 300 µl of 0.1% BSA-PBS, and the suspension was mixed with 10 µl of the stock solution of FITC-labeled anti-CD97 antibody (manufactured by Pharmingen) to carry out the reaction at room temperature for 15 minutes. After the reaction, the mixture was centrifuged, washed and then analyzed using a flow cytometer (Epics Elite, manufactured by Coulter). The results are shown in Fig. 6.

Reactivity of the monocytes of gate M with the anti-Flt-1 monoclonal antibody KM1730 (WO 98/22616) and the anti-CD97 antibody is shown in Fig. 6. The area 2 shows cells with which both of the anti-Flt-1 antibody and the anti-CD97 antibody reacted, and they occupied the majority of 83.1%. Thus, it was confirmed that the anti-Flt-1 antibody reacts with the cell surface of monocytes expressing CD97.

### Example 2

### Reactivity of anti-human Flt-1 monoclonal antibody with human cord venous cell and differentiated monocyte

Since it was confirmed that the anti-Flt-1 antibody reacts with monocytes prepared from peripheral blood of a healthy people, in order to examine reactivity of the anti-Flt-1 antibody with differentiated stage of monocytes, monocytes were differentiated from blood stem cells among human cord venous cells and reactivity of the anti-Flt-1 monoclonal antibody KM1730 (WO 98/22616) with cells before and after differentiation was confirmed in accordance with the following procedures using a fluorescent antibody staining method.

First, by the following procedure, a mononuclear corpuscle fraction containing monocytes and lymphocytes was separated from human cord blood, and its reactivities with an anti-CD34 antibody whose reactivity with blood stem cells has been reported (Leukocyte Typing VI: Kishimoto et al., Garland Publishing (1997)), an anti-CD97 antibody whose reactivity with monocyte has been reported (Leukocyte Typing VI: Kishimoto et al., Garland Publishing (1997)) and an anti-Flt-1 antibody were confirmed in accordance with the procedure shown below.

In accordance with the Ficoll/Paque density gradient centrifugation method described in a reference (Blood, 90, 4363 (1997)), a mononuclear corpuscle fraction containing monocytes and lymphocytes was recovered from human cord blood. The mononuclear corpuscle fraction was washed with PBS and then suspended in 0.1% BSA-PBS. The mononuclear corpuscles (3×10⁶) were suspended in 300 µl of 0.1% BSA-PBS and dispensed into 12 x 75 mm glass tubes (manufactured by Iwaki Glass) and 1 mg/ml of the anti-Flt-1 monoclonal antibody KM1730 was dispensed in 10 µl to carry out the reaction at room temperature for 15 minutes.

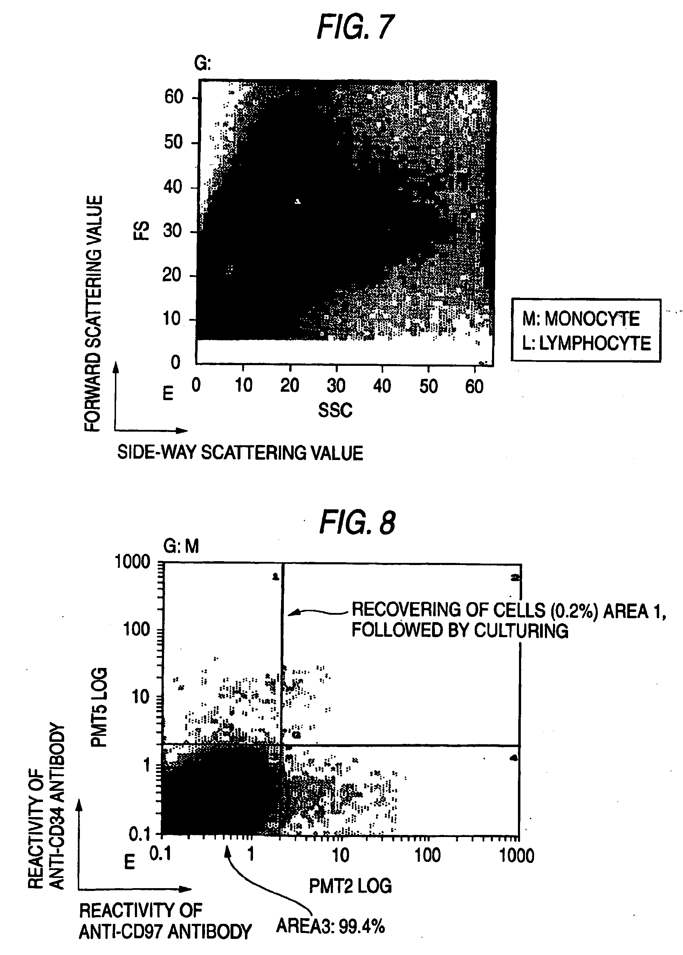

After the reaction, the mixture was centrifuged and then washed and suspended in 300 µl of 0.1% BSA-PBS, and the suspension was mixed with 10 µl of 50 times-diluted PE-labeled goat anti-mouse IgG (manufactured by Dako) to carry out the reaction at room temperature for 15 minutes. After the reaction, the mixture was centrifuged and then washed and suspended in 300 µl of 0.1% BSA-PBS, and the suspension was mixed with 10 µl of each of the stock solutions of PC5-labeled anti-CD34 antibody (class III, manufactured by Beckman Coulter) and FITC-labeled anti-CD97 antibody (manufactured by Pharmingen) to carry out the reaction at room temperature for 15 minutes. After the reaction, the mixture was washed by centrifugation and then analyzed using a flow cytometer (Epics Elite, manufactured by Coulter). The results are shown in Figs. 7, 8 and 9.

Fig. 7 shows a cytogram on the mononuclear corpuscle fraction, in which the forward scattering value is plotted as the ordinate and the side-way scattering value as the abscissa. The mononuclear corpuscle fraction contains monocytes indicated by M and lymphocytes indicated by L. Blood stem cells are contained in the lymphocyte fraction indicated by L. A result of the examination on the reactivity of the anti-CD34 antibody and the anti-CD97 antibody carried out by putting a gate on the lymphocyte fraction indicated by L is shown in Fig. 8. The fraction of L was CD34-negative as the blood stem cell marker, and the CD97-negative cells as the monocyte marker were the majority of 99.4% (area 3). The blood stem cell-containing CD34-positive cells were contained at a considerably low ratio of 0.2% (area 1), and the cell group hardly reacted with the anti-CD97 antibody.

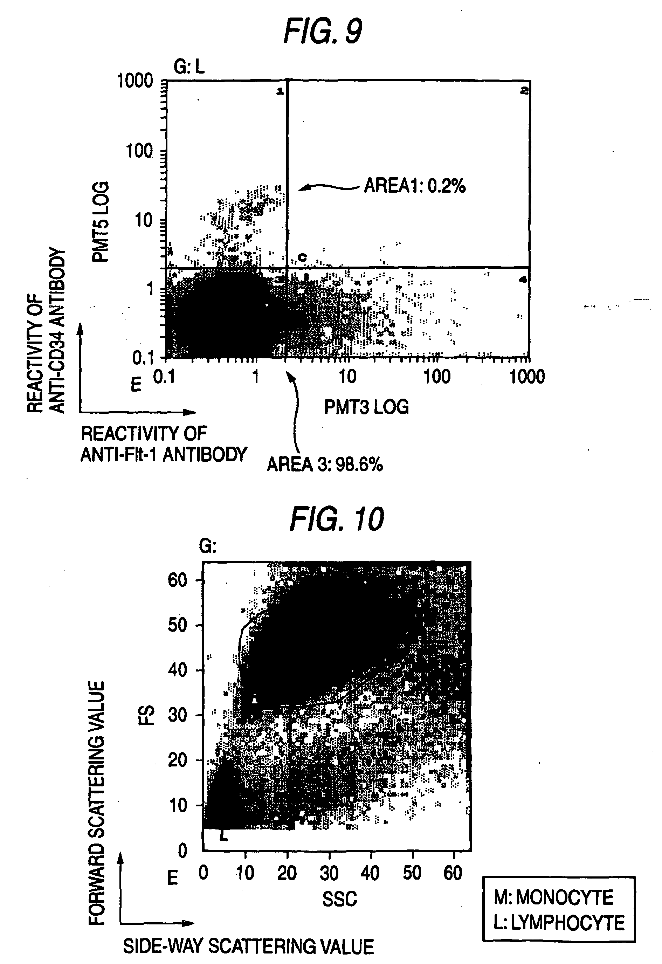

A result of the examination on the reactivity of the anti-CD34 antibody and the anti-Flt-1 antibody carried out by putting a gate on the lymphocyte fraction indicated by L is shown in Fig. 9. The fraction of L was CD34-negative as the blood stem cell marker, and the Flt-1-negative cells were the majority of 98.6% (area 3). The blood stem cell-containing CD34-positive cells were contained at a considerably low ratio of 0.2% (area 1), and this cell group hardly reacted with the anti-Flt-1 antibody.

From the above results, it was found that the CD34-positive cells containing blood stem cells prepared from human cord blood do not show reactivity with the anti-CD97 antibody whose reactivity with monocyte has been reported and the anti-Flt-1 antibody whose reactivity with monocyte has been confirmed in Example 1.

Next, CD34-positive cells containing blood stem cells were prepared from human cord blood, the thus prepared cells were differentiated into lineages of monocyte by adding various types of cytokine, and reactivities of lineages of monocyte with an anti-CD34 antibody, an anti-CD97 antibody and an anti-Flt-1 antibody were confirmed in accordance with the procedure shown below.

A cell group shown in Fig. 8 (area 1) which reacted with anti-CD34 antibody but did not react with anti-CD97 antibody was recovered by a sorting method in accordance with the method described in a reference (*Blood,* 93, 2525 (1999)). The thus recovered CD34-positive cells were suspended in α-MEM medium (manufactured by ICN Biochemicals) containing 30% FCS (manufactured by Hyclone), and the suspension was dispensed in 1×10⁴ cells/2 ml portions into wells of a 6 well plate, mixed with each of IL-3 (final concentration 200 U/ml: Proceeding of National Academy of Science USA, 92, 2859 (1995)), IL-6 (final concentration 200 U/ml: Bllod, 91, 187 (1998)), M-CSF (final concentration 100 ng/ml: Blood, 92, 118 (1998)), GM-CSF (final concentration 20 ng/ml: Proceeding of National Academy of Science USA, 92, 2859 (1995)) and Flt-3 ligand (final concentration 100 ng/ml, manufactured by R & D) and then cultured at 37°C for 7 days in a CO₂ incubator.

After culturing, the cells were recovered, washed with PBS and then suspended in 0.1% BSA-PBS. The recovered cells (3×10⁶) were suspended in 300 µl of 0.1% BSA-PBS and dispensed into 12 x 75 mm glass tubes (manufactured by Iwaki Glass) and 1 mg/ml of the anti-Flt-1 monoclonal antibody KM1730 was dispensed in 10 µl portions to carry out the reaction at room temperature for 15 minutes. After the reaction, the mixture was centrifuged and then washed and suspended in 300 µl of 0.1% BSA-PBS, and the suspension was mixed with 10 µl of 50 times-diluted PE-labeled goat anti-mouse IgG (manufactured by Dako) to carry out the reaction at room temperature for 15 minutes. After the reaction, the mixture was centrifuged and then washed and suspended in 300 µl of 0.1% BSA-PBS, and the suspension was mixed with 10 µl of each of the stock solutions of a PC5-labeled anti-CD34 antibody (class III, manufactured by Beckman Coulter) and an FITC-labeled anti-CD97 antibody (manufactured by Pharmingen) to carry out the reaction at room temperature for 15 minutes. After the reaction, the mixture was centrifuged, washed and then analyzed using a flow cytometer (Epics Elite, manufactured by Coulter). The results are shown in Figs. 10, 11 and 12.

Fig. 10 shows a cytogram on the cells obtained by culturing the CD34-positive cell by adding cytokine, in which the forward scattering value is plotted as the ordinate and the side-way scattering value as the abscissa. Since the monocyte indicated by M was the majority, differentiation of monocyte from the CD34-positive cell was confirmed.

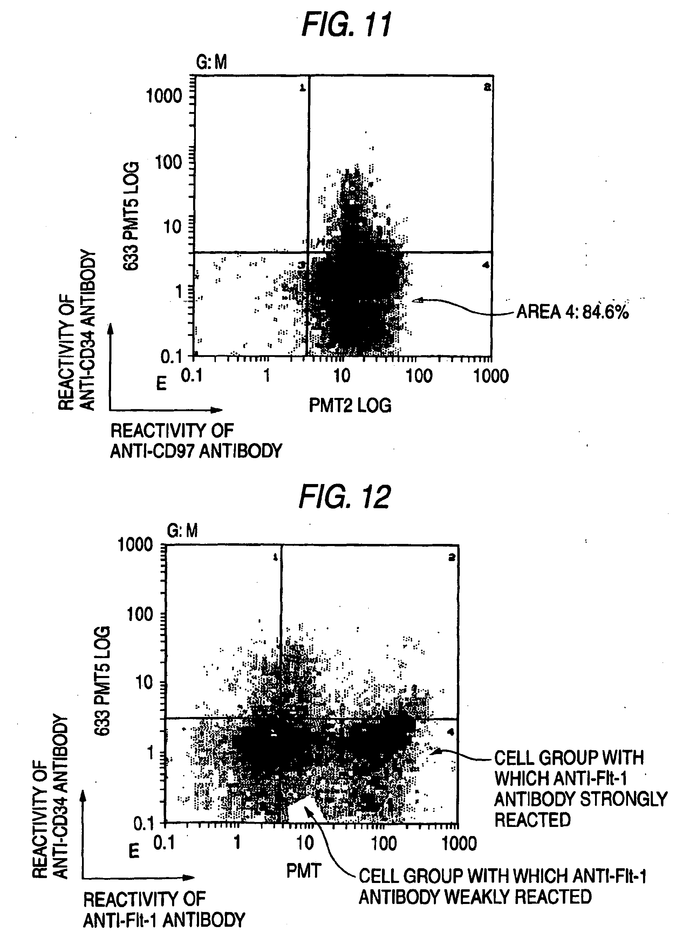

A result of the examination on the reactivity of the anti-CD34 antibody and the anti-CD97 antibody carried out by putting a gate on the M of Fig. 10 is shown in Fig. 11. The fraction of M was CD34-negative as the blood stem cell marker, and the CD97-positive cells as the monocyte marker were the majority of 84.6% (area 4), so that differentiation of CD34-positive blood stem cells into CD97-positive monocytes was confirmed.

A result of the examination on the reactivity of the anti-CD34 antibody and the anti-Flt-1 antibody carried out by putting a gate on the monocyte fraction indicated by M is shown in Fig. 12. The cells were divided into a cell group with which the anti-Flt-1 antibody strongly reacted and a cell group with which the anti-Flt-1 antibody weakly reacted. Reactivity with the anti-CD34 antibody was hardly observed in both of these groups. Accordingly, it was found that the cells differentiated into CD97-positive monocytes are divided into a cell group with which the anti-Flt-1 antibody strongly react and a cell group with which the anti-Flt-1 antibody weakly react.

Based on the above results, it was found that the anti-Flt-1 antibody does not react with CD34-positive blood stem cells in human cord blood, that the cells react with the anti-Flt-1 antibody when differentiated into monocytes and that the differentiated monocytes are divided into a cell group with which the anti-Flt-1 antibody strongly react and a cell group with which the anti-Flt-1 antibody weakly react.

### Example 3

### Inhibition of VEGF-dependent migration of human monocyte by anti-human Flt-1 monoclonal antibody:

### Separation of monocytes

A mononuclear corpuscle layer was recovered from peripheral blood of a healthy people in accordance with the method described in Example 1. The mononuclear corpuscle fraction was suspended in RPMI 1640 medium (manufactured by Nissui Pharmaceutical) containing 10% fetal calf serum (FCS, manufactured by JRH Biosciences) and 40 µg/ml of kanamycin (manufactured by Meiji Seika) (hereinafter referred to as "10% FCS-RPMI 1640 medium") to give a density of 2×10⁷ cells/10 ml. The mononuclear corpuscle suspension was added to a dish (bottom area: 225 cm²), which had been filled with 50 ml of the 10% FCS-RPMI 1640 medium and allowed to stand overnight at 4°C in advance, and cultured at 37°C for 2 hours in a CO₂ incubator to adhere monocytes to the dish. After culturing, the suspending cells were removed together with the medium and then a step in which 50 ml of PBS heated to 37°C is added and the suspending cells are removed was repeated twice. To the dish, 5 ml of PBS containing 0.2% EDTA-Na and 5% FCS was added, followed by incubating at room temperature for 20 minutes, and then the adhered monocytes were recovered using a pipette. The thus recovered monocytes were washed three times with 10% FCS-RPMI 1640 medium and then suspended to a density of 1×10⁶ cells/ml by adding 0.5% FCS-RPMI 1640 medium.

### Migration test

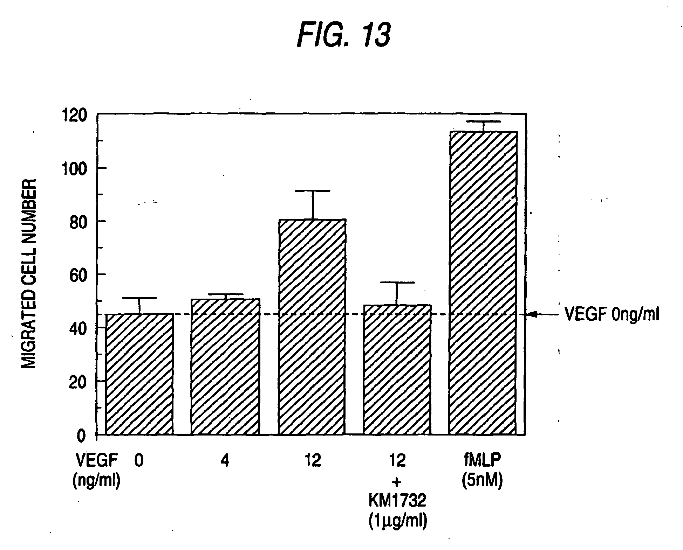

To the lower layer of Neuro Probe 48-well microchemotaxis Chamber (manufactured by Neuro Probe), 25 µl of 0.5% FCS-RPMI medium was added. A human recombinant VEGF expressed in an insect cell (Japanese Journal of Cancer Research, 88, 867 (1997)) was further added to give a final concentration of 0, 4 or 12 ng/ml, and an anti-human Flt-1 monoclonal antibody KM1732 was added to a part of the lower layer to give a final concentration of 1 µg/ml. As a positive control of monocyte migration accelerating active substance, 5 nM of fMLP (N-Formyl-Met-Leu-Phe, manufactured by Sigma) was added to the lower layer. A filter (5 µm-pore size PVP(-) polycarbonate filter, manufactured by Neuro Probe) was overlaid on the lower layer, and 50 µl of human monocyte suspended in the 0.5% FCS-RPMI 1640 medium to give a density of 1×10⁶ cells/ml was added to the upper layer of the filter. Two hours after culturing at 37°C in a CO₂ incubator, the filter was recovered and the cells migrated from the upper layer to the lower layer were stained with Diff-Quik (manufactured by Kokusai Reagent). By counting the number of cells in 10 split fields under a microscope of 400 x magnification, average value and standard deviation value of the number of cells in 10 split fields were calculated. The results are shown in Fig. 13.

As shown in Fig. 13, 5 nM of the positive control fMLP accelerated the migrated number of human monocytes to 2.5 times in comparison with the case of no addition of VEGF. Migration of the human monocytes was not accelerated by 4 ng/ml of VEGF, but the number of migrating monocyte was increased to 1.78 times by 12 ng/ml of VEGF. When the anti-human Flt-1 monoclonal antibody KM1732 was added at the time of the addition of 12 ng/ml of VEGF, the migrated number of monocytes was inhibited to the number at the time of no addition of VEGF (91.4% inhibition).

Based on the above, it was found that the migration of human monocytes accelerated by VEGF is completely inhibited by the anti-human Flt-1 monoclonal antibody.

### Reference Example 1

### 1. Isolation and analysis of cDNA encoding anti-human VEGF receptor Flt-1 mouse monoclonal antibody

### (1) Isolation of mRNA from hybridoma capable of producing anti-human VEGF receptor Flt-1 mouse monoclonal antibody

Using a mRNA extraction kit, Fast Track manufactured by In Vitrogen, and in accordance with the manual attached to the kit, mRNA was isolated from 1×10⁸ cells of each of hybridomas capable of producing anti-human VEGF receptor Flt-1 mouse monoclonal antibodies KM1732 and KM1750 (FERM BP-5698 and FERM BP-5700, respectively).

### (2) Production of heavy chain and light chain cDNA libraries of hybridoma capable of producing anti-human VEGF receptor Flt-1 mouse monoclonal antibody

Using cDNA Synthesis Kit (manufactured by Pharmacia Biotech) and in accordance with the manual attached to the kit, cDNA having *Eco*RI-*Not*I adapter on both termini was synthesized from 5 µg of each of KM1732 and KM1750 mRNA samples obtained in 1(1) of Reference Example 1. About 6 µg of each of the thus produced cDNA samples was dissolved in 10 µl of sterilized water and fractionated by agarose gel electrophoresis to recover about 0.1 µg of a cDNA fragment of about 1.5 kb which corresponds to the heavy chain (hereinafter referred to as "H chain") of the IgG type antibody and similar amount of a cDNA fragment of about 1.0 kb that corresponds to the light chain (hereinafter referred to as "L chain") thereof. Next, 0.1 µg of the cDNA fragment of about 1.5 kb or 0.1 µg of the cDNA fragment of about 1.0 kb and 1 µg of Lambda ZAPII Vector (a preparation obtained by digesting Lambda ZAPII Vector with *Eco*RI and then treating it with calf intestine alkaline phosphatase, manufactured by Stratagene) were dissolved in 11.5 µl of a T4 ligase buffer solution (manufactured by Takara Shuzo Co., Ltd.), and the thus prepared solution was mixed with 175 units of T4 DNA ligase (manufactured by Takara Shuzo Co., Ltd.) and incubated at 12°C for 24 hours and then at room temperature for 2 hours. Thereafter, a 4 µl portion of each of the reaction solutions was packaged in lambda phage using Gigapack Gold Packaging Kit (manufactured by Stratagene) in accordance with the conventional method (*Molecular Cloning*, 2nd edition), and *Escherichia coli* XL1-Blue (Biotechniques, 5: 376 (1987)) attached to Gigapack Gold Packaging Kit (manufactured by Stratagene) was infected with the resulting phage in accordance with the conventional method (Molecular Cloning, 2nd edition) to obtain about 4×10³ phage clones as each of the H chain cDNA libraries and L chain cDNA libraries of KM1732 and KM1750.

### (3) Cloning of cDNA encoding H chain and L chain of hybridoma capable of producing anti-human VEGF receptor Flt-1 mouse monoclonal antibody

Respective phage particles produced in 1(2) of Reference Example 1 were fixed on nitrocellulose filters in accordance with the conventional method (*Molecular Cloning*, 2nd edition). Each of the resulting nitrocellulose filters was treated in accordance with the manual attached to ECL direct nucleic acid labeling and detection systems (manufactured by Amersham) to obtain phage clones which strongly bonded to a probe cDNA encoding the constant region (hereinafter referred to as "C region") of mouse immunoglobulin (H chain is a fragment of mouse Cγ1 cDNA (Cell, 18: 559 (1979)) and L chain is a fragment of mouse Cκ cDNA (Cell, 22: 197 (1980))). Thereafter, the phage clones were transformed into plasmid pBluescript SK(-) in accordance with the manual attached to Lambda ZAPII Vector (manufactured by Stratagene), thus finally obtaining a recombinant plasmid KM1732HA2 containing cDNA encoding the H chain of KM1732, a recombinant plasmid KM1732L2-1 containing cDNA encoding L chain of KM1732, a recombinant plasmid KM1750H2-1 containing cDNA encoding the H chain of KM1750 and a recombinant plasmid KM1750L3-1 containing cDNA encoding L chain of KM1750. *Escherichia coli* XL1-Blue MRF'/KM1732HA2 containing the recombinant plasmid KM1732HA2, *Escherichia coli* XL1-Blue MRF'/KM1732L2-1 containing the recombinant plasmid KM1732L2-1, *Escherichia coli* XL1-Blue MRF'/KM1750H2-1 containing the recombinant plasmid KM1750H2-1 and *Escherichia coli* XL1-Blue MRF' /KM1750L3-1 containing the recombinant plasmid KM1750L3-1 have been deposited on May 14, 1998, in National Institute of Bioscience and Human Technology, Agency of Industrial Science and Technology (Higashi 1-1-3, Tsukuba-shi, Ibaraki, Japan) as FERM BP-6354, FERM BP-6352, FERM BP-6353 and FERM BP-6355, respectively.

### (4) Determination of variable region nucleotide sequence of cDNA encoding H chain and L chain of anti-human VEGF receptor Flt-1 mouse monoclonal antibody

Variable region (hereinafter referred to as "V region") nucleotide sequence of each of the cDNA samples obtained in 1(3) of Reference Example 1 encoding H chain and L chain of anti-human VEGF receptor Flt-1 mouse monoclonal antibody was determined by reacting 0.1 µg of each of the obtained plasmid samples in accordance with the manual attached to BigDye Terminator Cycle Sequencing Ready Reaction Kit (manufactured by Applied Biosystems) and then carrying out electrophoresis using ABI PRISM^{™} 377 (manufactured by Applied Biosystems). Based on the thus determined nucleotide sequence of each cDNA, amino acid sequences of the V region of H chain (hereinafter referred to as "VH") and the V region of L chain (hereinafter referred to as "VL") of KM1732 and KM1750 were determined. The nucleotide sequences of the VH of KM1732, the VL of KM1732, the VH of KM1750 and the VL of KM1750 are shown in SEQ ID NOs:1, 2, 3 and 4, respectively. The amino acid sequences of the VH of KM1732, the VL of KM1732, the VH of KM1750 and of the VL of KM1750 are shown in SEQ ID NOs:82, 83, 84 and 85, respectively.

### (5) Identification of CDR sequences of H chain and L chain of anti-human VEGF receptor Flt-1 mouse monoclonal antibody

Based on the amino acid sequences of the VH and VL of anti-human VEGF receptor Flt-1 mouse monoclonal antibody determined in 1(4) of Reference Example 1, CDR sequences of respective VH and VL were identified by comparing them with known antibody V region amino acid sequences (Sequences of Proteins of Immunological Interest). The amino acid sequences of CDR 1, 2 and 3 of the VH of KM1732 are shown in SEQ ID NOs:5, 6 and 7, respectively; those of CDR 1, 2 and 3 of the VL of KM1732 are shown in SEQ ID NOs:8, 9 and 10, respectively; those of CDR 1, 2 and 3 of the VH of KM1750 are shown in SEQ ID NOs:11, 12 and 13, respectively; and those of CDR 1, 2 and 3 of the VL of KM1750 are shown in SEQ ID NOs:14, 15 and 16, respectively.

### 2. Production of anti-human VEGF receptor Flt-1 human chimeric antibodies

Anti-human VEGF receptor Flt-1 human chimeric antibodies KM2532 and KM2550 originated from the anti-human VEGF receptor Flt-1 mouse monoclonal antibodies KM1732 and KM1750 having neutralization activity for human VEGF receptor Flt-1 were produced in the following manner.

### (1) Construction of expression vector pKANTEX1732 of anti-human VEGF receptor Flt-1 human chimeric antibody

The expression vector pKANTEX1732 of anti-human VEGF receptor Flt-1 human chimeric antibody was constructed in the following manner using a tandem cassette vector pKANTEX93 for humanized antibody expression use described in WO 97/10354 and the plasmids KM1732HA2 and KM1732L2-1 obtained in 1 of Reference Example 1.

A 3 µg portion of plasmid pBluescript SK(-) (manufactured by Stratagene) was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml bovine serum albumin (hereinafter referred to as "BSA") and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 2 µg of an ApaI-NotI fragment of about 2.95 kb. Next, 5 µg of the plasmid KM1732HA2 was added to 10 µl of a buffer solution comprising 20 mM Tris-HCl (pH 7.9), 10 mM magnesium acetate, 50 mM potassium acetate, 1 mM DTT and 100 µg/ml BSA, 10 units of a restriction enzyme *Nla*IV (manufactured by New England Biolabs) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme NotI (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Nla*IV-NotI fragment of about 0.41 kb. Next, a synthetic DNA having the nucleotide sequence of SEQ ID NO:17 or 18 was synthesized (manufactured by Sawady Technology), and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution (500 mM Tris-HCl (pH 7.6), 100 mM magnesium chloride and 50 mM DTT) and 2 µl of 10 mM ATP and then with 10 units of T4 polynucleotide kinase (manufactured by Takara Shuzo Co., Ltd.), and the mixture was allowed to react at 37°C for 30 minutes to phosphorylating the 5' end.

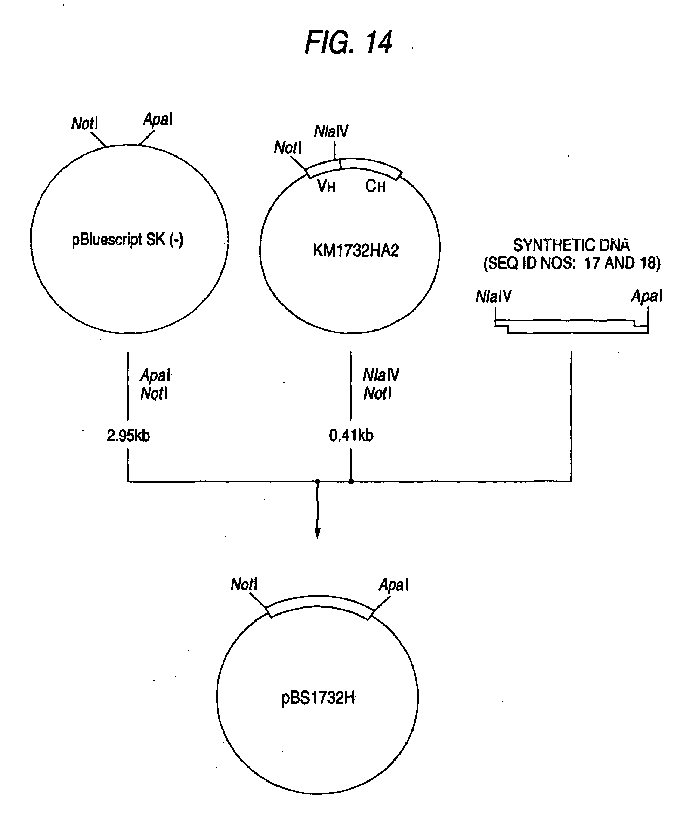

A 0.1 µg portion of the *Apa*I-*Not*I fragment derived from plasmid pBluescript SK(-), 0.1 µg of the *Nla*IV-*Not*I fragment derived from plasmid KM1732HA2 and 0.05. µg of the phosphorylated synthetic DNA, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pBS1732H shown in Fig. 14.

Next, 3 µg portion of plasmid phKM1259LV0 described in WO 97/10354 was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 2 µg of an *Eco*RI-*Spl*I fragment of about 2.95 kb. Next, 5 µg of the plasmid KM1732L2-1 was added to 10 µl of a buffer solution comprising 10 mM Tris-HCl (pH 7.5), 100 mM magnesium chloride and 1 mM DTT, 10 units of a restriction enzyme *Mbo*II (manufactured by TOYOBO CO., LTD.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride and 1 mM DTT, 10 units of a restriction enzyme EcoRI. (manufactured by Takara Shuzo Co., Ltd.) were further added thereto, and then the reaction was carried out at 37°C for 1 hour. The reaction solution was fractionated by agarose gel electrophoresis to recover about 0.5 µg of a MboII-EcoRI fragment of about 0.38 kb. Next, a synthetic DNA having the nucleotide sequence of SEQ ID NO:19 or 20 was synthesized (manufactured by Sawady Technology), and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution (500 mM Tris-HCl (pH 7.6), 100 mM magnesium chloride and 50 mM DTT) and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

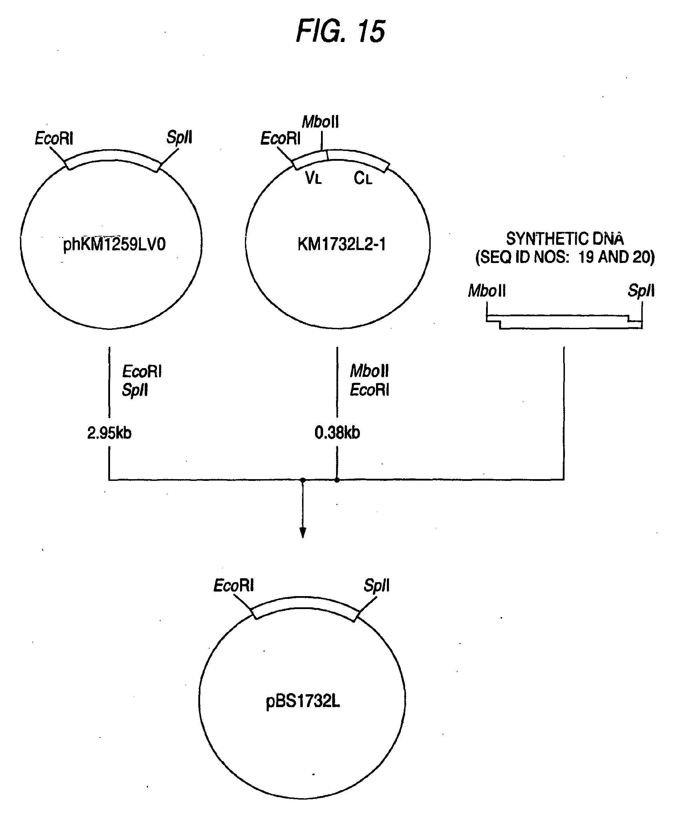

A 0.1 µg portion of the *Eco*RI-*Spl*I fragment derived from plasmid phKM1259LV0, 0.1 µg of the *Mbo*II-*Eco*RI fragment derived from plasmid KM1732L2-1 and 0.05 µg of the phosphorylated synthetic DNA, obtained by the just described procedures, were added to 10 µl in total volume of sterilized water and ligated using DNA Ligation Kit Ver. 2 (manufactured by Takara Shuzo Co., Ltd.) in accordance, with the manual attached thereto. *Escherichia coli* DH5α (manufactured by TOYOBO CO., LTD.) was transformed with the thus obtained recombinant plasmid DNA solution to obtain a plasmid pBS1732L shown in Fig. 15.

Next, 3 µg of the vector pKANTEX93 for humanized antibody expression was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 1 µg of an *Apa*I-*Not*I fragment of about 12.75 kb. Next, 5 µg of the plasmid pBS1732H obtained in the foregoing was added to 10 µl of a buffer solution comprising 10 mM Tris-HCl (pH 7.5), 10 mM magnesium chloride and 1 mM DTT, 10 units of a restriction enzyme ApaI (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme NotI (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Apa*I-*Not*I fragment of about 0.46 kb.

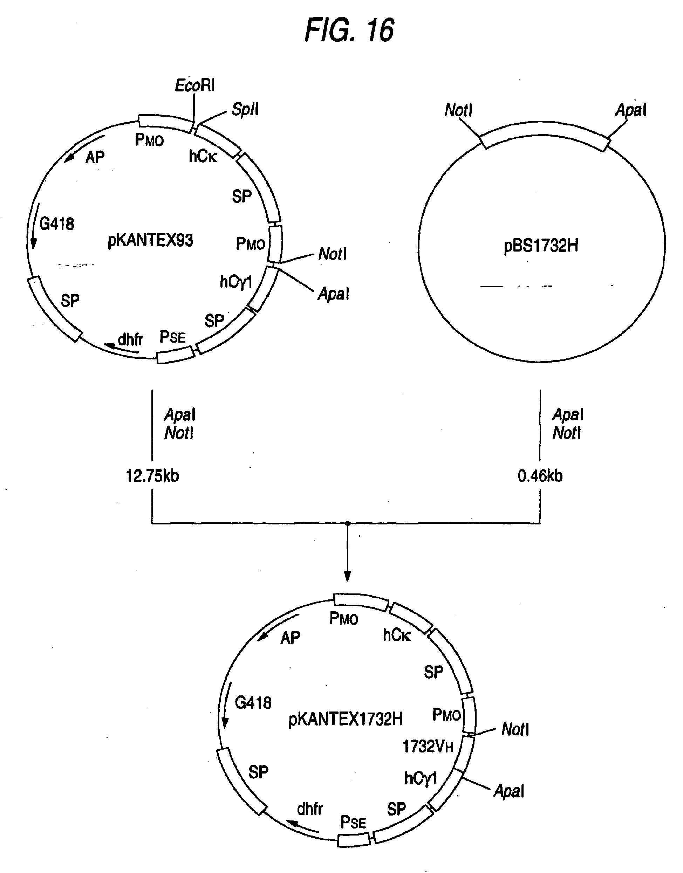

A 0.1 µg portion of the *Apa*I-*Not*I fragment derived from the vector pKANTEX93 for humanized antibody expression and 0.1 µg of the *Apa*I-*Not*I fragment derived from the plasmid pBS1732H, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1732H shown in Fig. 16.

Next, 3 µg of the just obtained plasmid pKANTEX1732H was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme EcoRI (manufactured by Takara Shuzo Co., Ltd.) and 10 units of a restriction enzyme SplI (manufactured by Takara Shuzo Co., Ltd.) which were further added thereto, and then the reaction was carried out at 37°C for 1 hour. The reaction solution was fractionated by agarose gel electrophoresis to recover about 1 µg of an EcoRI-SplI fragment of about 13.20 kb. Next, 5 µg of the plasmid pBS1732L obtained in the foregoing was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Eco*RI-*Spl*I fragment of about 0.39 kb.

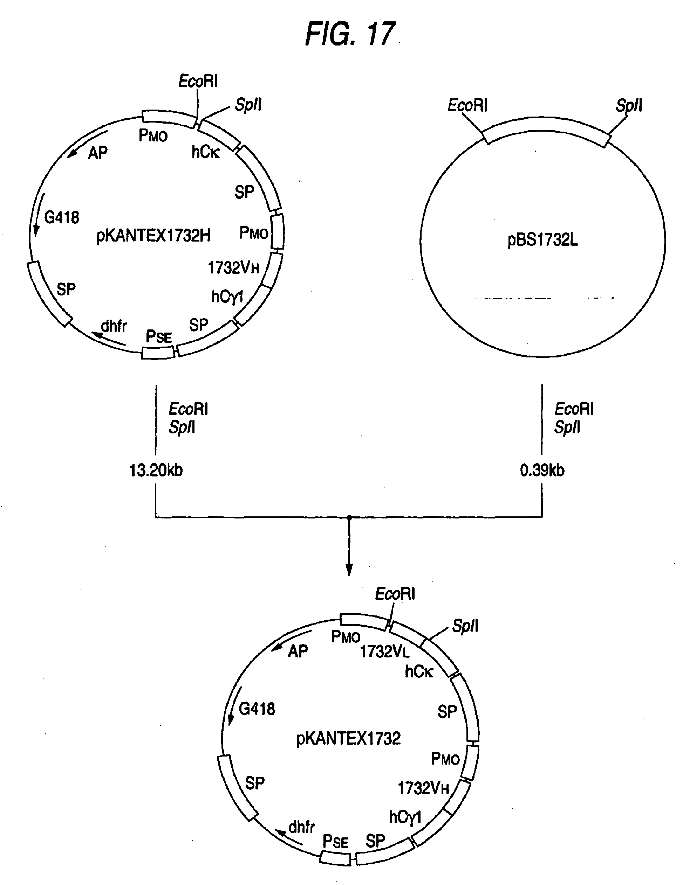

A 0.1 µg portion of the *Eco*RI-*Spl*I fragment derived from the plasmid pKANTEX1732H and 0.1 µg of the *Eco*RI-*Spl*I fragment derived from the plasmid pBS1732L, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1732 shown in Fig. 17.

### (2) Construction of expression vector pKANTEX1750 of anti-human VEGF receptor Flt-1 human chimeric antibody

The expression vector pKANTEX1750 of anti-human VEGF receptor Flt-1 human chimeric antibody was constructed in the following manner using a tandem cassette vector pKANTEX93 for humanized antibody expression described in WO 97/10354 and the plasmids KM1750H2-1 and KM1750L3-1 obtained in 1 of Reference Example 1.

A 5 µg portion of the plasmid KM1750H2-1 was added to 10 µl of a buffer solution comprising 33 mM Tris-HCl (pH 7.9), 10 mM magnesium acetate, 66 mM potassium acetate and 100 µg/ml BSA, 10 units of a restriction enzyme Alw26I (manufactured by New England Biolabs) were further added thereto, and then the reaction was carried out at 37°C for 1 hour. The reaction solution was precipitated with ethanol the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Alw*26I-*Not*I fragment of about 0.41 kb. Next, a synthetic DNA having the nucleotide sequence of SEQ ID NO:21 or 22 was synthesized (manufactured by Sawady Technology), and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution (500 mM Tris-HCl (pH 7.6), 100 mM magnesium chloride and 50 mM DTT) and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

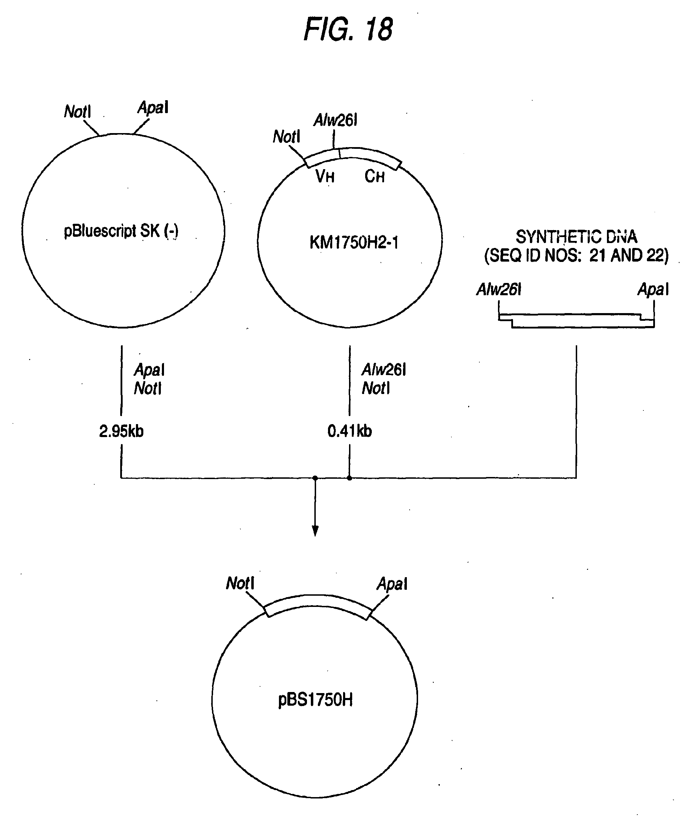

A 0.1 µg portion of the *Apa*I-*Not*I fragment derived from plasmid pBluescript SK(-) in 2(1) of Reference Example 1, 0.1 µg of the *Alw*26I-*Not*I fragment derived from plasmid KM1750H2-1 and 0.05 µg of the phosphorylated synthetic DNA were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pBS1750H shown in Fig. 18.

Next, 5 µg of the plasmid KM1750L3-1 was added to 10 µl of a buffer solution comprising 100 mM Tris-HCl (pH 8.8), 440 mM sodium chloride, 12 mM magnesium chloride, 14 mM 2-mercaptoethanol and 200 µg/ml BSA, 10 units of a restriction enzyme *Mae*II (manufactured by Boehringer-Mannheim) were further added thereto, and then the reaction was carried out at 50°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride and 1 mM DTT, 10 units of a restriction enzyme *Eco*RI (manufactured by Takara Shuzo Co., Ltd.) were further added thereto, and then the reaction was carried out at 37°C for 1 hour. The reaction solution was fractionated by agarose gel electrophoresis to recover about 0.5 µg of a *Mae*II-*Eco*RI fragment of about 0.38 kb. Next, a synthetic DNA having the nucleotide sequence of SEQ ID NO:23 or 24 was synthesized (manufactured by Sawady Technology), and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution (500 mM Tris-HCl (pH 7.6), 100 mM magnesium chloride and 50 mM DTT) and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

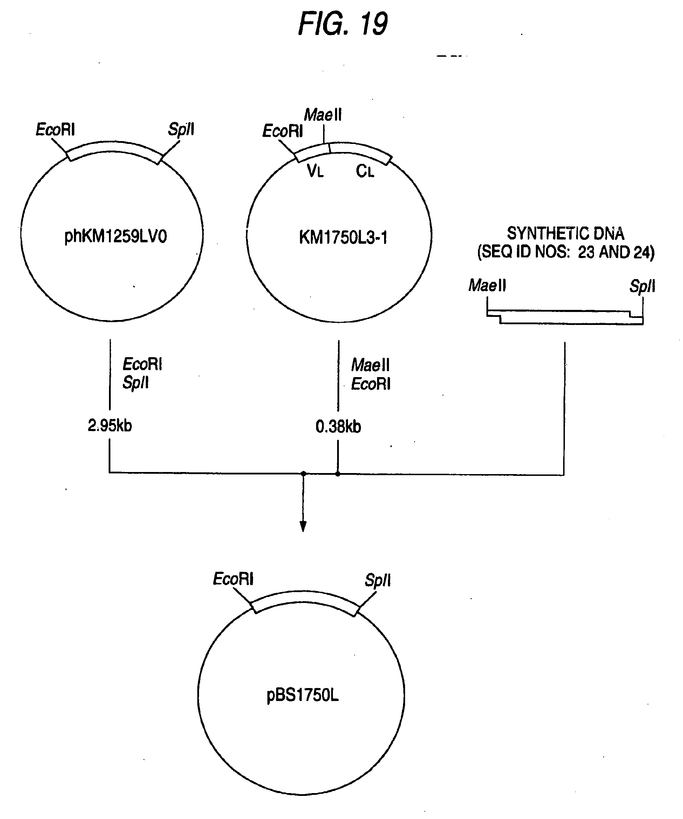

A 0.1 µg portion of the *Eco*RI-*Spl*I fragment derived from plasmid pphRM1259LV0 in 2(1) of Reference Example 1, 0.1 µg of the *Mae*II-*Eco*RI fragment derived from plasmid KM1750L3-1 and 0.05 µg of the phosphorylated synthetic DNA were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pBS1750L shown in Fig. 19.

Next, 5 µg of the plasmid pBS1750H obtained in the foregoing was added to 10 µl of a buffer solution comprising 10 mM Tris-HCl (pH 7.5), 10 mM magnesium chloride and 1 mM DTT, 10 units of a restriction enzyme ApaI (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Apa*I-*Not*I fragment of about 0.46 kb.

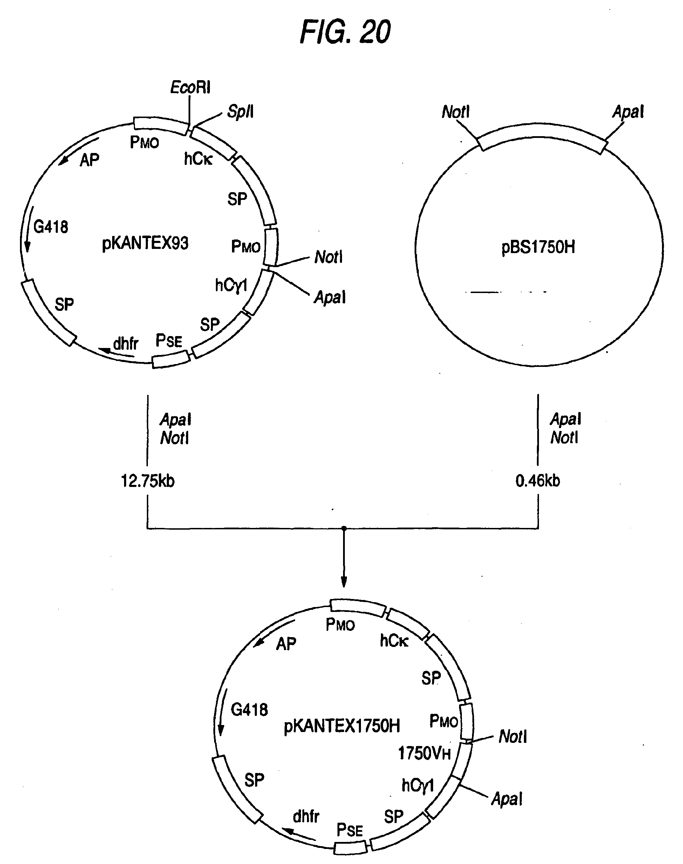

A 0.1 µg portion of the *Apa*I-*Not*I fragment derived in 2(1) of Reference Example 1 from a tandem cassette vector pKANTEX93 for humanized antibody expression and 0.1 µg of the *Apa*I-*Not*I fragment derived from plasmid pBS1750H were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1750H shown in Fig. 20.

Next, 3 µg of the just obtained plasmid pKANTEX1750H was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 1 µg of an EcoRI-SplI fragment of about 13.20 kb. Next, 5 µg of the plasmid pBS1750L obtained in the foregoing was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme EcoRI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for- 1 hour. The reaction solution was fractionated by agarose gel electrophoresis to recover about 0.5 µg of an *Eco*RI-*Spl*I fragment of about 0.39 kb.

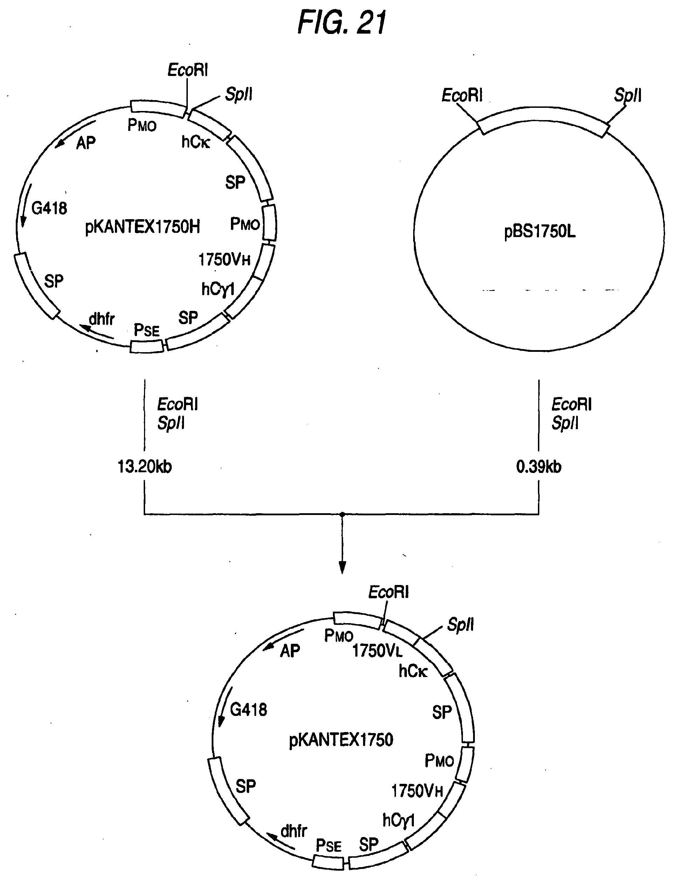

A 0.1 µg portion of the EcoRI-SplI fragment derived from plasmid pKANTEX1750H and 0.1 µg of the *Eco*RI-*Spl*I fragment derived from plasmid pBS1750L, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1750 shown in Fig. 21.

### (3) Expression of anti-human VEGF receptor Flt-1 human chimeric antibodies in rat myeloma YB2/0 cells (ATCC CRL1581) using pKANTEX1732 and pKANTEX1750

Introduction of anti-human VEGF receptor Flt-1 human chimeric antibody expression vectors pKANTEX1732 and pKANTEX1750 into YB2/0 cells was carried out by electroporation in accordance with the method of Miyaji et al. (Cytotechnology, 3: 133 (1990)).

A 5 µg portion of each of pKANTEX1732 and pKANTEX1750 obtained in 2(1) and 2(2) of Reference Example 1 was introduced into 4×10⁶ YB2/0 cells which were subsequently suspended in 40 ml of RPMI 1640-FCS(10) medium (RPMI 1640 medium (manufactured by Nissui Pharmaceutical) containing 10% fetal calf serum (FCS)) and dispensed in 200 µl portions into wells of a 96 well microtiter plate (manufactured by Sumilon). After 24 hours of culturing at 37°C in a 5% CO₂ incubator, geneticin (hereinafter referred to as "G 418", manufactured by Gibco) was added to a final concentration of 0.5 mg/ml, and the culturing was continued for 1 to 2 weeks. When colonies of G 418-resistant transformants were formed and became confluent, the culture supernatant was recovered from each well, and the activity of anti-human VEGF receptor Flt-1 human chimeric antibody in the supernatant was measured by the following enzyme immunoassay.

### Enzyme immunoassay

Soluble human VEGF receptor Flt-1 7N was prepared in accordance with the method of Tanaka et al. (Japanese Journal of Cancer Research, 88: 867 (1997)). The soluble human VEGF receptor Flt-1 7N diluted to 1 µg/ml with PBS was dispensed in 50 µl portions into wells of a 96 well plate for EIA (manufactured by Greiner) and allowed to stand overnight at 4°C for its adsorption. After washing with PBS, 100 µl of PBS containing 1% BSA (hereinafter referred to as "1% BSA-PBS") was added to each well, and the remaining active residues was blocked by carrying out the reaction at room temperature for 1 hour. After discarding 1% BSA-PBS, the culture supernatant of each transformant was dispensed in 50 µl portions into the resulting wells to carry out the reaction at room temperature for 1 hour. After washing the plate with PBS containing 0.05% Tween 20 (hereinafter referred to as "0.05% Tween-PBS"), a peroxidase-labeled anti-human IgG antibody (manufactured by American Quarex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

In order to increase productivity, each of the transformants which showed the anti-human VEGF receptor Flt-1 human chimeric antibody activity in the culture supernatant was suspended in RPMI 1640-FCS(10) medium supplemented with 0.5 mg/ml of G 418 and 50 nM of methotrexate (hereinafter referred to as "MTX", manufactured by Sigma) and cultured at 37°C for 1 to 2 weeks in a 5% CO₂ incubator to induce transformants having 50 nM MTX resistance. When the transformants became confluent in the wells, the anti-human VEGF receptor Flt-1 human chimeric antibody activity in the culture supernatants was measured by the aforementioned enzyme immunoassay. Transformants in which the activity was found were cultured similarly by further increasing the MTX concentration to 100 nM and then to 200 nM to obtain transformants which can grow in RPMI 1640-FCS(10) medium containing 0.5 mg/ml of G 418 and 200 nM of MTX and are able to produce large amounts of anti-human VEGF receptor Flt-1 human chimeric antibodies. The thus obtained transformants were subjected to two times of cloning by limiting dilution to obtain final transformant cell lines capable of producing anti-human VEGF receptor Flt-1 human chimeric antibodies. KM2532 can be cited as an example of the transformant cell line obtained by introducing the expression vector pKANTEX1732, namely a transformant which produces an anti-human VEGF receptor Flt-1 human chimeric antibody originated from the anti-human VEGF receptor Flt-1 mouse monoclonal antibody KM1732, and the anti-human VEGF receptor Flt-1 human chimeric antibody produced by this transformant was named KM2532. Also, KM2550 can be cited as an example of the transformant cell line obtained by introducing the expression vector pKANTEX1750, namely a transformant which produces an anti-human VEGF receptor Flt-1 human chimeric antibody originated from the anti-human VEGF receptor Flt-1 mouse monoclonal antibody KM1750, and the anti-human VEGF receptor Flt-1 human chimeric antibody produced by this transformant was named KM2550. The productivity of anti-human VEGF receptor Flt-1 human chimeric antibodies by the thus cloned transformants was found to be about 5 µg/10⁶ cells/24 hours.

### (4) Purification of anti-human VEGF receptor Flt-1 human chimeric antibodies from culture supernatants

Each of the anti-human VEGF receptor Flt-1 human chimeric antibody producing cell lines KM2532 and KM2550 obtained in 2(3) of Reference Example 1 was suspended in GIT medium (manufactured by Japan Pharmaceutical) supplemented with 0.5 mg/ml of G 418 and 200 nM of MTX, to a density of 1×10⁵ to 2×10⁵ cells/ml, and dispensed in 200 ml portions into a total of five 175 cm² flasks (manufactured by Greiner). When the cells became confluent after 5 to 7 days of culturing at 37°C in a 5% CO₂ incubator, about 1.0 liter of each culture supernatant was recovered. A column was packed with about 1 ml of ProSep A (manufactured by Bioprocessing) and washed with 10 ml of 1 M glycine-0.15 M NaCl (pH 8.6) at a flow rate of 1 ,ml/minute. After the washing, 1,700 ml or 1,800 ml of the culture supernatant prepared in the above containing the anti-human VEGF receptor Flt-1 human chimeric antibody KM2532 or KM2550 was passed through the ProSep A column at a flow rate of 70 ml/hour. This was further washed with 10 ml of 1 M glycine-0.15 M NaCl (pH 8.6) at a flow rate of 1 ml/minute, again washed stepwise with 4 ml of 50 mM citrate buffer of pH 6, 5 and 4, and then 7 ml of 50 mM citrate buffer (pH 3.0) was passed through the column to elute the human chimeric antibody. As a result, 0.4 mg and 0.3 mg of the anti-human VEGF receptor Flt-1 human chimeric antibodies KM2532 and KM2550, respectively, were obtained.

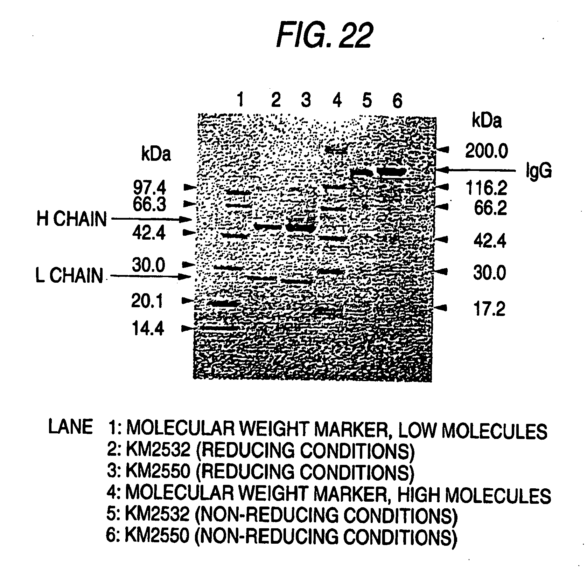

The thus purified anti-human VEGF receptor Flt-1 human chimeric antibodies KM2532 and KM2550 were analyzed by SDS-PAGE in accordance with a known method (Anticancer Research, 12: 1121 (1992)). As the gel, 5 to 20% gradient gel (manufactured by Atto Corp.) was used, a low molecular weight protein molecular marker "Daiichi" II and a high molecular weight protein molecular marker "Daiichi" III (both manufactured by Daiichi Pure Chemicals) were used as molecular markers, and 2.5 µg as protein per lane of each of the anti-human VEGF receptor Flt-1 human chimeric antibodies KM2532 and KM2550 was electrophoresed under reducing and non-reducing conditions and stained with Coomassie Brilliant Blue. The results are shown in Fig. 22. The human chimeric antibodies KM2532 and KM2550 showed a band of IgG at a position corresponding to a molecular weight of about 150 kDa under the non-reducing condition, and a band of H chain at about 50 kDa and a band of L chain at about 25 kDa were found under the reducing condition. This result coincided with that the IgG type antibody is separated into two H chains and L chains under the reducing conditions caused by the cutting of intermolecular disulfide bond and present as a molecule of 150 kDa, and it is shown that the human chimeric antibodies KM2532 and 2550 are antibody molecules having a correct structure.

### (5) Binding activity of anti-human VEGF receptor Flt-1 human chimeric antibodies upon human VEGF receptor Flt-1

Binding activity of the purified anti-human VEGF receptor Flt-1 human chimeric antibodies KM2532 and KM2550 upon human VEGF receptor Flt-1 was confirmed by the following procedure.

Soluble human VEGF receptor Flt-1 7N was prepared in accordance with the method of Tanaka et al. (Japanese Journal of Cancer Research, 88: 867 (1997)).

First, the binding activity was examined using an enzyme immunoassay by fixing amount of the soluble human VEGF receptor Flt-1 7N to be adsorbed on a 96 well plate for EIA and varying concentration of the human chimeric antibody to be added. The soluble human VEGF receptor Flt-1 7N diluted to 1 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of from 0.152 to 333 ng/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

The results are shown in Fig. 23 (A). The anti-human VEGF receptor Flt-1 human chimeric antibodies KM2532 and KM2550 bound to the human VEGF receptor Flt-1 7N in an antibody concentration dependent manner. In addition, the anti-human VEGF receptor Flt-1 human chimeric antibodies KM2532 and KM2550 showed almost the same binding activity.

Next, the binding activity of human chimeric antibodies was examined using an enzyme immunoassay by varying amount of the soluble human Flt-1 7N to be adsorbed on a 96 well plate for EIA. The soluble human VEGF receptor Flt-1 7N diluted to a concentration of from 0.04 to 10 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 10 µg/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

The results are shown in Fig. 23 (B). Each of the anti-human VEGF receptor Flt-1 human chimeric antibodies KM2532 and KM2550 showed its binding activity dependently upon the concentration of the soluble human VEGF receptor Flt-1 7N adsorbed on the plate. In addition, the anti-human VEGF receptor Flt-1 human chimeric antibodies KM2532 and KM2550 showed almost the same binding activity.

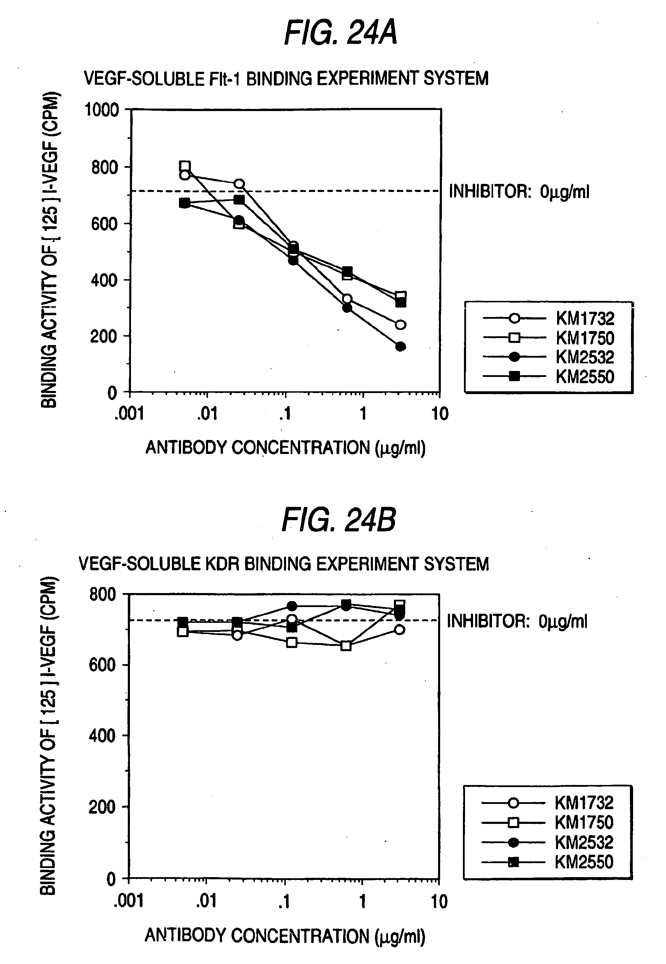

Next, the activity of anti-human VEGF receptor Flt-1 human chimeric antibodies to inhibit binding of human VEGF with human VEGF receptor Flt-1 was examined in the following manner. Methanol was dispensed in 100 µl portions into wells of a 96 well multi-screen IP plate (manufactured by Millipore) to give hydrophilic nature to the PVDF membrane on the bottom of the plate. After washing the plate with water, the soluble human VEGF receptor Flt-1 7N which had been diluted to 0.2 µg/ml with PBS was dispensed in 50 µl portions into the wells and allowed to stand overnight at 4°C for its adsorption. After washing the plate with PBS, 50 µl of 1% BSA-PBS was added to each well, and the remaining active residues was blocked by carrying out the reaction at room temperature for 1 hour. After washing the plate with PBS, each of the purified anti-human VEGF receptor Flt-1 human chimeric antibodies KM2532 and KM2550, and purified anti-human VEGF receptor Flt-1 mouse monoclonal antibodies KM1732 and KM1750, which had been diluted with 1% BSA-PBS to a concentration of from 0.004 to 2 µg/ml, was dispensed in 50 µl portions into the resulting wells and then 3 ng/ml of ¹²⁵I-labeled human VEGF (manufactured by Amersham) was dispensed in 50 µl portions into the wells to carry out the reaction at room temperature for 1.5 hours. After washing the plate with 0.05% Tween-PBS, the wells were dried at 50°C, Microscinti-O (manufactured by Packard) was dispensed in 30 µl portions into the wells and then radioactivity of the ¹²⁵I-labeled human VEGF bonded on each well was measured using Top Count (manufactured by Packard). The results are shown in Fig. 24. As shown in Fig. 24, each of the anti-human VEGF receptor Flt-1 human chimeric antibodies KM2532 and KM2550 inhibited binding of the human VEGF with the human VEGF receptor Flt-1 in an antibody concentration dependent manner. In addition, the activity of the anti-human VEGF receptor.Flt-1 human chimeric antibodies KM2532 and KM2550 to inhibit binding of the human VEGF with the human VEGF receptor Flt-1 was almost the same as that of the anti-human VEGF receptor Flt-1 mouse monoclonal antibodies KM1732 and KM1750, thus showing that the human chimeric antibodies maintain the activity of mouse monoclonal antibodies.

### (6) Analysis of epitope which is recognized by anti-human VEGF receptor Flt-1 human chimeric antibody

### (6-1) Preparation of soluble human VEGF receptor KDR 7N and soluble human VEGF receptor chimeric protein Flt-1 7N.K2

Soluble human VEGF receptor KDR 7N and soluble human VEGF receptor chimeric protein Flt-1 7N.K2 were prepared in the following manner.

### (6-2) Construction of soluble human VEGF receptor KDR 7N expression vector

A vector was produced in the following manner for the expression of 19 amino acids which constitute the signal peptide of human VEG receptor KDR, a soluble human VEGF receptor KDR fragment (hereinafter referred to as "soluble human VEGF receptor KDR 7N") which corresponds to 738th position from the N-terminal amino acid of the mature protein and two amino acid residues derived from a linker.

The soluble human VEGF receptor KDR 7N consists of the seven immunoglobulin-like moieties from the N-terminal side of the extracellular region of soluble human VEGF receptor KDR.

A cDNA clone which encodes full length cDNA of human VEGF receptor KDR, BCMGS-neo-KDR (A. Sawano et al., Cell Growth & Differentiation, 7: 213-221 (1996)), was digested with EcoRI, and a fragment of about 2.8 kb encoding the extracellular region and membrane-binding region of KDR was integrated into the EcoRI site of pUC18 to produce pUC-KDR. The pUC-KDR was digested with XhoI and treated with Klenow and then an *Xba*I linker (SEQ ID NO:53) was inserted to produce pUC-KDR-Xb. An *Xba*I-*Bam*HI fragment (2.3 kbp) of pUC-KDR-Xb was inserted into the *Xba*I/*Bam*HI site of pBluescriptII KS(+) to prepare an *Sph*I-*Bam*HI fragment (5.2 kbp) into which were subsequently inserted synthetic linkers (SEQ ID NOs:54 and 55) containing SnaBI site to produce pBS-KDR-Xb-S.

The thus obtained pBS-KDR-Xb-S was digested with *Sna*BI/*Bam*HI, and synthetic linkers (SEQ ID NOs:56 and 57) containing terminal codon and NotI site were inserted to produce pBS-KDR-Xb-S-N. An *Xba*I-*Not*I fragment (2.3 kb) of pBS-KDR(Xb)-S-N was inserted into downstream 5' side XbaI and 3' side NotI site of the transcription initiation point of polyhedrin gene of baculovirus recombinant plasmid pVL1393 to obtain soluble human VEGF receptor KDR 7N expression vector pVL-KDR-7N.

### (6-3) Construction of soluble human VEGF receptor chimeric protein Flt-1 7N.K2 expression vector

A vector was constructed in the following manner for the expression of a chimeric protein Flt-1.K2 in which an amino acid sequence of from the 100th amino acid to the 204th amino acid which corresponds to the second immunoglobulin-like moiety counting from the N-terminal side of the soluble human VEGF receptor Flt-1 7N that corresponds to 750 amino acids from the N-terminal containing the signal sequence of human VEGF receptor Flt-1 was replaced with an amino acid sequence of from the 95th amino acid to the 199th amino acid which corresponds to the second immunoglobulin-like moiety of the soluble human VEGF receptor KDR, via linkers of two amino acids (Gly-Ala and Gly-Thr).

An *Eco*RI/*Hind*III fragment (1.9 kb) of human Flt-1 cDNA (Shibuya et al., Oncogene, 5: 519 (1990)) was inserted into the EcoRI/HindIII site of a vector M13mp1 to produce pM13-flt. *Escherichia coli* XL1Blue was infected with the thus obtained pM13-flt and cultured, and ssbNA was prepared from the resulting culture supernatant in accordance with a manual of a site-specific mutation introduction kit Mutan K supplied by Takara Shuzo. Using an oligonucleotide of 56 bases (SEQ ID NO:58) and a site-specific mutation introducing kit Mutan K (manufactured by Takara Shuzo Co., Ltd.), site-specific mutation was carried out using the thus obtained ssDNA as the template to produce a plasmid pM13-flt'-D2N containing a Flt-1 mutant gene in which the region encoding the second immunoglobulin-like moiety of the extracellular region of Flt-1 was deleted. Next, using 10 ng of BCMGS-neo-KDR DNA (A. Sawano et al., Cell Growth & Differentiation, 7: 213 (1996)) as the template, PCR was carried out by adding 2.5 units of TaqDNA polymerase to 100 ml of a solution containing 10 pmol of each of primers having nucleotide sequences shown in SEQ ID NOs:59 and 60, TaqDNA polymerase buffer and 10 mM deoxynucleotide triphosphate. A serial reaction of 90 seconds at 95°C, 90 seconds at 50°C and 90 seconds at 72°C was repeated 30 times, and the thus amplified DNA fragment encoding the second immunoglobulin-like moiety counting from the N-terminal side of the extracellular region of KDR was recovered. This fragment was digested with *Nar*I/*Kpn*I to obtain an *Nar*I/*Kpn*I fragment of 340 bp. The thus obtained fragment and an *Sma*I/*Nar*I fragment (0.5 kb) of pM13-flt'-D2N were inserted into the *Sma*I/*Kpn*I site of pBluescriptII to produce pBS-flt-1'-KDR2N. Thereafter, a *Bam*H/*Kpn*I fragment (0.8 kb) of pBS-flt-1'-KDR2N, a *Kpn*I/*Mun*I fragment (80 b) of pM13-flt'-D2N and an *Mun*I/*Not*I fragment (1.5 kb) of pVL-flt-1 were inserted into the *Bam*HII/*Not*I site of pVL1393 to obtain a plasmid PVL-fkf which carries the Flt-1 K2 gene.

### (6-4) Production of recombinant virus for the expression of soluble human VEGF receptor KDR 7N and soluble human VEGF receptor chimeric protein Flt-1 7N.K2 in insect cells

In order to produce a protein in insect cells, it is necessary to obtain a recombinant virus into which the gene of interest is integrated, and such a production process comprises a step in which a cDNA called transfer vector which encodes the protein of interest is inserted into a special plasmid and a subsequent step in which a wild type virus and the transfer vector are co-transfected into insect cells to obtain the recombinant virus by homologous recombination, These steps were carried out in the following manner using BaculoGold Starter Kit manufactured by Fermingen (Product No. PM-21001K) in accordance with the manual attached thereto.

Recombinant baculovirus was produced in the following manner by a lipofectin method (Protein, Nucleic Acid and Enzyme, 37: 2701 (1992)), by introducing filamentous baculovirus DNA (BaculoGold baculovirus DNA, manufactured by Fermingen) and transfer vector DNA into cells of an insect cell line Sf9 (manufactured by Fermingen) which had been cultured using TMN-FH insect medium (manufactured by Fermingen).

A 1 µg portion of the expression vector produced in (6-2) and 20 ng of the filamentous baculovirus DNA were dissolved in 12 µl of distilled water, and a mixture consisting of 6 µl of lipofectin and 6 µl of distilled water was added to the resulting solution and allowed to stand at room temperature for 15 minutes. Separately from this, **1x10⁶** of Sf9 cells were suspended in 2 ml of Sf900-II medium (manufactured by Gibco) and put into a plastic Petri dish for cell culture having a diameter of 35 mm. Entire portion of the aforementioned mixture solution of plasmid DNA, filamentous baculovirus DNA and lipofectin was added to the dish, the cells were cultured at 27°C for 3 days and then a 1 ml portion of the culture supernatant containing recombinant virus was collected. A 1 ml portion of fresh Sf900-II medium was added to the resulting Petri dish, and the cells were further cultured at 27°C for 3 days to obtain 1.5 ml of the culture supernatant containing recombinant virus. Thereafter, the same procedure was repeated using the expression vector obtained in (6-3).

Next, in order to use it in protein expression, each of the thus obtained recombinant viruses was grown in the following manner.

A total of 2×10⁷ Sf9 cells were suspended in 10 ml of Sf900-II medium, put into a 175 cm² flask (manufactured by Greiner) and then allowed to stand at room temperature for 1 hour to adhere the cells to the flask. Thereafter, the supernatant was discarded and 15 ml of fresh TMN-FH insect medium and a 1 ml of portion of the aforementioned culture supernatant containing recombinant virus were added to the flask to carry out 3 days of culturing at 27°C. After the culturing, the supernatant was centrifuged at 1,500 × g for 10 minutes to remove cells to obtain a recombinant virus solution to be used in protein expression.

The titer of virus in the thus obtained recombinant virus solution was calculated in accordance with the method described in BaculoGold Starter Kit Manual (manufactured by Farmigen).

A total of 6×10⁶ Sf9 cells were suspended in 4 ml of Sf900-II medium, put into a plastic Petri dish for cell culture having a diameter of 60 mm and then allowed to stand at room temperature for 1 hour to adhere the cells to the dish. Next, the supernatant was discarded, 400 µl of fresh Sf900-II medium and the aforementioned recombinant virus solution diluted 1,000 times with Sf900-II medium in advance were added to the dish and allowed to stand at room temperature for 1 hour, and then the medium was discarded and 5 ml of a medium containing 1% low melting point agarose (Agarplaque Agarose, manufactured by Farmigen) (a medium produced by mixing 1 ml of sterilized 5% Agarplaque Agarose aqueous solution with 4 ml of TMN-FH insect medium and kept at 42°C) was poured into the Petri dish. After 15 minutes of standing at room temperature, the Petri dish was sealed with a vinyl tape to prevent drying and put into a sealing type plastic container to carry out 6 days of culturing at 27°C. A 1 ml portion of PBS containing 0.01% Neutral Red was added to the Petri dish, and the culturing was continued for 1 day to count the number of formed plaques. It was found by the above procedure that each of the recombinant virus solutions contained about 1×10⁷ plaque forming units (hereinafter referred to as "PFU")/ml of viral particles.

### (6-5) Expression of soluble human VEGF receptor KDR 7N and soluble human VEGF receptor chimeric protein Flt-1 7N.K2 in insect cells and their purification

The soluble human VEGF receptor KDR 7N and soluble human VEGF receptor chimeric protein Flt-1 7N.K2 were obtained in the following manner. A total of 4×10⁷ High Five cells were suspended in 30 ml of EX-CELL^{™} 400 medium (manufactured by JRH Bioscience) contained in a 175 cm² flask (manufactured by Greiner) and adhered to the flask by allowing them to stand at room temperature for 1 hour. A 1 ml portion of a solution containing about 1×10⁸ to 3×10⁸ PFU/ml of the transfer vector-originated recombinant virus obtained in (6-3) and (6-4) was added to the flask to carry out infection at room temperature for 2 hours. The culture supernatant was discarded and replaced with 30 ml of fresh EX-CELL^{™} 400 medium to carry out 3 to 4 days of culturing at 27°C. After completion of the culturing, the culture supernatant was collected and centrifuged at 1,500 × g for 10 minutes to recover the supernatant.

The soluble human VEGF receptor KDR 7N was purified in the following manner.

A column packed with 50 ml of DEAE-Sepharose CL-6B (manufactured by Pharmacia Biotech) and a column packed with 40 ml of Heparin Sepharose CL-6B (manufactured by Pharmacia Biotech) were connected in series, the former column on the inlet side and the latter on the outlet side, and washed with 300 ml of 20 mM sodium phosphate buffer (pH 8). After the washing, 400 to 800 ml of a culture solution containing soluble human VEGF receptor KDR was passed through the columns at a flow rate of from 50 to 100 ml/hour. The columns were again washed with 300 ml of 20 mM sodium phosphate buffer (pH 8) and then the adsorbed protein was eluted from the Heparin Sepharose CL-6B column by continuous density gradient using 400 ml of 0 to 1 M NaCl/20 mM sodium phosphate buffer. The eluate was fractionated in 7 ml portions, and 60 to 80 ml of fractions containing soluble human VEGF receptor KDR 7N were recovered by analyzing the protein contained in each fraction by SDS-PAGE. The thus recovered purification fractions were concentrated using CentriPrep 10 (manufactured by Amicon) to obtain 4.8 ml of a soluble human VEGF receptor KDR 7N solution (protein concentration and purity were 815 µg/ml and 70 to 80%, respectively).

The soluble human VEGF receptor chimeric protein Flt-1 7N.K2 was purified in the following manner.

A column was packed with about 20 ml of Heparin-Sepharose CL-6B gel (manufactured by Pharmacia Biotech AB) and washed with 100 ml of 20 mM Tris-HCl buffer (pH 7.5) at a flow rate of 0.5 ml/minute. After the washing, 900 ml of the culture solution containing soluble human VEGF receptor chimeric protein Flt-1 7N.K2 prepared in the above was passed through the Heparin-Sepharose CL-6B column at a flow rate of 0.5 ml/minute. The column was washed again with 100 ml of 20 mM Tris-HCl buffer (pH 7.5) at a flow rate of 0.5 ml/minute, and then 200 ml of 20 mM Tris-HCl buffer (pH 7.5) containing NaCl having a density gradient of from 0 to 1.5 M was passed through the column to elute the protein adsorbed to Heparin-Sepharose, while the eluate was fractionated in 8 ml portions. By analyzing the protein contained in each fraction by SDS polyacrylamide gel electrophoresis (SDS-PAGE), 50 to 70 ml of fractions containing soluble human VEGF receptor chimeric protein Flt-1 7N.K2 were recovered and concentrated using CentriPrep 10 (manufactured by Amicon). After the concentration, human VEGF receptor chimeric protein Flt-1 7N.K2 was obtained as a 5.8 ml of solution (protein concentration, 588 µg/ml; purity, 65-75%).

### (6-6) Analysis of epitope which is recognized by anti-human VEGF receptor Flt-1 human chimeric antibody

The epitope which is recognized by purified anti-human VEGF receptor Flt-1 human chimeric antibodies KM2532 and KM2550 was analyzed in the following manner.

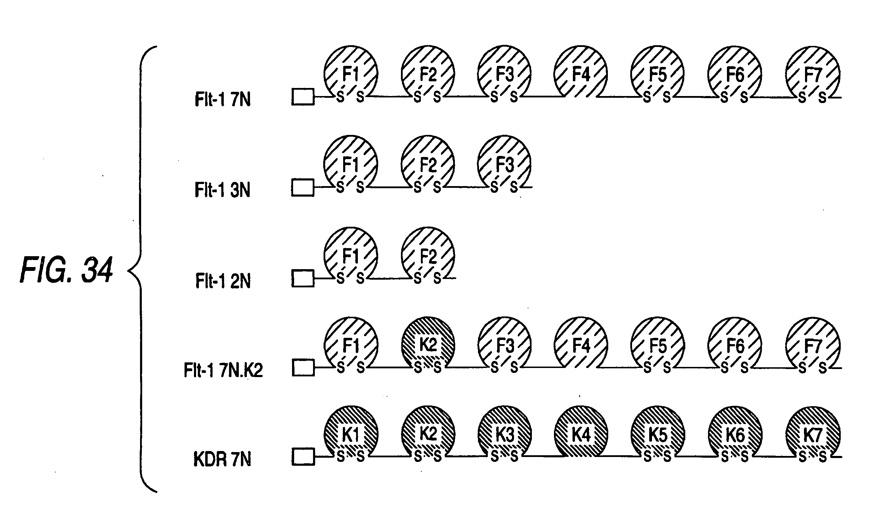

Soluble human VEGF receptor Flt-1 7N (corresponds to a sequence from the N-terminal amino acid to the 750th amino acid, including signal sequence), soluble human VEGF receptor Flt-1 3N (corresponds to a sequence from the N-terminal amino acid to the 338th amino acid, including signal sequence) and soluble human VEGF receptor Flt-1 2N (corresponds to a sequence from the N-terminal amino acid to the 223rd amino acid, including signal sequence) were prepared in accordance with the method of Tanaka et al. (Japanese (Journal of Cancer Research, 88: 867 (1997)). Soluble human VEGF receptor KDR 7N (corresponds to a sequence from the N-terminal amino acid to the 738th amino acid, does not include signal sequence) and soluble human VEGF receptor chimeric protein Flt-1 7N.K2 (100th to 204th amino acids of human Flt-1 7N are replaced with 95th to 199th amino acids of human KDR via links of two amino acids) were prepared in accordance with the method described in (6-5). Schematic illustration of each of the soluble human VEGF receptor derivatives used in the experiment is shown in Fig. 34.

First, the reactivity of anti-human VEGF receptor Flt-1 mouse monoclonal antibodies KM1732 and KM1750 and anti-human VEGF receptor Flt-1 human chimeric antibodies KM2532 and KM2550 was compared using an enzyme immunoassay. Each of Flt-1 7N, Flt-1 3N, Flt-1 2N, Flt-1 7N.K2 and KDR 7N diluted to 4 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 mouse monoclonal antibodies KM1732 or KM1750 or purified anti-human VEGF receptor Flt-1 human chimeric antibodies KM2532 or KM2550, having a concentration of from 0.1 µg/ml, was dispensed in 50 µl portions into the resulting wells to carry out the reaction at room temperature for 1 hour. After washing the plate with 0.05% Tween-PBS, a peroxidase-labeled anti-mouse IgG antibody (manufactured by Dako) which had been diluted 400 times with 1% BSA-PBS, or a peroxidase-labeled anti-human IgG antibody (manufactured by American Qualex) which had been diluted 3,000 times, was dispensed in 50 µl portions into the wells to carry out the reaction at room temperature for 1 hour, the resulting plate was washed with 0.05% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

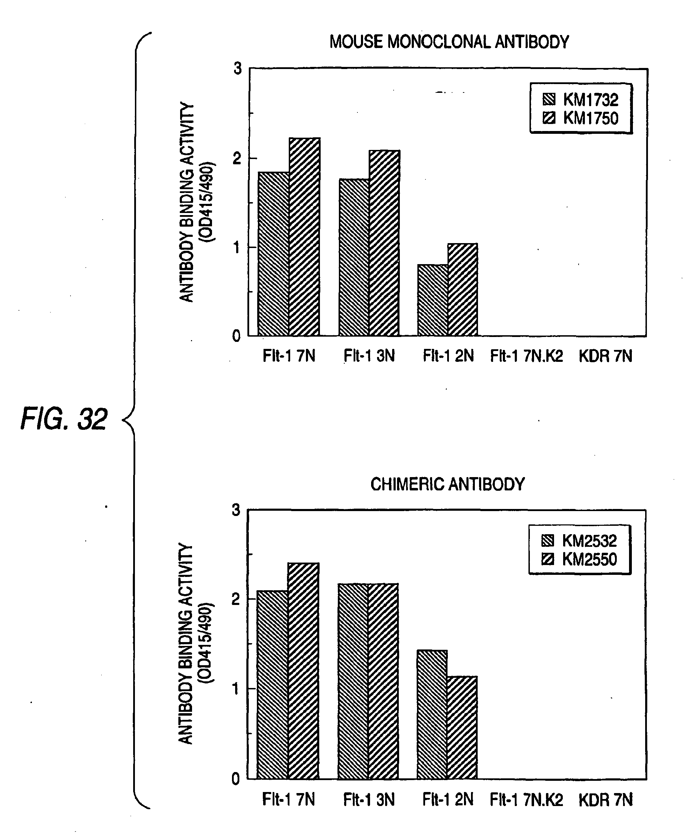

The results are shown in Fig. 32. The anti-human VEGF receptor Flt-1 mouse monoclonal antibodies KM1732 and KM1750 and anti-human VEGF receptor Flt-1 human chimeric antibodies KM2532 and KM2550 bound to Flt-1 7N, Flt-1 3N and Flt-1 2N, but not to Flt-1 7N.K2 and KDR 7N. In consequence, it was confirmed that the epitope which is recognized by anti-human VEGF receptor Flt-1 mouse monoclonal antibodies KM1732 and KM1750 and anti-human VEGF receptor Flt-1 human chimeric antibodies KM2532 and KM2550 is contained in a region of from 100th to 204th amino acids of human Flt-1 from the N-terminal including a signal sequence.

Next, the binding activity was examined using an enzyme immunoassay by fixing the amount of each of Flt-1 7N, Flt-1 3N, Flt-1 2N, Flt-1 7N.K2 and KDR 7N to be adsorbed on a 96 well plate for EIA and varying the concentration of human chimeric antibody to be added. Each of Flt-1 7N, Flt-1 3N, Flt-1 2N, Flt-1 7N.K2 and KDR 7N diluted to 4 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 0.0152 to 100 ng/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.05% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

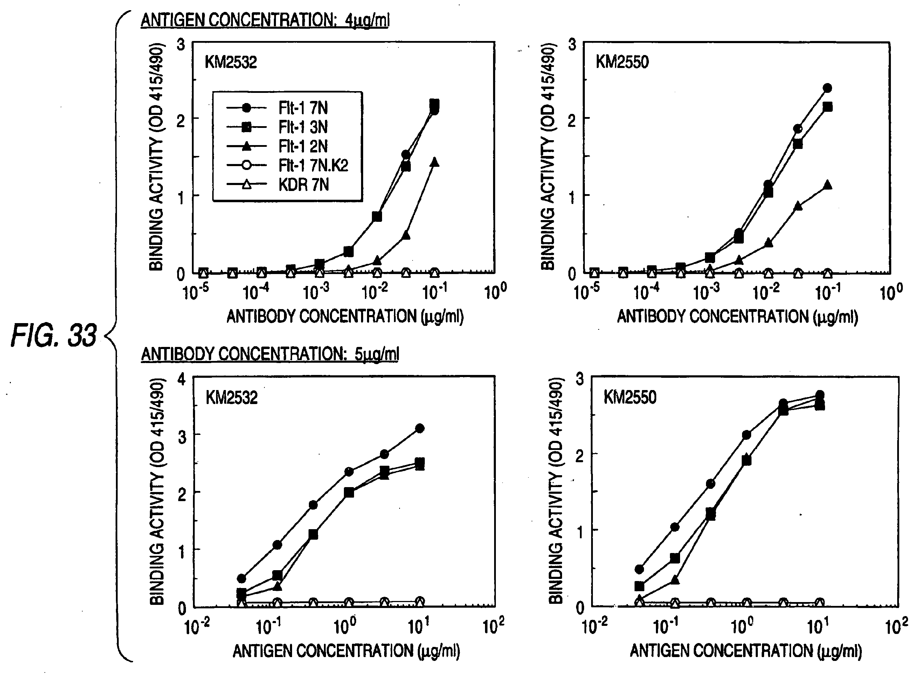

The results are shown in Fig. 33 (upper drawings). The anti-human VEGF receptor Flt-1 human chimeric antibodies KM2532 and KM2550 bound to Flt-1 7N, Flt-1 3N and Flt-1 2N in an antibody concentration dependent manner, but not to Flt-1 7N.K2 and KDR 7N. In addition, the anti-human VEGF receptor Flt-1 human chimeric antibodies KM2532 and KM2550 showed almost the same binding activity.

Next, the binding activity of human chimeric antibodies was examined using an enzyme immunoassay by varying the amount of each of Flt-1 7N, Flt-1 3N, Flt-1 2N, Flt-1 7N.K2 or KDR 7N to be adsorbed on a 96 well plate for EIA. Each of the soluble human VEGF receptors Flt-1 7N, Flt-1 3N, Flt-1 2N, Flt-1 7N.K2 and KDR 7N diluted to a concentration of from 0.041 to 10 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 5 µg/ml was dispensed in 50 µl portions into the resulting wells to carry out the reaction at room temperature for 1 hour. After washing of the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.05% Tween-PBS, and then an ABTS (ammonium 2,2-azinobis(3-ethylbenzothiazole-6-sulfonate)) substrate solution was dispensed in 50 µl portions into the wells to develop color and measure its absorbance at OD415nm using Emax (manufactured by Molecular Devices).

The results are shown in Fig. 33 (lower drawings). The anti-human VEGF receptor Flt-1 human chimeric antibodies KM2532 and KM2550 showed concentration dependent binding activity upon the human VEGF receptors Flt-1 7N, Flt-1 3N and Flt-1 2N, but did not react with Flt-1 7N.K2 and KDR 7N. In addition, the anti-human VEGF receptor Flt-1 human chimeric antibodies KM2532 and KM2550 showed almost the same binding activity.

### 3. Production of anti-human VEGF receptor Flt-1 human CDR-grafted antibody

Anti-human VEGF receptor Flt-1 human CDR-grafted antibodies having activity similar to that of anti-human VEGF receptor Flt-1 mouse monoclonal antibodies KM1732 and KM1750 and anti-human VEGF receptor Flt-1 human chimeric antibodies KM2532 and KM2550 having activity which neutralizes human VEGF receptor Flt-1 was produced as follows.

### (1) Construction of cDNA encoding VH of anti-human VEGF receptor Flt-1 human CDR-grafted antibody based on common sequence of VH of known human antibodies

According to Kabat et al. (Sequences of Proteins of Immunological Interest, US Dept. Health and Human Services, 1991, hereinafter the same), known human antibody VHs are classifiable into subgroups I to III (HSG I to III) based on the homology of amino acid sequences, and common sequences have been identified for respective subgroups. An amino acid sequence of VH of an anti-human VEGF receptor Flt-1 human CDR-grafted antibody was designed based on the common sequences. For selecting the common sequences to serve as the base, the homology was examined between the amino acid sequence of framework regions (hereinafter referred to as "FR") of the common- sequences of human antibody VH of each subgroup and amino acid sequences of FR of VH of anti-human VEGF receptor Flt-1 mouse monoclonal antibodies KM1732 and K1750 (Table 1).

**TABLE 1**

| | HSG I | HSG II | HSG III |
|---|---|---|---|
| KM1732 | 65.5 | 51.7 | 55.2 |
| KM1750 | 67.8 | 49.4 | 54.0 |

As a result, it was confirmed that KM1732 and KM1750 shows the highest homology with subgroup I. Thus, based on the common sequence of subgroup I, an anti-human VEGF receptor Flt-1 human CDR-grafted antibody was designed, and the cDNA encoding the amino acid sequence was constructed by using the polymeraze chain reaction method (hereinafter referred to as "PCR") in the following manner.

The construction of cDNA encoding VH of an anti-human VEGF receptor Flt-1 human CDR-grafted antibody derived from the VH of anti-human VEGF mouse receptor Flt-1 mouse monoclonal antibody KM1732 is explained below. Six synthetic DNAs having a nucleotide sequence of SEQ ID NOs:25 to 30 were synthesized (manufactured by Sawady Technology). The DNAs synthesized each was added, to have a final concentration of 0.1 µM, to 50 µl of 1 × Ex Taq buffer (manufactured by Takara Shuzo Co., Ltd.) containing 200 µM dNTP, 0.5 µM M13primer RV, 0.5 µM M13primer M4 and 2.5 units of TaKaRa Ex Taq DNA polymerase (all manufactured by Takara Shuzo Co., Ltd.), the mixture was covered with 50 µl of mineral oil, DNA thermal cycler PJ480 (manufactured by Perkin Elmer) was loaded with the mixture, and 30 PCR cycles (at 94°C for 2 minutes, at 55°C for 2 minutes and at 72°C for 2 minutes per cycle) were conducted. The reaction mixture was purified using QIAquick PCR Purification Kit (manufactured by, Qiagen) according to the manual attached hereto, and the mixture was purified and eluted with 20 µl of sterilized water. Next, the obtained elution was added to 30 µl of a buffer solution comprising 10 mM Tris-HC1 (pH 7.5), 10 mM magnesium chloride and 1 mM DTT, 20 units of a restriction enzyme ApaI (manufactured by Takara Shuzo Co., Ltd.) were further added thereto, and then the reaction was carried out at 37°C for 1 hour. The reaction mixture was precipitated with ethanol, the precipitate was added to 10 µl of a buffer solution comprising 50 mM Tris-HCl (pH 7.5), 100 mM sodium chloride, 10 mM magnesium chloride, 1 mM DTT, 100 µg/ml BSA, and 0.01% Triton X-100, 10 units of restriction enzyme *Not*I (manufactured by Takara Shuzo Co., Ltd.) were further added thereto, and then the reaction was allowed to proceed at 37°C for 1 hour. The reaction mixture- was fractionated by agarose gel electrophoresis, and about 0.5 µg of an *Apa*I-*Not*I fragment about 0.44 kb was recovered.

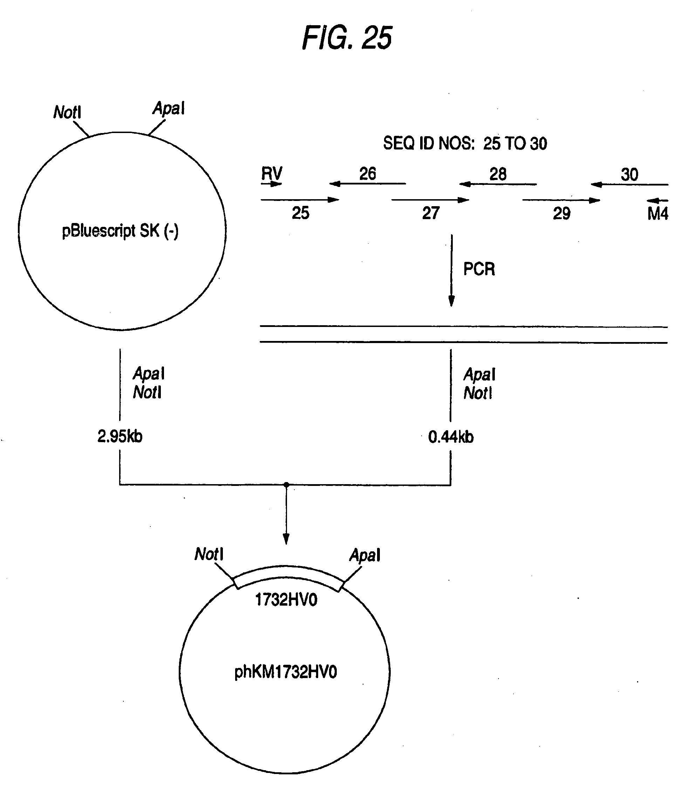

To 10 µl in total volume of sterilized water, 0.1 µl of the ApaI-NotI fragment derived from plasmid pBluescript SK(-) obtained in 2(1) of Reference Example 1, and 0.1 µg of the *Apa*I-*Not*I fragment which was the PCR amplified fragment obtained in the above were added, and the fragments were linked using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution. Plasmid DNA was prepared from each of ten *Escherichia coli* transformants. As a result that the nucleotide sequence was determined according to the method described in 1(4) of Reference Example 1, plasmid phKM1732HV0 shown in Fig. 25 and containing cDNA encoding the amino acid sequence of interest was obtained. The nucleotide sequence and the amino acid sequence of the VH of the anti-human VEGF receptor Flt-1 human CDR-grafted antibody (hereinafter referred to as "1732HV0") contained in phKM1732HVO are shown in SEQ ID NOs:31 and 86, respectively.

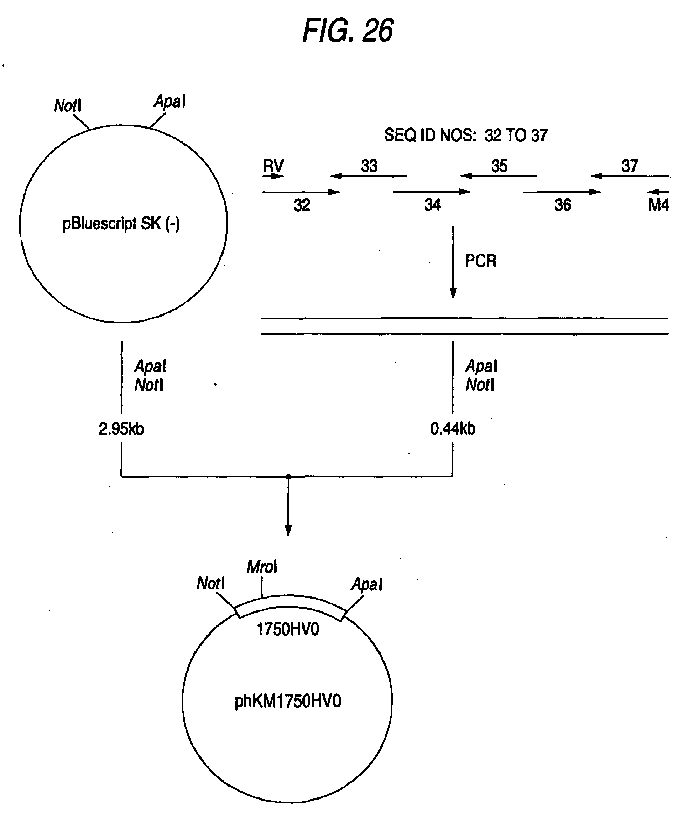

With regard to the construction of cDNA encoding VH of an anti-human VEGF receptor Flt-1 human CDR-grafted antibody derived from the VH of the anti-human VEGF mouse receptor Flt-1 mouse monoclonal antibody KM1750, the reactions were carried out in the same manner as described above using six synthetic DNAs having a nucleotide sequence of SEQ ID NOs:32 to 37 were synthesized (manufactured by Sawady Technology) to obtain plasmid phKM1750HV0 containing cDNA encoding an amino acid sequence of interest shown in Fig. 26. The nucleotide sequence and the amino acid sequence of the VH of the anti-human VEGF receptor Flt-1 human CDR-grafted antibody (hereinafter referred to as "1750HV0") contained in phKM1750HV0 are shown in SEQ ID NOs:38 and 87, respectively.

### (2) Construction of cDNA encoding VL of anti-human VEGF receptor Flt-1 human CDR-grafted antibody based on common sequence of VL of known human antibodies

According to Kabat *et al*., known human antibody VLs are classifiable into subgroups I to IV (HSG I to IV) based on the homology of amino acid sequences, and the common sequence has been identified for respective subgroups. An amino acid sequence of VL of an anti-human VEGF receptor Flt-1 human CDR-grafted antibody was designed based on the common sequence. For selecting the common sequence to serve as the base, the homology was examined between the amino acid sequence of FR of the common sequence of human antibody VL of each subgroup and amino acid sequences of FR of VL of anti-human VEGF receptor Flt-1 mouse monoclonal antibodies KM1732 and KM1750 (Table 2).

**TABLE 2**

| | HSG I | HSG II | HSG III | HSG IV |
|---|---|---|---|---|
| KM1732 | 66.2 | 57.5 | 62.5 | 63.8 |
| KM1750 | 68.8 | 63.8 | 68.8 | 70.0 |

As a result, it was confirmed that KM1732 shows the highest homology with subgroup I and KM1750 shows almost the same high homology with subgroups I and IV, so that the amino acid sequence of the VL of anti-human VEGF receptor Flt-1 human CDR-grafted antibody was designed based on the common sequence of subgroup I in the case of KM1732 and the common sequence of subgroups I and IV in the case of KM1750, and cDNA encoding the amino acid sequence was constructed by PCR in the following manner.

The construction of cDNA encoding VL of an anti-human VEGF receptor Flt-1 human CDR-grafted antibody derived from the VL of anti-human VEGF mouse receptor Flt-1 mouse monoclonal antibody KM1732 is explained below. Six synthetic DNAs having a nucleotide sequence of SEQ ID NOs:39 to 44 were synthesized (manufactured by Sawady Technology). The DNAs synthesized each was added, to have a final concentration of 0.1 µM, to 50 µl of 1 × Ex Taq buffer (manufactured by Takara Shuzo Co., Ltd.) containing 200 µM dNTP, 0.5 µM M13primer RV, 0.5 µM M13primer M4 and 2.5 units of TaKaRa Ex Taq DNA polymerase (all manufactured by Takara Shuzo Co., Ltd.), the mixture was covered with 50 µl of mineral oil, DNA thermal cycler PJ480 (manufactured by Perkin Elmer) was loaded with the mixture, and 30 PCR cycles (at 94°C for 2 minutes, at 55°C for 2 minutes and at 72°C for 2 minutes per cycle) were conducted. The reaction mixture was purified using QIAquick PCR Purification Kit (manufactured by Qiagen) according to the manual attached hereto, and the mixture was purified and eluted with 20 µl of sterilized water. Next, the obtained elution was added to 30 µl of a buffer solution comprising 50 mM Tris-HCl (pH 7.5), 100 mM sodium chloride, 10 mM sodium magnesium, 1 mM DTT, and 100 µg/ml BSA, 10 units of a restriction enzyme EcoRI (manufactured by Takara Shuzo Co., Ltd.), 10 units of a restriction enzyme SplI (manufactured by Takara Shuzo Co., Ltd.) were further added thereto, and then the reaction was carried out at 37°C for 1 hour. The reaction mixture was fractionated by agarose gel electrophoresis, and about 0.5 µg of an *EcoR*I-*Spl*I fragment about 0.39 kb was recovered.

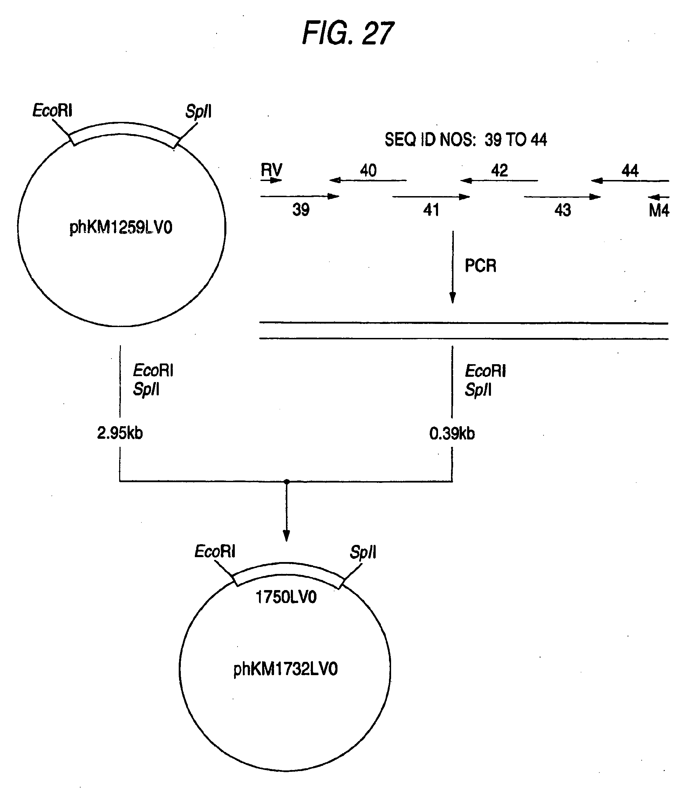

To 10 µl in total volume of sterilized water, 0.1 µg of the *EcoR*I-*Spl*I fragment derived from plasmid phKM1259LV0 obtained in 2(1) of Reference Example 1, and 0.1 µg of the *EcoR*I-*Spl*I fragment which was the PCR amplified fragment obtained in the above were added, and the fragments were linked using DNA ligation Kit Ver. 2 (manufactured by Takara Shuzo Co., Ltd.) According to the manual attached hereto. *Escherichia* coli DH5α (manufactured by TOYOBO CO., LTD.) was transformed by using the thus-obtained recombinant plasmid DNA solution. Plasmid DNA was prepared from each of ten *Escherichia coli* transformants. As a result that the nucleotide sequence was determined according to the method described in 1(4) of Reference Example 1, plasmid phKM1732LV0 shown in Fig. 27 and containing cDNA encoding the amino acid sequence of interest was obtained. The nucleotide sequence and the amino acid sequence of the VL of the anti-human VEGF receptor Flt-1 human CDR-grafted antibody (hereinafter referred to as "1732LV0") contained in phKM1732LV0 are shown in SEQ ID NOs:45 and 88, respectively.

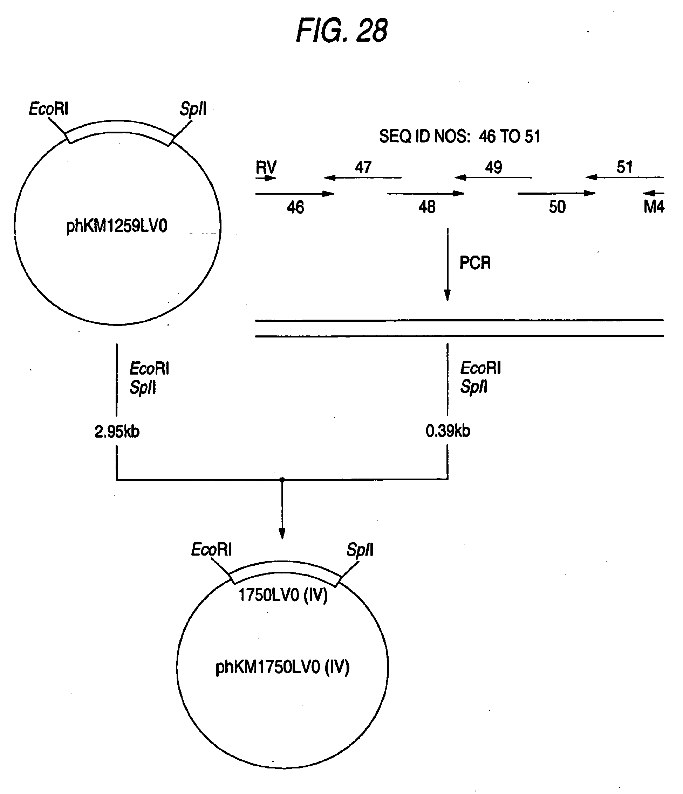

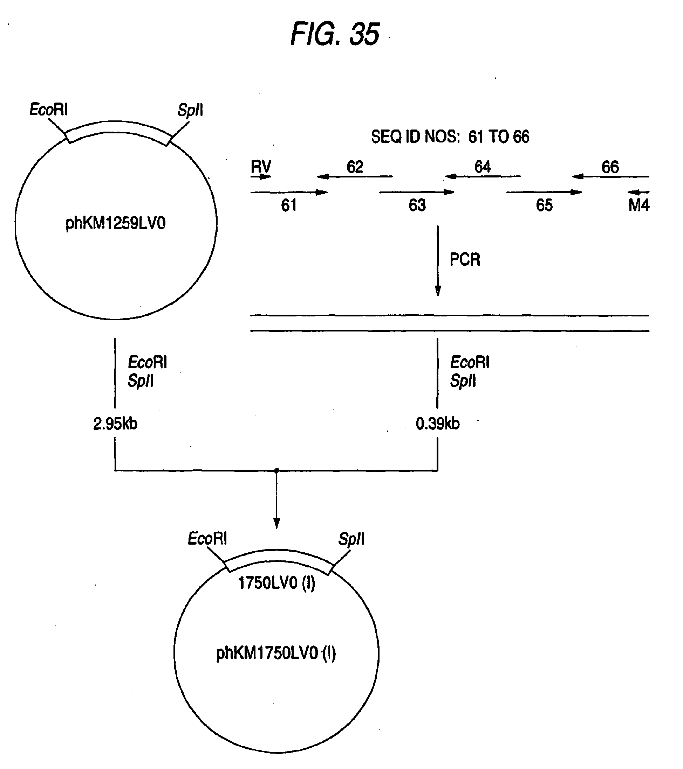

When the common sequence of human subgroup IV was used as the base in constructing cDNA encoding the VL of anti-human VEGF receptor Flt-1 human CDR-grafted antibody derived from the VL of anti-human VEGF receptor Flt-1 mouse monoclonal antibody KM1750, plasmid phKM1750LV0(IV) containing cDNA encoding the amino acid sequence of interest shown in Fig. 28 was obtained by carrying out the reaction similar to the above using 6 synthetic DNA fragments (manufactured by Sawady Technology) having nucleotide sequences of SEQ ID NOs:46 to 51. Corresponding nucleotide sequence and amino acid sequence of the VL of anti-human VEGF receptor Flt-1 human CDR-grafted antibody contained in phKM1750LV0(IV) (hereinafter referred to as "1750LV0(IV)") are shown in SEQ ID NOs:52 and 89, respectively. When the common sequence of human subgroup I was used as the base, plasmid phKM1750LVO(I) containing cDNA encoding the amino acid sequence of interest shown in Fig. 35 was obtained by carrying out the reaction similar to the above using 6 synthetic DNA fragments (manufactured by Sawady Technology) having nucleotide sequences of SEQ ID NOs:61 to 66. Corresponding nucleotide sequence and amino acid sequence of the VL of anti-human VEGF receptor Flt-1 human CDR-grafted antibody contained in phKM1750LV0(I) (hereinafter referred to as "1750LV0(I)") are shown in SEQ ID NOs:67 and 90, respectively.

### (-3) Construction of anti-human VEGF receptor Flt-1 human CDR-grafted antibody expression vectors based on common sequence of V regions of known human antibodies

Expression vectors of anti-human VEGF receptor Flt-1 human CDR-grafted antibody were constructed in the following manner using tandem cassette vector, pKANTEX93, for humanized antibody expression described in WO 97/10354 and plasmids phKM1732HV0, phKM1732LV0, phKM1750HV0, phKM1750LV0(I) and phKM1750LV0(IV) containing cDNA encoding the V region of anti-human VEGF receptor Flt-1 human CDR-grafted antibody obtained in 3(1) and 3(2) of Reference Example 1.

The construction of an expression vector of anti-human VEGF receptor Flt-1 human CDR-grafted antibody derived from anti-human VEGF receptor Flt-1 mouse monoclonal antibody KM1732 is explained below.

To 10 µl of a buffer solution comprising 10 mM Tris-HCl (pH 7.5), 10 mM magnesium chloride and 1 mM DTT, 5 µg of plasmid phKM1732HVO was added, 10 units of a restriction enzyme *Apa*I (manufactured by Takara Shuzo Co., Ltd.) were further added thereto, and then the reaction was carried out at 37°C for 1 hour. The reaction mixture was precipitated with ethanol, the precipitate was added to 10 µl of a buffer solution comprising 50 mM Tris-HCl (pH 7.5), 100 mM sodium chloride, 10 mM magnesium chloride, 1 mM DTT, 100 µg/ml BSA, and 0.01% Triton X-100, 10 units of a restriction enzyme NotI (Takara Shuzo Co., Ltd.) were further added thereto, and then the reaction was carried out at 37°C for 1 hour. The reaction mixture was fractionated by agarose gel electrophoresis, and about 0.5 µg of an *Apa*I-*Not*I fragment about 0.44 kb was recovered.

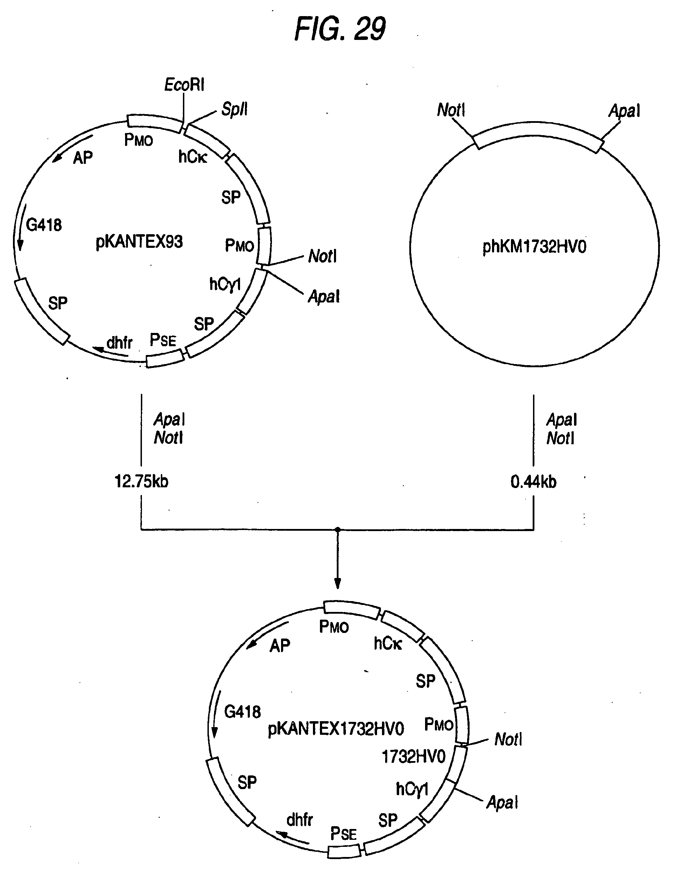

To 10 µl in total volume of sterilized water, the *Apa*RI-*Not*I fragment obtained in 2(1) of Reference Example 1 derived from tandem cassette vector for humanized antibody expression, pKANTEX93, and 0.1 µg of the *Apa*I-*Not*I fragment of phKM1732HVO obtained in the above were added, and the fragments were linked using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution to obtain plasmid pKANTEX1732HV0 shown in Fig. 29.

Next, 3 µg of the thus obtained plasmid pKANTEX1732HV0 was added to a buffer solution comprising 50 mM Tris-Hcl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were added thereto, and the reaction was carried out at 37°C for 1 hour. The reaction mixture was fractionated by agarose gel electrophoresis, and about 1 µg of an *Eco*I-*Spl*I fragment about 13.20 kb in size was recovered. Further, 5 µg of plasmid phKM1732LV0 was added to a buffer solution comprising 50 mM Tris-HC1 (pH 7.5), 100 mM sodium chloride, 10 mM magnesium chloride, 1 mM DTT and 100 µg/ml BSA, 10 units of a restriction enzyme EcoRI (manufactured by Takara Shuzo Co., Ltd.) and 10 units of a restriction enzyme *Spl*I (manufactured by Takara Shuzo Co., Ltd.) were added thereto, and the reaction was carried out at 37°C for 1 hour. The reaction mixture was fractionated by agarose gel electrophoresis, and about 0.5 µg of an *Eco*I-*Spl*I fragment about 0.39 kb in size was recovered.

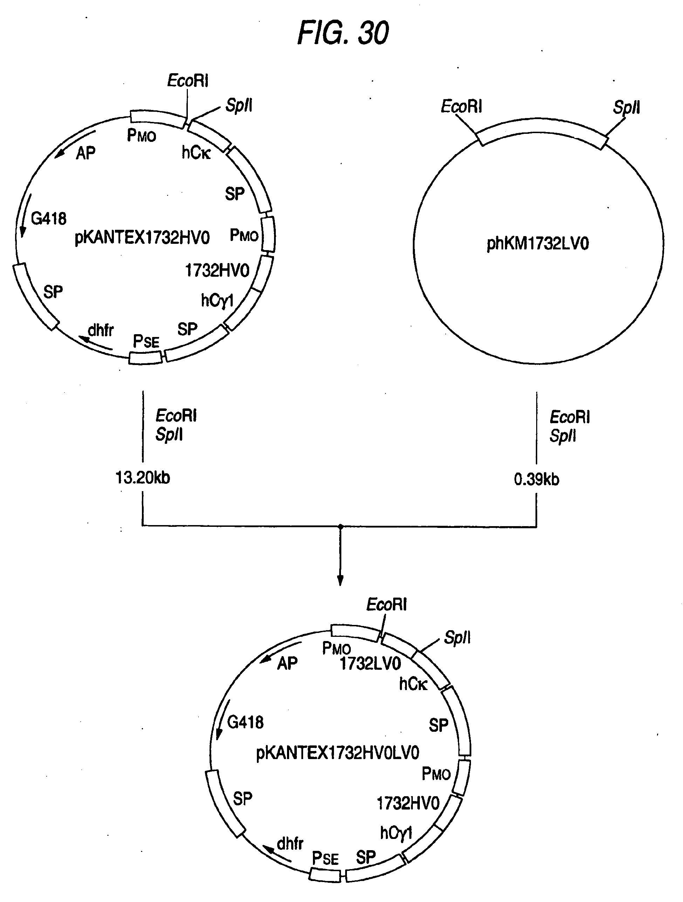

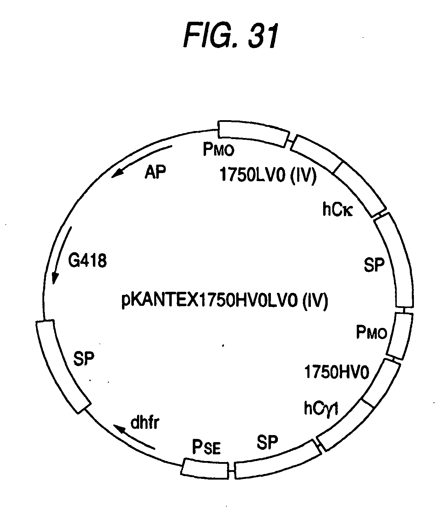

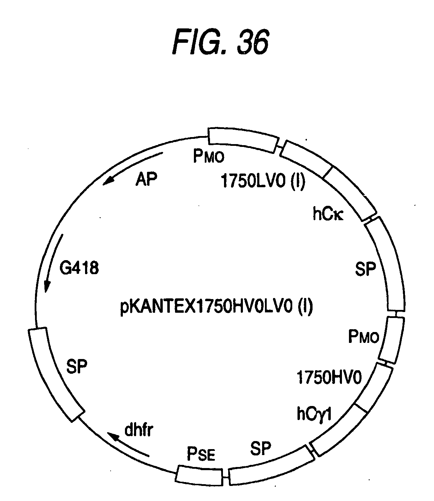

To 10 µl in total volume of sterilized water, 0.1 µg of the thus obtained *Eco*RI-*Spl*I fragment derived from plasmid pKANTEX1732HV0 and 0.1 µg of the *Eco*I-*Spl*I fragment derived from plasmid phKM1732LV0 were added, and the fragments were ligated using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution to obtain an expression vector of anti-human VEGF receptor Flt-1 human CDR-grafted antibody, pKANTEX1732HV0LV0, shown in Fig. 30. Regarding the construction of expression vectors of anti-human VEGF receptor Flt-1 human CDR-grafted antibody originated from anti-human VEGF receptor Flt-1 mouse monoclonal antibody KM1750, the reaction similar to the above was carried out to obtain vectors for anti-human VEGF receptor Flt-1 human CDR-grafted antibody expression, pKANTEX1750HVOLVO(IV) and pKANTEX1750HV0LV0(I), shown in Figs. 31 and 36, respectively.

### 4. Analysis of amino acid sequence of FR of V region of anti-human VEGF receptor Flt-1 human CDR-grafted antibody and production of expression vector for modified human CDR-grafted antibody in which amino acids of FR are modified

Among amino acid residues which are different between the amino acid sequence of the FR of the V region of each anti-human VEGF receptor Flt-1 human CDR-grafted antibody and the amino acid sequence of the FR of the V region of each anti-human VEGF receptor Flt-1 mouse monoclonal antibody, the positions of the amino acid residues which were considered to be important for binding to human VEGF receptor Flt-1 in the amino acid sequence of the FR of the V region of each anti-human VEGF receptor Flt-1 human CDR-grafted antibody were identified by constructing and comparing a computer three-dimensional structure model of each of the V region of anti-human VEGF receptor Flt-1 mouse monoclonal antibody KM1732 and KM1750 and that of each of the anti-human VEGF receptor Flt-1 human CDR-grafted antibodies produced in 3 of Reference Example 1. As a result, regarding the anti-human VEGF receptor Flt-1 human CDR-grafted antibody derived from anti-human VEGF receptor Flt-1 mouse monoclonal antibody KM1732, it was considered that the positions of 24-position alanine, 27-position, tyrosine, 40-position alanine, 67-position arginine, 69-position threonine, 70-position isoleucine, 82-position glutamic acid and 93-position valine in the amino acid sequence of the VH of SEQ ID NO:86 and the positions of 39-position proline, 45-position leucine, 46-position leucine, 69-position asparatic acid, and 70-position phenylalanine in the amino acid sequence of VL of SEQ ID N0:88 are playing an important role, in its binding to the human VEGF receptor Flt-1.

Regarding the anti-human VEGF receptor Flt-1 human CDR-grafted antibody derived from anti-human VEGF receptor Flt-1 mouse monoclonal antibody KM1750, it was considered that the positions of 3-position glutamine, 67-position arginine, 82-position glutamic acid, 84-position serine and 95-position tyrosine in the amino acid sequence of the VH of SEQ ID N0:87 and the positions of 17-position aspartic acid, 18-position arginine, 39-position proline, 59-position serine, 69-position aspartic acid and 70-position phenylalanine in the amino acid sequence of the VL of SEQ ID N0:90 are playing an important role in its binding to the human VEGF receptor Flt-1. These facts suggest that the activity of anti-human VEGF receptor Flt-1 human CDR-grafted antibody to bind to human VEGF receptor Flt-1 can be modified, for example, by modifying the just described positions of amino acid residues of anti-human VEGF receptor Flt-1 human CDR-grafted antibody into the amino acid residues of the corresponding positions of anti-human VEGF receptor Flt-1 mouse monoclonal antibody.

### (1) Construction of cDNA encoding VH of modified human CDR-grafted antibody in which amino acids of FR are modified

Regarding the H chain of anti-human VEGF receptor Flt-1 human CDR-grafted antibody derived from anti-human VEGF receptor Flt-1 mouse monoclonal antibody KM1750, the 3 residues (82-position glutamic acid, 84-position serine and 95-position tyrosine) among the above-described 5 amino acid residues considered to be playing an important role in its binding to the human VEGF receptor Flt-1 were considered to be particularly important, so that cDNA encoding an amino acid sequence which was converted into the corresponding residues (82-position glutamine, 84-position arginine and 95-position phenylalanine) found in KM1750 was constructed by PCR in the following manner.

Using 6 synthetic DNA fragments having the nucleotide sequences of SEQ ID NOs:68 to 73 (manufactured by Sawady Technology), an *Mro*I-*Apa*I fragment of about 0.39 kb was recovered by PCR in accordance with the above-described method.

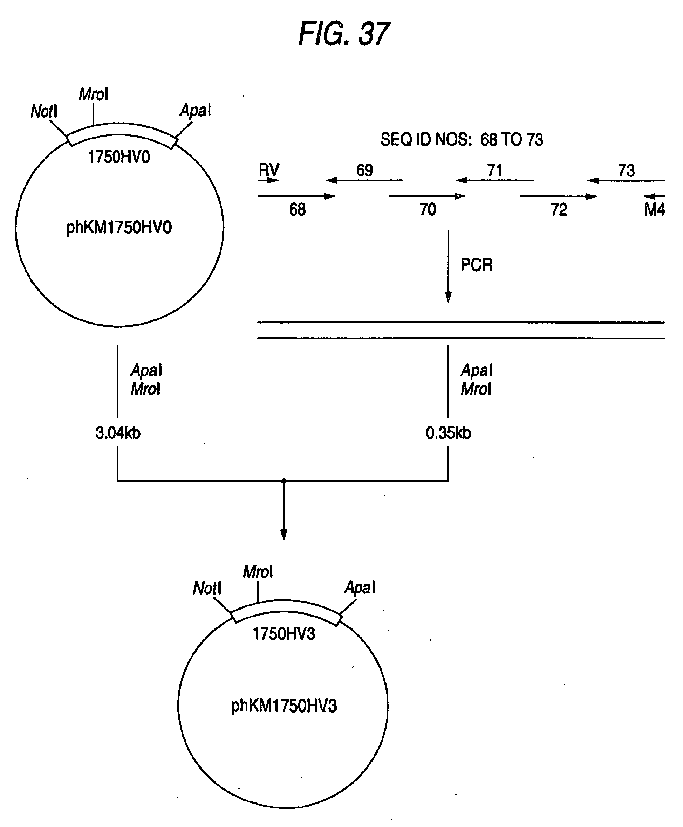

Plasmid phKM1750HV0 -was treated with restriction enzymes *Apa*I and *Mro*l to recover an *Apa*I-*Mro*I fragment of about 3.04 kb which was subsequently connected with the PCR amplified fragment obtained in the above, and then *Escherichia coli* DH5α (manufactured by TOYOBO CO., LTD.) was transformed using the thus obtained recombinant plasmid DNA solution. Plasmid was prepared from each of ten *Escherichia coli* transformants and its nucleotide sequence was determined in accordance with the method described in 1(4) of Reference Example 1 to obtain plasmid phKM1750HV3 shown in Fig. 37 which contains cDNA encoding the amino acid sequence of interest.

Corresponding nucleotide sequence and amino acid sequence of the VH of anti-human VEGF receptor Flt-1 human CDR-grafted antibody contained in phKM1750HV3 (hereinafter referred to as "1750HV3") are shown in SEQ ID NOs:74 and 91, respectively.

### (2) Construction of cDNA encoding VL of modified human CDR-grafted antibody in which amino acids of FR are modified

Regarding L chain of anti-human VEGF receptor Flt-1 human CDR-grafted antibody derived from anti-human VEGF receptor Flt-1 mouse monoclonal antibody KM1750, the 4 residues (17-position aspartic acid, 18-position arginine, 69-position aspartic acid and 70-position phenylalanine) among the above-described 6 amino acid residues considered to be playing an important role in its binding to the human VEGF receptor Flt-1 were considered to be particularly important in KM1750LVO(I), so that cDNA encoding an amino acid sequence which was converted into the corresponding residues (17-position glutamic acid, 18-position glutamic, 69-position phenylalanine and 70-position tyrosine) found in KM1750 was constructed by PCR in the following manner.

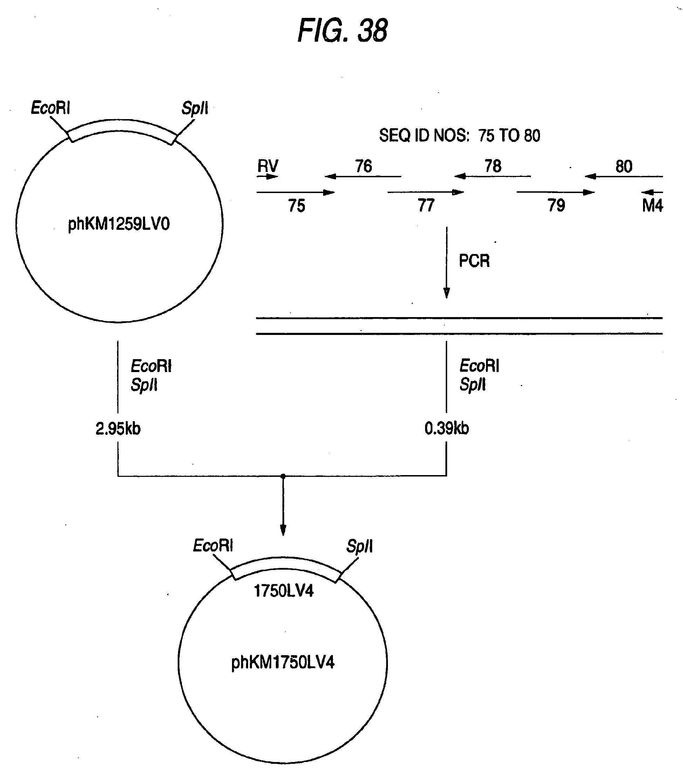

Using 6 synthetic DNA fragments having the nucleotide sequences of SEQ ID NOs:75 to 80 (manufactured by Sawady Technology), the same reaction was carried out to obtain the plasmid phKM1750LV4 shown in Fig. 38 which contains cDNA encoding the amino acid sequence of interest. Corresponding nucleotide sequence and amino acid sequence of the VL of ant-i-human VEGF receptor Flt-1 human CDR-grafted antibody contained in phKM1750LV4 (hereinafter referred to as "1750V4") are shown in SEQ ID NOs:81 and 92, respectively.

### (3) Construction of expression vector for modified human CDR-grafted antibody in which amino acids of FR are modified

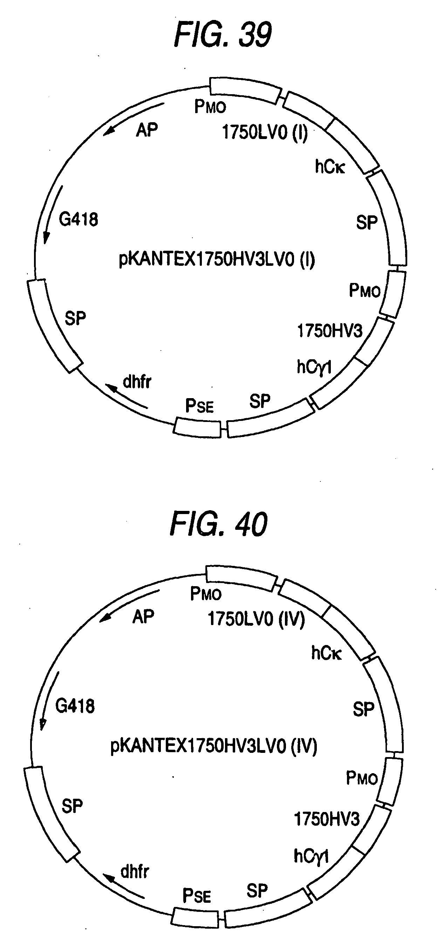

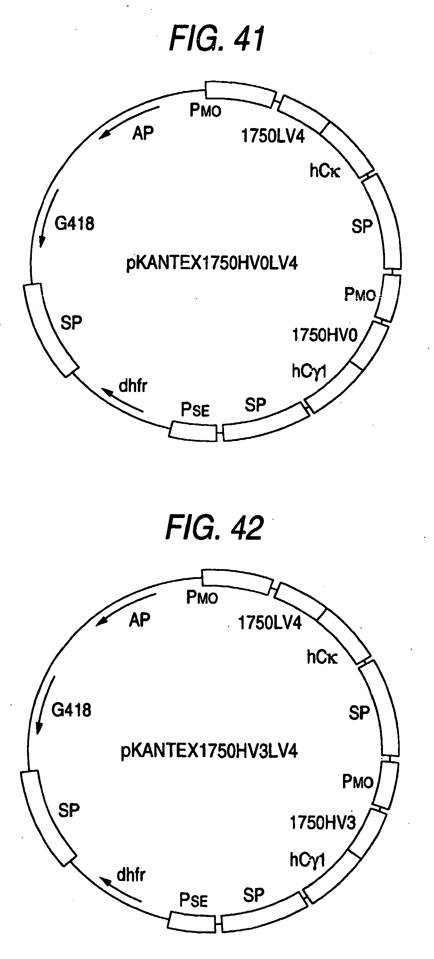

Using plasmids phKM1750HV3 and phKM1750LV4 obtained in 4(3) of Reference Example 1 containing cDNA encoding the V region of anti-human VEGF receptor Flt-1 human CDR-grafted antibody and plasmids phKM1750HV0, phKM1750LV0(I) and phKM1750LV0(IV) obtained in 3(1) and 3(2) of Reference Example 1, the reaction similar to that in 3(3) of Reference Example 1 was carried out to obtain pKANTEX1750HV3LV0(I), pKANTEX1750HV3LV0(IV), pKANTEX1750HV0LV4, and pKANTEX1750HV3LV4 as expression vectors of modified anti-human VEGF receptor Flt-1 human CDR-grafted antibodies in which amino acids of FR were modified shown in Figs. 39, 40, 41, and 42, respectively.

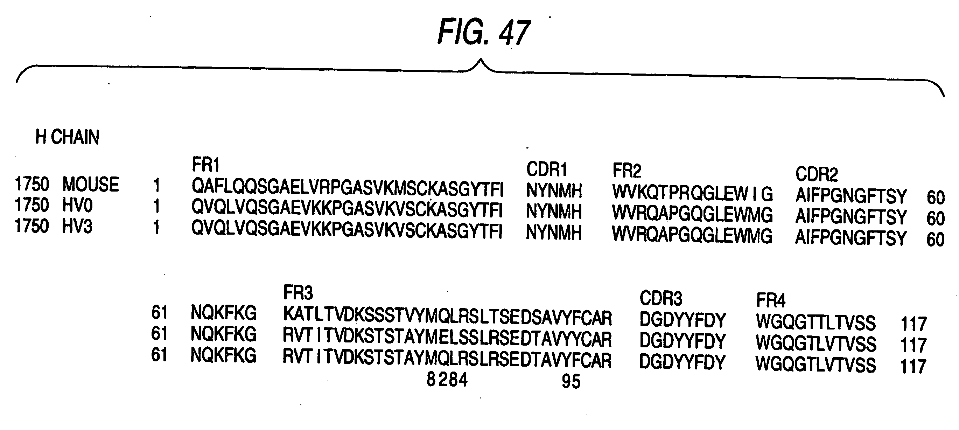

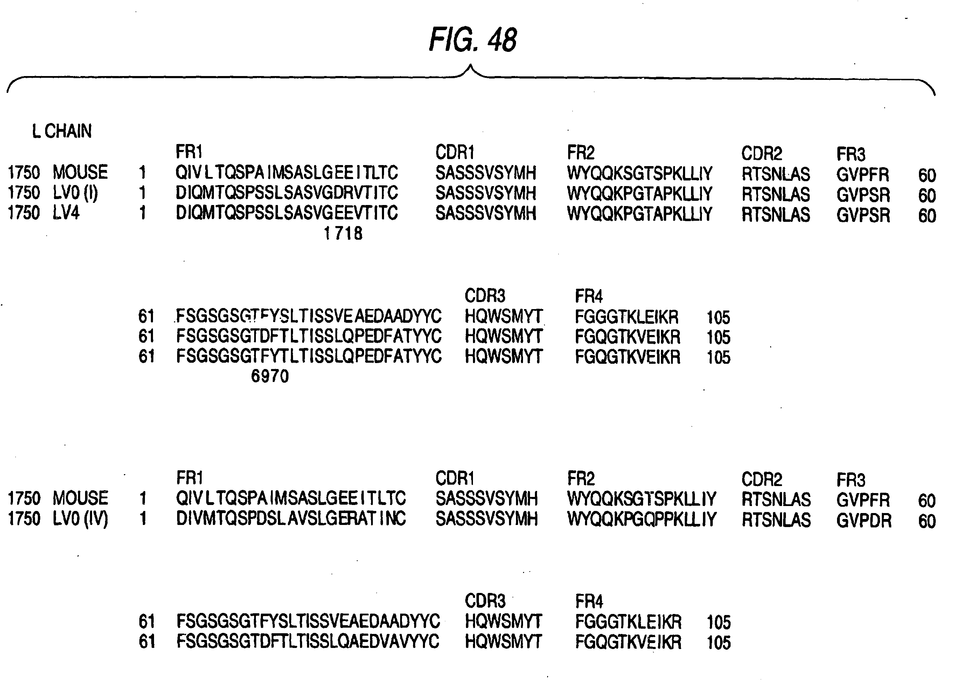

Amino acid sequences of KM1750 mouse H chain, KM1750HV0 and KM1750HV3 are shown in Fig. 47. Amino acid sequences of KM1750 mouse L chain, KM1750LV0(I), KM1750LV0(IV) and KM1750LV4 are shown in Fig. 48.

*Escherichia coli* DH5α/phKM1750HV0 in which recombinant plasmid phKM1750HV0 is introduced, *Escherichia coli* DHSa/phKM1750HV3 in which recombinant plasmid phKM1750HV3 is introduced, *Escherichia coli* XL1-Blue/phKM1750LV0(I) in which recombinant plasmid phKM1750LV0(I) is introduced, *Escherichia coli* XL1-Blue/phKM1750LV0(IV) in which recombinant plasmid phKM1750LV0(IV) is introduced, and *Escherichia coli* DH5α/pKM1750LV4 in which recombinant plasmid phKM1750LV4 is introduced have been deposited on May 12, 1999, in National Institute of Bioscience and Human Technology, Agency of Industrial Science and Technology (Higashi 1-1-3, Tsukuba-shi, Ibaraki, Japan) as FERM BP-6719, FERM BP-6720, FERM BP-6716, FERM BP-6717, and FERM BP-6718, respectively.

### 5. Production and activity evaluation of anti-human VEGF receptor Flt-1 human CDR-grafted antibody

### (1) Expression of anti-human VEGF receptor Fit-1 human CDR-grafted antibody in rat myeloma YB2/0 cells (ATCC CRL1581) using pKANTEX1750HV0LV0(I), pKANTEX1750HV0LV0(IV), pKANTEX1750HV3LV0(I), pKANTEX1750HV3LV0(IV), pKANTEX1750HV0LV4 and pKANTEX1750HV3LV4

Introduction of anti-human VEGF receptor Flt-1 human CDR-grafted antibody expression vectors pKANTEX1750HV0LV0(I), pKANTEX1750HV0LV0(IV), pKANTEX1750HV3LV0(I), pKANTEX1750HV3LV0(IV), pKANTEX1750HVOLV4 and pKANTEX1750HV3LV4 into YB2/0 cells was carried out by the electroporation method (*Cytotechnology*, 3: 133 (1990)) according to 1(1) of Reference Example 1.

A 5 µg portion of each of pKANTEX1750HV0LV0(I), pKANTEX1750HV0LV0(IV), pKANTEX1750HV3LV0(I), pKANTEX1750HV3LV0(IV), pKANTEX1750HV0LV4 and pKANTEX1750HV3LV4 obtained in 4(3) of Reference Example 1 was introduced into 4×10⁶ of YB2/0 cells, and the resulting cells were suspended in 40 ml of RPMI1640-FCS(10) medium (RPMI1640 medium (manufactured by Nissui Pharmaceutical) containing 10% fetal calf serum (FCS)) and dispensed in 200 µl portions into wells of a 96 well microtiter plate (manufactured by Sumilon). After 24 hours of culturing at 37°C in a 5% CO₂. incubator, G 418 (manufactured by Gibco) was added to a concentration of 0.5 mg/ml, and the culturing was continued for 1 to 2 weeks. Culture supernatants were recovered from wells in which transformant colonies having G 418 resistance were formed and became confluent, and the activity of anti-human VEGF receptor Flt-1 human CDR-grafted antibody in each culture supernatant was measured by the enzyme immunoassay shown in 2(3) of Reference Example 1.

Transformants which showed the activity of anti-human VEGF receptor Flt-1 human CDR-grafted antibody in their culture supernatants were subjected to cloning by carrying out limiting dilution twice to obtain transformant cell lines capable of producing anti-human VEGF receptor Flt-1 human CDR-grafted antibody. Examples of the transformants obtained by introducing the expression vectors pKANTEX1750HV0LV0(I), pKANTEX1750HV0LV0(IV), -pKANTEX1750HV3LV0(I), pKANTEX1750HV3LV0(IV), pKANTEX1750HV0LV4 and pKANTEX1750HV3LV4, namely transformants capable of producing the anti-human VEGF receptor Flt-1 human CDR-grafted antibody originated from anti-human VEGF receptor Flt-1 monoclonal antibody KM1750, include KM8550, KM8551, KM8552, KM8553, KM8554 and KM8555, respectively, and the anti-human VEGF receptor Flt-1 human CDR-grafted antibodies produced by these transformants were named KM8550, KM8551, KM8552, KM8553, KM8554 and KM8555, respectively. Each of the thus obtained transformant cell clones showed an anti-human VEGF receptor Flt-1 human CDR-grafted antibody productivity of about from 0.1 to 1 µg/ml.

### (2) Purification of anti-human VEGF receptor Flt-1 human CDR-grafted antibody from culture supernatant

Each of the anti-human VEGF receptor Flt-1 human CDR-grafted antibody-producing cell lines KM8550, KM8551, KM8552, KM8553, KM8554 and KM8555 obtained in 5(1) of Reference Example 1 was suspended in GIT medium (manufactured by Nippon Pharmaceutical) containing 0.5 mg/ml of G 418, to a cell density of 1×10⁵ to 2×10⁵ cells/ml, and the suspension was dispensed in 200 ml portions into a total of five 175 cm² flasks (manufactured by Greiner). When the cells became confluent after 5 to 7 days of culturing at 37°C in a 5% CO₂ incubator, about 1.1 to 2.5 L of each culture supernatant was recovered. A column was packed with about 1 ml of ProSep A (manufactured by Bioprocessing) and washed with 10 ml of 1 M glycine-0.15 M NaCl (pH 8.6) at a flow rate of 1 ml/minute. After washing, 2.3 L, 2.5 L, 1.9 L, 2.4 L, 1.1 L or 2 L of the culture filtrate of anti-human VEGF receptor Flt-1 human CDR-grafted antibody-producing cell line KM8550, KM8551, KM8552, KM8553, KM8554 or KM8555, respectively, prepared in the above was passed through the ProSep A column at a flow rate of 70 ml/hour. After washing with 10 ml of 1 M glycine-0.15 M NaCl (pH 8.6) at a flow rate of 1 ml/minute, the column was further washed step by step with 4 ml each of 50 mM citrate buffer of pH 6, 5 and 4 in that order and then 7 ml of 50 mM citrate buffer (pH 3.0) was passed through the column to elute the human CDR-grafted antibody. As a result, anti-human VEGF receptor Flt-1 human CDR-grafted antibodies KM8550, KM8551, KM8552, KM8553, KM8554 and KM8555 were obtained in amounts of 1.1 mg, 1.8 mg, 1.6 mg, 2.2 mg, 1.3 mg and 1.6 mg, respectively.

The thus purified anti-human VEGF receptor Flt-1 human CDR-grafted antibodies KM8550, KM8551, KM8552, KM8553, KM8554 and KM8555 were analyzed by the SDS-PAGE method shown in 2(3) of Reference Example 1. Each of the anti-human VEGF receptor Flt-1 human CDR-grafted antibodies KM8550, KM8551, KM8552, KM8553, KM8554 and KM8555, the anti-human VEGF receptor Flt-1 human chimeric antibody KM2550 shown in 2(4) of Reference Example 1 and KM8969 of IgG type (Japanese Published Unexamined Patent Application No. 257893/98) which reacts to ganglioside GM₂ and does not react to human VEGF receptor Flt-1 as a control human CDR-grafted antibody was applied to the electrophoresis in an amount of 2 µg protein per lane under reducing and non-reducing conditions, and then the gels were stained with Coomassie Brilliant Blue. The results are shown in Fig. 43. The human CDR-grafted antibodies KM8550, KM8551, KM8552, KM8553, KM8554 and KM8555 showed a band of IgG at a position of about 150 kDa under the non-reducing condition, and a band of H chain at a position of about 50 kDa and a band of L chain at about 25 kDa under the reducing condition. This result coincided with that the IgG type antibody is present as a molecule of 150 kDa, and separated into two H chains and two L chains under the reducing conditions caused by the cutting of intermolecular disulfide bond, and it is shown that the human CDR-grafted antibodies KM8550, KM8551, KM8552, KM8553, KM8554 and KM8555 are antibody molecules having a correct structure.

### (3) Binding activities of anti-human VEGF receptor Flt-1 human CDR-grafted antibodies upon human VEGF receptor Flt-1

The activity of purified anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 to bind to the human VEGF receptor Flt-1 was confirmed according to the method in 2(5) of Reference Example 1.

Amount of Flt-1 7N to be coated on the plate was fixed to 2 µg/ml, and concentration of each of the purified antibodies was varied from 1.23 to 900 ng/ml (3-fold dilutions).

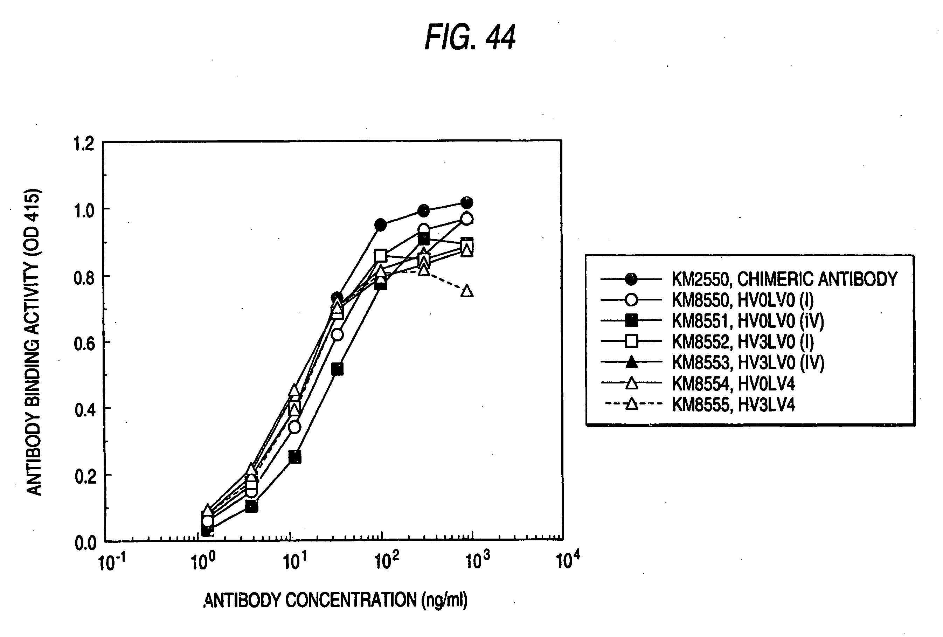

The results are shown in Fig. 44. The anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 bound to the human VEGF receptor - Flt-1 7N in an antibody concentration dependent manner. In addition, the anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 showed almost the same binding activity. In consequence, it was revealed that the six human CDR-grafted antibodies of the present invention keep the binding activity of human chimeric antibody KM2550.

Next, in order to examine binding specificity of anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550, their reactivity for five soluble human VEGF receptor derivative proteins Flt-1 7N, Flt-1 3N, Flt-1 2N (WO 98/22616), Flt-1 7N.K2 (2(6-5) of Reference Example 1) and KDR 7N (2(6-5) of Reference Example 1) was examined using an enzyme immunoassay. Concentration of each soluble human VEGF receptor derivative protein to be adsorbed on a 96 well plate for EIA was fixed to 10 µg/ml (3-fold dilutions), and the antibody concentration was fixed to 5 µg/ml.

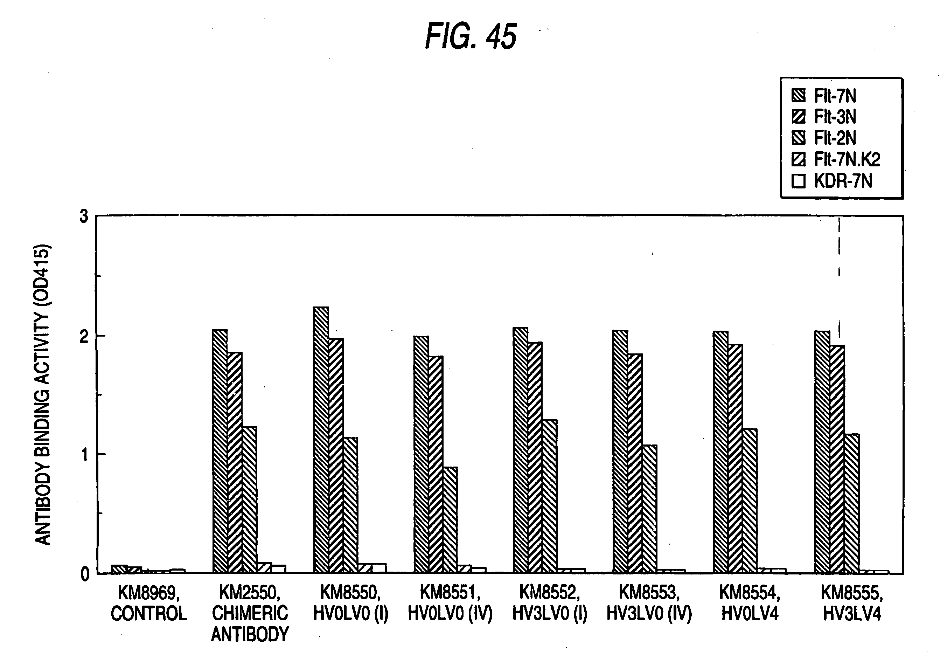

The results are shown in Fig. 45. The anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 showed almost the same reaction specificity, reacting with Flt-1 7N, Flt-1 3N and Flt-1 2N and not reacting with Flt-1 7N.K2 and KDR 7N. The anti-GM₂ human CDR-grafted antibody KM8969 (Japanese Published Unexamined Patent Application No. 257893/98) used as a negative control antibody did not react with the soluble human VEGF receptor derivative proteins tested. In consequence, it was revealed that the six human CDR-grafted antibodies of the present invention keep the binding specificity of human chimeric antibody KM2550.

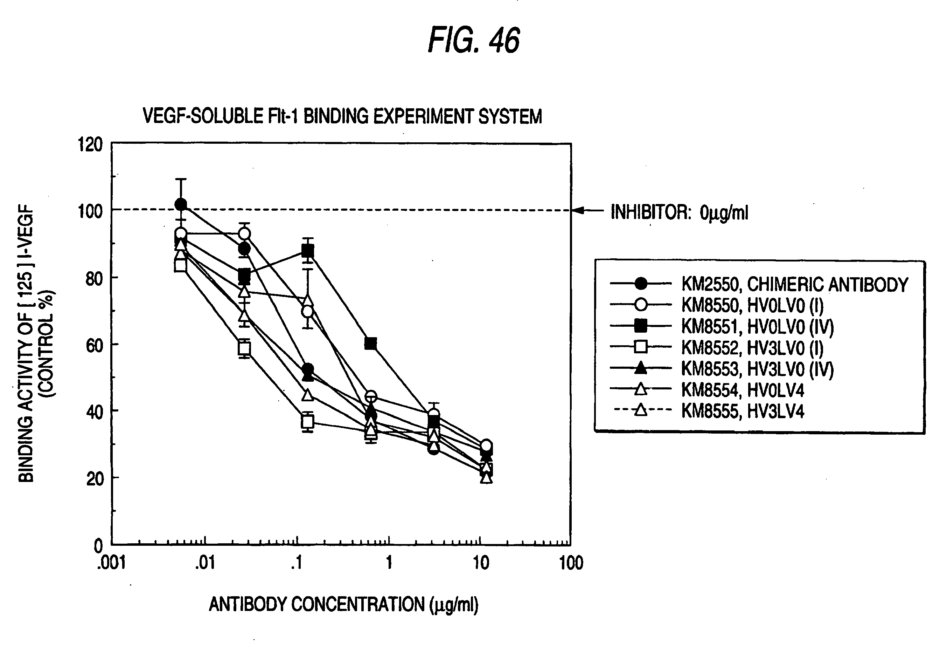

Next, the activity of anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 to inhibit binding between human VEGF and human VEGF receptor Flt-1 was examined in accordance with the method described in 2(5) of Reference Example 1. Concentration of each antibody to be added was varied from 0.0048 to 15 µg/ml (5-fold dilutions). The results are shown in Fig. 46. As shown in Fig. 46, anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 inhibited the binding between human VEGF and human VEGF receptor Flt-1 in an antibody concentration dependent manner. In consequence, it was revealed that the six human CDR-grafted antibodies of the present invention also keep the VEGF-Flt-1 binding inhibition activity of human chimeric antibody KM2550.

### INDUSTRIAL APPLICABILITY

The present invention relates to:
The use of an antibody against a human VEGF receptor Flt-1 in the manufacture of an agent for diagnosing a delayed type hypersensitivity wherein the diagnosis comprises detecting monocyte-macrophage infiltrates in the affected part of the living body.
The use of an antibody against a human VEGF receptor Flt-1 which inhibits signal transduction via a human VEGF receptor Flt-1 in the manufacture of a medicament for treating a delayed type hypersensitivity.

## Claims

1. The use of an antibody against a human VEGF receptor Flt-1 in the manufacture of an agent for diagnosing a delayed type hypersensitivity wherein the diagnosis comprises detecting monocyte-macrophage infiltrates in the affected part of the living body

2. The use of an antibody against a human VEGF receptor Flt-1 which inhibits signal transduction via a human VEGF receptor Flt-1 in the manufacture of a medicament for treating a delayed type hypersensitivity.

3. The use of claim 2, wherein the medicament is a pharmaceutical preparation comprising the antibody and a pharmaceutically acceptable carrier.

4. The use according to any one of claims 1 to 3, wherein the antibody against a human VEGF receptor Flt-1 is a polyclonal antibody or a monoclonal antibody.

5. The use according to claim 4, wherein the monoclonal antibody is an antibody selected from the group consisting of an antibody obtainable from a hybridoma, a humanized antibody and antibody fragments thereof.

6. The use according to claim 5, wherein the antibody obtainable from a hybridoma is an antibody selected from the group consisting of an antibody obtainable from hybridoma KM1730 (FERM BP-5697), an antibody obtainable from hybridoma KM1731 (FERM BP-5718), an antibody obtainable from hybridoma KM1732 (FERM BP-5698), an antibody obtainable from hybridoma KM1748 (FERM BP-5699), and an antibody obtainable from hybridoma KM1750 (FERM BP-5700).

7. The use according to claim 5, wherein the humanized antibody is an antibody selected from the group consisting of a human chimeric antibody and a human complementarity determining region-grafted antibody.

8. The use according to claim 7, wherein the human chimeric antibody is an antibody selected from the group consisting of
an antibody comprising the H chain variable region encoded by DNA contained in Escherichia coli XL-1 Blue MRF'/KM1732HA2 (FERM BP-6354) and the L chain variable region encoded by DNA contained in Escherichia coli XL-1 Blue MRF'/KM1732L2-1 (FERM BP-6352), and
an antibody comprising the H chain variable region encoded by DNA contained in Escherichia coli XL-1 Blue MRF'/KM1750-H2-1 (FERM BP-6353) and the L chain variable region encoded by DNA contained in Escherichia coli XL-1 Blue MRF'/KM1750L3-1 (FERM BP-6355).

9. The use according to claim 7, wherein the human complementary determining region-grafted antibody is selected from the group consisting of
an antibody comprising the H chain variable region encoded by DNA contained in Escherichia coli DH5α/phKM1750HV0 (FERM BP-6719) and the L chain variable region encoded by DNA contained in Escherichia coli XL1-Blue/phKM1750LV0(I) (FERM BP-6716);
an antibody comprising the H chain variable region encoded by DNA contained in Escherichia coli DH5a/phKM1750HV0 (FERM BP-6719) and the L chain variable region encoded by DNA contained in Escherichia coli XL1-Blue/phKM1750LV0(IV) (FERM BP-6717);
an antibody comprising the H chain variable region encoded by DNA contained in Escherichia coli DH5α/phKM1750HV3 (FERM BP-6720) and the L chain variable region encoded by DNA contained in Escherichia coli XL1-Blue/phKM1750LV0(I) (FERM BP-6716);
an antibody comprising the H chain variable region encoded by DNA contained in Escherichia coli DH5α/phKM1750HV3 (FERM BP-6720) and the L chain variable region encoded by DNA contained in Escherichia coli XL1-Blue/phKM1750LV0(IV) (FERM BP-6717);
an antibody comprising the H chain variable region encoded by DNA contained in Escherichia coli DH5α/phKM1750HV0 (FERM BP-6719) and the L chain variable region encoded by DNA contained in Escherichia coli DH5α/phKM1750LV4 (FERM BP-6718); and
an antibody comprising the H chain variable region encoded by DNA contained in Escherichia coli DH5α/phKM1750HV3 (FERM BP-6720) and the L chain variable region encoded by DNA contained in Escherichia coli DH5α/phKM1750LV4 (FERM BP-6718).

10. The use according to claim 5, wherein the antibody fragment is an antibody fragment selected from the group consisting of Fab, Fab', F(ab')₂, a single chain antibody, a disulfide-stabilized Fv and a peptide comprising a complementarity determining region.

11. The use according to any one of claims 1 to 10, wherein the antibody is an antibody fused with a radioisotope, a protein or a low molecular weight agent.

## Patentansprüche

1. Verwendung eines Antikörpers gegen einen humanen VEGF Rezeptor Flt-1 bei der Herstellung eines Mittels zur Diagnose einer Hypersensitivität vom verzögerten Typ, wobei die Diagnose den Nachweis von Monozyten-Makrophagen-Infiltraten in den befallenen Teil des lebenden Körpers umfasst.

2. Verwendung eines Antikörpers gegen einen humanen VEGF Rezeptor Flt-1, der die Signaltransduktion über einen humanen VEGF Rezeptor Flt-1 inhibiert, bei der Herstellung eines Medikaments zur Behandlung einer Hypersensitivität vom verzögerten Typ.

3. Verwendung nach Anspruch 2, wobei das Medikament eine pharmazeutische Zubereitung ist, die den Antikörper und einen pharmazeutisch verträglichen Träger umfasst.

4. Verwendung nach einem der Ansprüche 1 bis 3, wobei der Antikörper gegen einen VEGF Rezeptor Flt-1 ein polyklonaler Antikörper oder ein monoklonaler Antikörper ist.

5. Verwendung nach Anspruch 4, wobei der monoklonale Antikörper ein Antikörper ist, der ausgewählt ist unter einem von einem Hybridom erhältlichen Antikörper, einem humanisierten Antikörper und Antikörperfragmenten davon.

6. Verwendung nach Anspruch 5, wobei der von einem Hybridom erhältliche Antikörper ein Antikörper ist, der ausgewählt ist unter einem vom Hybridom KM1730 (FERM BP-5697) erhältlichen Antikörper, einem vom Hybridom KM1731 (FERM BP-5718) erhältlichen Antikörper, einem vom Hybridom KM1732 (FERM BP-5698) erhältlichen Antikörper, einem vom Hybridom KM1748 (FERM BP-5699) erhältlichen Antikörper und einem vom Hybridom KM 1750 (FERM BP-5700) erhältlichen Antikörper.

7. Verwendung nach Anspruch 5, wobei der humanisierte Antikörper ein Antikörper ist, der ausgewählt ist unter einem humanen chimären Antikörper und einem humanen Antikörper mit verpflanzter komplementaritätsbestimmender Region.

8. Verwendung nach Anspruch 7, wobei der humane chimäre Antikörper ein Antikörper ist, der ausgewählt ist unter
einem Antikörper, der die variable Region der H-Kette, von der in *Escherichia coli* XL-1 Blue MRF'/KM1732HA2 (FERM BP-6354) enthaltenen DNA kodiert, und die variable Region der L-Kette, von der in *Escherichia coli* XL-1 Blue MRF'/KM1732L2-1 (FERM BP-6352) enthaltenen DNA kodiert, umfasst, und
einem Antikörper, der die variable Region der H-Kette, von der in *Escherichia coli* XL-1 Blue MRF'/KM1750-H2-1 (FERM BP-6353) enthaltenen DNA kodiert, und die variable Region der L-Kette, von der in *Escherichia coli* XL-1 Blue MRF'/KM1750L3-1 (FERM BP-6355) enthaltenen DNA kodiert, umfasst.

9. Verwendung nach Anspruch 7, wobei der Antikörper mit verpflanzter komplementaritätsbestimmender Region ausgewählt ist unter
einem Antikörper, der die variable Region der H-Kette, von der in *Escherichia coli* DH5α/phKM1750HV0 (FERM BP-6719) enthaltenen DNA kodiert, und die variable Region der L-Kette, von der in *Escherichia coli* XL1-Blue/phKM1750LV0(I) (FERM BP-6716) enthaltenen DNA kodiert, umfasst;
einem Antikörper, der die variable Region der H-Kette, von der in *Escherichia coli* DH5α/phKM1750HV0 (FERM BP-6719) enthaltenen DNA kodiert, und die variable Region der L-Kette, von der in *Escherichia coli* XL1-Blue/phKM1750LV0(IV) (FERM BP-6717) enthaltenen DNA kodiert, umfasst;
einem Antikörper, der die variable Region der H-Kette, von der in *Escherichia coli* DH5α/phKM1750HV3 (FERM BP-6720) enthaltenen DNA kodiert, und die variable Region der L-Kette, von der in *Escherichia coli* XL1-Blue/phKM1750LV0(I) (FERM BP-6716) enthaltenen DNA kodiert, umfasst;
einem Antikörper, der die variable Region der H-Kette, von der in *Escherichia coli* DH5α/phKM1750HV3 (FERM BP-6720) enthaltenen DNA kodiert, und die variable Region der L-Kette, von der in *Escherichia coli* XL1-Blue/phKM1750LV0(IV) (FERM BP-6717) enthaltenen DNA kodiert, umfasst;
einem Antikörper, der die variable Region der H-Kette, von der in *Escherichia coli* DH5α/phKM1750HV0 (FERM BP-6719) enthaltenen DNA kodiert, und die variable Region der L-Kette, von der in *Escherichia coli* DH5α/phKM1750LV4 (FERM BP-6718) enthaltenen DNA kodiert, umfasst; und
einem Antikörper, der die variable Region der H-Kette, von der in *Escherichia coli* DH5α/phKM1750HV3 (FERM BP-6720) enthaltenen DNA kodiert, und die variable Region der L-Kette, von der in *Escherichia coli* DH5α/phKM1750LV4 (FERM BP-6718) enthaltenen DNA kodiert, umfasst.

10. Verwendung nach Anspruch 5, wobei das Antikörperfragment ein Antikörperfragment ist, das ausgewählt ist unter Fab, Fab', F(ab')₂, einem Einzelkettenantikörper, einem Disulfid-stabilisierten Fv und einem Peptid, das eine komplementaritätsbestimmende Region umfasst.

11. Verwendung nach einem der Ansprüche 1 bis 10, wobei der Antikörper ein Antikörper ist, der mit einem Radioisotop, einem Protein oder einem niedermolekularen Agens fusioniert ist.

## Revendications

1. Utilisation d'un anticorps dirigé contre un récepteur du VEGF humain Flt-1 dans la fabrication d'un agent destiné au diagnostic d'une hypersensibilité de type retardé où le diagnostic comprend la détection d'infiltrats de monocytes-macrophages dans la partie affectée du corps vivant.

2. Utilisation d'un anticorps dirigé contre un récepteur du VEGF humain Flt-1 qui inhibe la transduction des signaux par l'intermédiaire d'un récepteur du VEGF humain Flt-1 dans la fabrication d'un médicament destiné au traitement d'une hypersensibilité de type retardé.

3. Utilisation selon la revendication 2, où le médicament est une préparation pharmaceutique comprenant l'anticorps et un support pharmaceutiquement acceptable.

4. Utilisation selon l'une quelconque des revendications 1 à 3, où l'anticorps dirigé contre un récepteur du VEGF humain Flt-1 est un anticorps polyclonal ou un anticorps monoclonal.

5. Utilisation selon la revendication 4, où l'anticorps monoclonal est un anticorps choisi dans le groupe constitué d'un anticorps pouvant être obtenu à partir un hybridome, d'un anticorps humanisé et de fragments d'anticorps de ceux-ci.

6. Utilisation selon la revendication 5, où l'anticorps pouvant être obtenu à partir d'un hybridome est un anticorps choisi dans le groupe constitué d'un anticorps pouvant être obtenu à partir de l'hybridome KM1730 (FERM BP-5697), d'un anticorps pouvant être obtenu à partir de l'hybridome KM1731 (FERM BP-5718), d'un anticorps pouvant être obtenu à partir de l'hybridome KM1732 (FERM BP-5698), d'un anticorps pouvant être obtenu à partir de l'hybridome KM1748 (FERM BP-5699) et d'un anticorps pouvant être obtenu à partir de l'hybridome KM1750 (FERM BP-5700).

7. Utilisation selon la revendication 5, où l'anticorps humanisé est un anticorps choisi dans le groupe constitué d'un anticorps chimérique humain et d'un anticorps humain à greffe de région déterminant la complémentarité.

8. Utilisation selon la revendication 7, où l'anticorps chimérique humain est un anticorps choisi dans le groupe constitué
d'un anticorps comprenant la région variable de la chaîne H codée par l'ADN contenu dans Escherichia coli XL-1 Blue MRF'/KM1732HA2 (FERM BP-6354) et la région variable de la chaîne L codée par l'ADN contenu dans Escherichia coli XL-1 Blue MRF'/KM1732L2-1 (FERM BP-6352), et
d'un anticorps comprenant la région variable de la chaîne H codée par l'ADN contenu dans Escherichia coli XL-1 Blue MRF'/KM1750-H2-1 (FERM BP-6353) et la région variable de la chaîne L codée par l'ADN contenu dans Escherichia coli XL-1 Blue MRF'/KM1750L3-1 (FERM BP-6355).

9. Utilisation selon la revendication 7, où l'anticorps humain à greffe de région déterminant la complémentarité est choisi dans le groupe constitué
d'un anticorps comprenant la région variable de la chaîne H codée par l'ADN contenu dans Escherichia coli DH5α/phKM1750HV0 (FERM BP-6719) et la région variable de la chaîne L codée par l'ADN contenu dans Escherichia coli XL-1-Blue/phKM1750LV0(I) (FERM BP-6716) ;
d'un anticorps comprenant la région variable de la chaîne H codée par l'ADN contenu dans Escherichia coli DH5α/phKM1750HV0 (FERM BP-6719) et la région variable de la chaîne L codée par l'ADN contenu dans Escherichia coli XL-1-Blue/phKM1750LV0(IV) (FERM BP-6717) ;
d'un anticorps comprenant la région variable de la chaîne H codée par l'ADN contenu dans Escherichia coli DH5α/phKM1750HV3 (FERM BP-6720) et la région variable de la chaîne L codée par l'ADN contenu dans Escherichia coli XL-1-Blue/phKM1750LV0 (I) (FERM BP-6716) ;
d'un anticorps comprenant la région variable de la chaîne H codée par l'ADN contenu dans Escherichia coli DH5α/phKM1750HV3 (FERM BP-6720) et la région variable de la chaîne L codée par l'ADN contenu dans Escherichia coli XL-1-Blue/phKM1750LV0 (IV) (FERM BP-6717) ;
d'un anticorps comprenant la région variable de la chaîne H codée par l'ADN contenu dans Escherichia coli DH5α/phKM1750HV0 (FERM BP-6719) et la région variable de la chaîne L codée par l'ADN contenu dans Escherichia coli DH5α/phKM1750LV4 (FERM BP-6718) ; et
d'un anticorps comprenant la région variable de la chaîne H codée par l'ADN contenu dans Escherichia coli DH5α/phKM1750HV3 (FERM BP-6720) et la région variable de la chaîne L codée par l'ADN contenu dans Escherichia coli DH5α/phKM1750LV4 (FERM BP-6718).

10. Utilisation selon la revendication 5, où le fragment d'anticorps est un fragment d'anticorps choisi dans le groupe constitué de Fab, Fab', F(ab')₂, un anticorps à chaîne unique, un Fv stabilisé par un pont disulfure et un peptide comprenant une région déterminant la complémentarité.

11. Utilisation selon l'une quelconque des revendications 1 à 10, où l'anticorps est un anticorps fusionné avec un radioisotope, une protéine ou un agent de bas poids moléculaire.


### SEQUENCE LISTING

<110> KYOWA HAKKO KOGYO CO., LTD
<120> Diagnostic and therapeutic agents for the diseases related monocyte s and macrophages
<130> 11214W01
<140>
   <141>
<150> H11-171709
   <151> 1999-06-17
<160> 92
<170> PatentIn Ver. 2.0
<210> 1
   <211> 415
   <212> DNA
   <213> Mus musculus
<220>
   <223>
<220>
   <221> CDS
   <222> (1)..(414)
<400> 1
   atg gaa tgg aac tgg gtc gtt ctc ttc ctc ctg tca tta act gca ggt 48
   Met Glu Trp Asn Trp Val Val Leu Phe Leu Leu Ser Leu Thr Ala Gly
   -19             -15                 -10                 -5
   gtc tat gcc cag ggt cag atg cag cag tct gga gct gag ctg gtg aag 96
   Val Tyr Ala Gln Gly Gln Met Gln Gln Ser Gly Ala Glu Leu Val Lys
            -1   1              5                  10
   cct ggg gct tca gtg aag ctg tcc tgc aag cct tct ggc ttc acc ttc 144
   Pro Gly Ala Ser Val Lys Leu Ser Cys Lys Pro Ser Gly Phe Thr Phe
       15                  20                  25
   agt agt aac tat ata agt tgg ttg aag cag aag cct gga cag agt ctt 192
   Ser Ser Asn Tyr Ile Ser Trp Leu Lys Gln Lys Pro Gly Gln Ser Leu
    30                  35                  40                  45
   gag tgg att gct tgg att tat gct gga act ggt gat gcc agc tat aat 240
   Glu Trp Ile Ala Trp Ile Tyr Ala Gly Thr Gly Asp Ala Ser Tyr Asn
                    50                  55                  60
   cag aag ttc aca gcc aag gcc cac gtg act gta gac aca tcc tcc agc 288
   Gln Lys Phe Thr Ala Lys Ala His Val Thr Val Asp Thr Ser Ser Ser
                65                  70                  75
   aca gcc tac atg cag ttg agt agc ctg aca act gag gac tct gcc atc 336
   Thr Ala Tyr Met Gln Leu Ser Ser Leu Thr Thr Glu Asp Ser Ala Ile
            80                  85                  90
   tat tac tgt gca cga cac ggg ggg gac ggc tac tgg ttt gct tac tgg 384
   Tyr Tyr Cys Ala Arg His Gly Gly Asp Gly Tyr Trp Phe Ala Tyr Trp
       95                  100                 105
   ggc caa ggg act ctg gtc act gtc tct gca g                       415
   Gly Gln Gly Thr Leu Val Thr Val Ser Ala
   110                 115                 119
<210> 2
   <211> 385
   <212> DNA
   <213> Mus musculus
<220>
   <223>
<220>
   <221> CDS
   <222> (1)..(384)
<400> 2
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22     -20                     -15                     -10
   gtc ata ata tcc aga gga caa ctt gtt ctc acc cag tct cca gca atc 96
   Val Ile Ile Ser Arg Gly Gln Leu Val Leu Thr Gln Ser Pro Ala Ile
       -5                  -1  1               5                  10
   atg tct gca tct caa ggg gag aag gtc acc atg acc tgc agt gcc agc 144
   Met Ser Ala Ser Gln Gly Glu Lys Val Thr Met Thr Cys Ser Ala Ser
                   15                  20                  25
   tca agt gtc agt tac atg cac tgg tac cag cag aag tca ggc acc tcc 192
   Ser Ser Val Ser Tyr Met His Trp Tyr Gln Gln Lys Ser Gly Thr Ser
               30                  35                  40
   ccc aaa aga tgg att tat gac aca tcc aaa ctg cct tct ggt gtc cct 240
   Pro Lys Arg Trp Ile Tyr Asp Thr Ser Lys Leu Pro Ser Gly Val Pro
           45                  50                  55
   gct cgc ttc agt ggc agt ggg tct ggg acc tct tac tct ctc aca atc 288
   Ala Arg Phe Ser Gly Ser Gly Ser Gly Thr Ser Tyr Ser Leu Thr Ile
       60                  65                  70
   agc agc atg gag gct gaa gat gct gcc act tat tat tgc cag cag tgg 336
   Ser Ser Met Glu Ala Glu Asp Ala Ala Thr Tyr Tyr Cys Gln Gln Trp
   75                  80                  85                  90
   agt agt aac cca ccc acg ttc ggt gct ggg acc aag ctg gaa ctg aaa 384
   Ser Ser Asn Pro Pro Thr Phe Gly Ala Gly Thr Lys Leu Glu Leu Lys
                   95                  100                 105 106
   c                                                               385
<210> 3
   <211> 409
   <212> DNA
   <213> Mus musculus
<220>
   <223>
<220>
   <221> CDS
   <222> (1)..(408)
<400> 3
   atg gga ttc agc agg atc ttt ctc ttc ctc ctg tca gtg act aca ggt 48
   Met Gly Phe Ser Arg Ile Phe Leu Phe Leu Leu Ser Val Thr Thr Gly
   -19             -15                 -10                 -5
   gtc cac tcc cag gct ttt cta cag cag tct ggg gct gag ctg gtg agg 96
   Val His Ser Gln Ala Phe Leu Gln Gln Ser Gly Ala Glu Leu Val Arg
            -1   1               5                  10
   cct ggg gcc tca gtg aag atg tcc tgc aag gct tct ggc tac aca ttt 144
   Pro Gly Ala Ser Val Lys Met Ser Cys Lys Ala Ser Gly Tyr Thr Phe
        15                  20                  25
   atc aat tac aat atg cac tgg gta aag cag aca cct aga cag ggc ctg 192
   Ile Asn Tyr Asn Met His Trp Val Lys Gln Thr Pro Arg Gln Gly Leu
    30                  35                  40                  45
   gaa tgg att gga gct att ttt cca gga aat ggt ttt act tcc tac aat 240
   Glu Trp Ile Gly Ala Ile Phe Pro Gly Asn Gly Phe Thr Ser Tyr Asn
                    50                  55                  60
   cag aag ttc aag ggc aag gcc aca ctg act gta gac aaa tcc tcc agc 288
   Gln Lys Phe Lys Gly Lys Ala Thr Leu Thr Val Asp Lys Ser Ser Ser
                65                  70                  75
   aca gtc tac atg cag ctc cgc agc ctg aca tct gaa gac tct gcg gtc 336
   Thr Val Tyr Met Gln Leu Arg Ser Leu Thr Ser Glu Asp Ser Ala Val
            80                  85                  90
   tat ttc tgt gca aga gat ggt gac tat tac ttt gac tac tgg ggc caa 384
   Tyr Phe Cys Ala Arg Asp Gly Asp Tyr Tyr Phe Asp Tyr Trp Gly Gln
        95                 100                 105
   ggc acc act ctc aca gtc tcc tca g                               409
   Gly Thr Thr Leu Thr Val Ser Ser
   110                 115     117
<210> 4
   <211> 379
   <212> DNA
   <213> Mus musculus
<220>
   <223>
<220>
   <221> CDS
   <222> (1)..(378)
<400> 4
   atg gat ttt cag gtg cag att ttc agc ttc ctg cta atc agt gcc tca 48
   Met Asp Phe Gln Val Gln Ile Phe Ser Phe Leu Leu Ile Ser Ala Ser
   -22     -20                 -15                     -10
   gtc ata atg tcc aga gga caa att gtt ctc acc cag tcg cca gca atc 96
   Val Ile Met Ser Arg Gly Gln Ile Val Leu Thr Gln Ser Pro Ala Ile
       -5              -1   1               5                   10
   atg tct gca tct cta ggg gag gag atc acc cta acc tgc agt gcc agc 144
   Met Ser Ala Ser Leu Gly Glu Glu Ile Thr Leu Thr Cys Ser Ala Ser
                       15                  20                  25
   tcg agt gta agt tac atg cac tgg tac cag cag aag tca ggc act tct 192
   Ser Ser Val Ser Tyr Met His Trp Tyr Gln Gln Lys Ser Gly Thr Ser
                   30                  35                  40
   ccc aaa ctc ttg att tat aga aca tcc aac ctg gct tct gga gtc cct 240
   Pro Lys Leu Leu Ile Tyr Arg Thr Ser Asn Leu Ala Ser Gly Val Pro
               45                  50                  55
   ttt cgc ttc agt ggc agt ggg tct ggg acc ttt tat tct ctc aca atc 288
   Phe Arg Phe Ser Gly Ser Gly Ser Gly Thr Phe Tyr Ser Leu Thr Ile
       60                  65                  70
   agc agt gtg gag gct gaa gat gct gcc gat tat tac tgc cat cag tgg 336
   Ser Ser Val Glu Ala Glu Asp Ala Ala Asp Tyr Tyr Cys His Gln Trp
   75                  80                  85                  90
   agt atg tac acg ttc gga ggg ggg acc aag ctg gaa ata aaa c       379
   Ser Met Tyr Thr Phe Gly Gly Gly Thr Lys Leu Glu Ile Lys
                   95                  100             104
<210> 5
   <211> 5
   <212> PRT
   <213> Mus musculus
<220>
   <223>
<400> 5
   Ser Asn Tyr Ile Ser
    1               5
<210> 6
   <211> 17
   <212> PRT
   <213> Mus musculus
<220>
   <223>
<400> 6
   Trp Ile Tyr Ala Gly Thr Gly Asp Ala Ser Tyr Asn Gln Lys Phe Thr
   1 5 10 15
   Ala
   17
<210> 7
   <211> 10
   <212> PRT
   <213> Mus musculus
<220>
   <223>
<400> 7
   His Gly Gly Asp Gly Tyr Trp Phe Ala Tyr
   1 5 10
<210> 8
   <211> 10
   <212> PRT
   <213> Mus musculus
<220>
   <223>
<400> 8
   Ser Ala Ser Ser Ser Val Ser Tyr Met His
   1 5 10
<210> 9
   <211> 7
   <212> PRT
   <213> Mus musculus
<220>
   <223>
<400> 9
   Asp Thr Ser Lys Leu Pro Ser
   1 5 7
<210> 10
   <211> 9
   <212> PRT
   <213> Mus musculus
<220>
   <223>
<400> 10
   Gln Gln Trp Ser Ser Asn Pro Pro Thr
   1 5 9
<210> 11
   <211> 5
   <212> PRT
   <213> Mus musculus
<220>
   <223>
<400> 11
   Asn Tyr Asn Met His
   1 5
<210> 12
   <211> 17
   <212> PRT
   <213> Mus musculus
<220>
   <223>
<400> 12
   Ala Ile Phe Pro Gly Asn Gly Phe Thr Ser Tyr Asn Gln Lys Phe Lys
   1 5 10 15
   Gly
   17
<210> 13
   <211> 8
   <212> PRT
   <213> Mus musculus
<220>
   <223>
<400> 13
   Asp Gly Asp Tyr Tyr Phe Asp Tyr
   1 5 8
<210> 14
   <211> 10
   <212> PRT
   <213> Mus musculus
<220>
   <223>
<400> 14
   Ser Ala Ser Ser Ser Val Ser Tyr Met His
   1 5 10
<210> 15
   <211> 7
   <212> PRT
   <213> Mus musculus
<220>
   <223>
<400> 15
   Arg Thr Ser Asn Leu Ala Ser
   1 5 7
<210> 16
   <211> 7
   <212> PRT
   <213> Mus musculus
<220>
   <223>
<400> 16
   His Gln Trp Ser Met Tyr Thr
   1 5 7
<210> 17
   <211> 45
   <212> DNA
   <213> Artificial Sequence
<220>
   <223> Description of Artificial Sequence: Synthetic DNA
<400> 17
   gccaagggac tctggtcact gtctctgcag cctccaccaa gggcc 45
<210> 18
   <211> 41
   <212> DNA
   <213> Artificial Sequence
<220>
   <223> Description of Artificial Sequence: Synthetic DNA
<400> 18
   cttggtggag gctgcagaga cagtgaccag agtcccttgg c 41
<210> 19
   <211> 69
   <212> DNA
   <213> Artificial Sequence
<220>
   <223> Description of Artificial Sequence: Synthetic DNA
<400> 19
   cttattattg ccagcagtgg agtagtaacc cacccacgtt cggtgctggg accaagctgg 60
   aactgaaac 69
<210> 20
   <211> 74
   <212> DNA
   <213> Artificial Sequence
<220>
   <223> Description of Artificial Sequence: Synthetic DNA
<400> 20
   gtacgtttca gttccagctt ggtcccagca ccgaacgtgg gtgggttact actccactgc 60
   tggcaataat aagt 74
<210> 21
   <211> 19
   <212> DNA
   <213> Artificial Sequence
<220>
   <223> Description of Artificial Sequence: Synthetic DNA
<400> 21
   tcagcctcca ccaagggcc 19
<210> 22
   <211> 11
   <212> DNA
   <213> Artificial Sequence
<220>
   <223> Description of Artificial Sequence: Synthetic DNA
<400> 22
   cttggtggag g 11
<210> 22
   <211> 33
   <212> DNA
   <213> Artificial Sequence
<220>
   <223> Description of Artificial Sequence: Synthetic DNA
<400> 23
   cgttcggagg ggggaccaag ctggaaataa aac 33
<210> 24
   <211> 35
   <212> DNA
   <213> Artificial Sequence
<220>
   <223> Description of Artificial Sequence: Synthetic DNA
<400> 24
   gtacgtttta tttccagctt ggtcccccct ccgaa 35
<210> 25
   <211> 94
   <212> DNA
   <213> Artificial Sequence
<220>
   <223> Description of Artificial Sequence: Synthetic DNA
<400> 25
   caggaaacag ctatgacgcg gccgccacca tggaatggaa ctgggtcgtt ctcttcctcc 60
   tgtcattaac tgcaggtgtc tatgcccagg tgca 94
<210> 26
   <211> 95
   <212> DNA
   <213> Artificial Sequence
<220>
   <223> Description of Artificial Sequence: Synthetic DNA
<400> 26
   gtgtatccag aagccttgca ggagaccttc actgaggcgc caggcttctt cacctcagcc 60
   ccagactgca ccagctgcac ctgggcatag acacc 95
<210> 27.
   <211> 94
   <212> DNA
   <213> Artificial Sequence
<220>
   <223> Description of Artificial Sequence: Synthetic DNA
<400> 27
   tgcaaggctt ctggatacac cttcagcagt aactatataa gttgggtgcg acaggcccct 60
   ggacaagggc ttgagtggat gggatggatt tatg 94
<210> 28
   <211> 101
   <212> DNA
   <213> Artificial Sequence
<220>
   <223> Description of Artificial Sequence: Synthetic DNA
<400> 28
   aggctgtgct cgtggatgtg tcgacggtaa tggtgactct ggctgtgaac ttctgattat 60
   agctggcatc accagttcca gcataaatcc atcccatcca c 101
<210> 29
   <211> 100
   <212> DNA
   <213> Artificial Sequence
<220>
   <223> Description of Artificial Sequence: Synthetic DNA
<400> 29
   cacatccacg agcacagcct acatggagct gagcagcctg agatctgagg acacggccgt 60
   gtattactgt gcgagacacg ggggggacgg ctactggttt 100
<210> 30
   <211> 93
   <212> DNA
   <213> Artificial Sequence
<220>
   <223> Description of Artificial Sequence: Synthetic DNA
<400> 30
   gttttcccag tcacgacggg cccttggtgg aggctgagga gacggtgacc agggttccct 60
   ggccccagta agcaaaccag tagccgtccc ccc 93
<210> 31
   <211> 421
   <212> DNA
   <213> Artificial Sequence
<220>
   <223> Description of Artificial Sequence:Synthetic DNA
<220>
   <221> CDS
   <222> (1)..(420)
<400> 31
<210> 32
   <211> 96
   <212> DNA
   <213> Artificial Sequence
<220>
   <223> Description of Artificial Sequence: Synthetic DNA
<400> 32
   caggaaacag ctatgacgcg gccgccacca tgggattcag caggatcttt ctcttcctcc 60
   tgtcagtgac tacaggtgtc cactcccagg tgcagc                           96
<210> 33
   <211> 94
   <212> DNA
   <213> Artificial Sequence
<220>
   <223> Description of Artificial Sequence: Synthetic DNA
<400> 33
   ggtgtatcca gaagccttgc aggagacctt cactgaggcc ccaggcttct tcacctcagc 60
   tccggactgc accagctgca cctgggagtg gaca 94
<210> 34
   <211> 90
   <212> DNA
   <213> Artificial Sequence
<220>
   <223> Description of Artificial Sequence: Synthetic DNA
<400> 34
   gcaaggcttc tggatacacc ttcattaatt acaatatgca ctgggtgcga caggcccctg 60
   gacaagggct tgagtggatg ggagctattt 90
<210> 35
   <211> 90
   <212> DNA
   <213> Artificial Sequence
<220>
   <223> Description of Artificial Sequence: Synthetic DNA
<400> 35
   tgtgctcgtg gacttgtcga cggtaatggt gactctgccc ttgaacttct gattgtagga 60
   agtaaaacca tttcctggaa aaatagctcc 90
<210> 36
   <211> 95
   <212> DNA
   <213> Artificial Sequence
<220>
   <223> Description of Artificial Sequence: Synthetic DNA
<400> 36
   tcgacaagtc cacgagcaca gcctacatgg agctgagcag cctgagatct gaggacacgg 60
   ccgtgtatta ctgtgcgaga gatggtgact attac 95
<210> 37
   <211> 96
   <212> DNA
   <213> Artificial Sequence
<220>
   <223> Description of Artificial Sequence: Synthetic DNA
<400> 37
   gttttcccag tcacgacggg cccttggtgg aggctgagga gacggtgacc agggttccct 60
   ggccccagta gtcaaagtaa tagtcaccat ctctcg 96
<210> 38
   <211> 409
   <212> DNA
   <213> Artificial Sequence
<220>
   <223> Description of Artificial Sequence:Synthetic DNA
<220>
   <221> CDS
   <222> (1)..(408)
<400> 38
   atg gga ttc agc agg atc ttt ctc ttc ctc ctg tca gtg act aca ggt 48
   Met Gly Phe Ser Arg Ile Phe Leu Phe Leu Leu Ser Val Thr Thr Gly
   -19              -15                   -10                   -5
   gtc cac tcc cag gtg cag ctg gtg cag tcc gga gct gag gtg aag aag 96
   Val His Ser Gln Val Gln Leu Val Gln Ser Gly Ala Glu Val Lys Lys
            -1   1               5                   10
   cct ggg gcc tca gtg aag gtc tcc tgc aag gct tct gga tac acc ttc 144
   Pro Gly Ala Ser Val Lys Val Ser Cys Lys Ala Ser Gly Tyr Thr Phe
       15                  20                  25
   att aat tac aat atg cac tgg gtg cga cag gcc cct gga caa ggg ctt 192
   Ile Asn Tyr Asn Met His Trp Val Arg Gln Ala Pro Gly Gln Gly Leu
   30                  35                  40                  45
   gag tgg atg gga gct att ttt cca gga aat ggt ttt act tcc tac aat 240
   Glu Trp Met Gly Ala Ile Phe Pro Gly Asn Gly Phe Thr Ser Tyr Asn
                   50                  55                  60
   cag aag ttc aag ggc aga gtc acc att acc gtc gac aag tcc acg agc 288
   Gln Lys Phe Lys Gly Arg Val Thr Ile Thr Val Asp Lys Ser Thr Ser
               65                  70                  75
   aca gcc tac atg gag ctg agc agc ctg aga tct gag gac acg gcc gtg 336
   Thr Ala Tyr Met Glu Leu Ser Ser Leu Arg Ser Glu Asp Thr Ala Val
           80                  85                  90
   tat tac tgt gcg aga gat ggt gac tat tac ttt gac tac tgg ggc cag 384
   Tyr Tyr Cys Ala Arg Asp Gly Asp Tyr Tyr Phe Asp Tyr Trp Gly Gln
       95                  100                 105
   gga acc ctg gtc acc gtc tcc tca g                               409
   Gly Thr Leu Val Thr Val Ser Ser
   110                 115     117
<210> 39
   <211> 87
   <212> DNA
   <213> Artificial Sequence
<220>
   <223> Description of Artificial Sequence: Synthetic DNA
<400> 39
   caggaaacag ctatgacgaa ttccaccatg gattttcaag tgcagatttt cagcttcctg 60
   ctaatcagtg cctcagtcat aatatcc                                     87
<210> 40
   <211> 93
   <212> DNA
   <213> Artificial Sequence
<220>
   <223> Description of Artificial Sequence: Synthetic DNA
<400> 40
   aagtgatggt gactctgtct cctacagatg cagacaggga ggatggagac tgggtcatct 60
   ggatatctcc tctggatatt atgactgagg cac 93
<210> 41
   <211> 85
   <212> DNA
   <213> Artificial Sequence
<220>
   <223> Description of Artificial Sequence: Synthetic DNA
<400> 41
   agacagagtc accatcactt gtagtgctag ctcaagtgtc agttacatgc actggtatca 60
   gcagaaacca gggaaagccc ctaag 85
<210> 42
   <211> 84
   <212> DNA
   <213> Artificial Sequence
<220>
   <223> Description of Artificial Sequence: Synthetic DNA
<400> 42
   atccactgcc gctgaacctt gatgggaccc cagaaggcag tttggatgtg tcatagatca 60
   gaagcttagg ggctttccct ggtt 84
<210> 43
   <211> 94
   <212> DNA
   <213> Artificial Sequence
<220>
   <223> Description of Artificial Sequence: Synthetic DNA
<400> 43
   aaggttcagc ggcagtggat ctgggacaga tttcactctc accatcagca gcctgcagcc 60
   tgaagatttt gcaacttatt actgtcagca gtgg 94
<210> 44
   <211> 91
   <212> DNA
   <213> Artificial Sequence
<220>
   <223> Description of Artificial Sequence: Synthetic DNA
<400> 44
   gttttcccag tcacgaccgt acgtttgatc tctaccttgg tcccttggcc gaacgtgggt 60
   gggttactac tccactgctg acagtaataa g                                91
<210> 45
   <211> 385
   <212> DNA
   <213> Artificial Sequence
<220>
   <223> Description of Artificial Sequence:Synthetic DNA
<220>
   <221> CDS
   <222> (1)..(384)
<400> 45
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22     -20                 -15                 -10
   gtc ata ata tcc aga gga gat atc cag atg acc cag tct cca tcc tcc 96
   Val Ile Ile Ser Arg Gly Asp Ile Gln Met Thr Gln Ser Pro Ser Ser
       -5              -1  1               5                   10
   ctg tct gca tct gta gga gac aga gtc acc atc act tgt agt gct agc 144
   Leu Ser Ala Ser Val Gly Asp Arg Val Thr Ile Thr Cys Ser Ala Ser
                   15                  20                  25
   tca agt gtc agt tac atg cac tgg tat cag cag aaa cca ggg aaa gcc 192
   Ser Ser Val Ser Tyr Met His Trp Tyr Gln Gln Lys Pro Gly Lys Ala
               30                  35                  40
   cct aag ctt ctg atc tat gac aca tcc aaa ctg cct tct ggg gtc cca 240
   Pro Lys Leu Leu Ile Tyr Asp Thr Ser Lys Leu Pro Ser Gly Val Pro
           45                  50                  55
   tca agg ttc agc ggc agt gga tct ggg aca gat ttc act ctc acc atc 288
   Ser Arg Phe Ser Gly Ser Gly Ser Gly Thr Asp Phe Thr Leu Thr Ile
       60                  65                  70
   agc agc ctg cag cct gaa gat ttt gca act tat tac tgt cag cag tgg 336
   Ser Ser Leu Gln Pro Glu Asp Phe Ala Thr Tyr Tyr Cys Gln Gln Trp
   75                  80                  85                  90
   agt agt aac cca ccc acg ttc ggc caa ggg acc aag gta gag atc aaa 384
   Ser Ser Asn Pro Pro Thr Phe Gly Gln Gly Thr Lys Val Glu Ile Lys
   95 100 105 106
   c 385
<210> 46
   <211> 84
   <212> DNA
   <213> Artificial Sequence
<220>
   <223> Description of Artificial Sequence: Synthetic DNA
<400> 46
   caggaaacag ctatgacgaa ttccaccatg gattttcagg tgcagatttt cagcttcctg 60
   ctaatcagtg cctcagtcat aatg 84
<210> 47
   <211> 96
   <212> DNA
   <213> Artificial Sequence
<220>
   <223> Description of Artificial Sequence: Synthetic DNA
<400> 47
   agttgatggt ggccctctcg cccagagaca cagccaggga gtctggagac tgggtcatca 60
   cgatgtctcc tctggacatt atgactgagg cactga 96
<210> 48
   <211> 85
   <212> DNA
   <213> Artificial Sequence
<220>
   <223> Description of Artificial Sequence: Synthetic DNA
<400> 48
   cgagagggcc accatcaact gcagtgccag ctcgagtgta agttacatgc actggtacca 60
   gcagaaacca ggacagcctc ctaag 85
<210> 49
   <211> 87
   <212> DNA
   <213> Artificial Sequence
<220>
   <223> Description of Artificial Sequence: Synthetic DNA
<400> 49
   cagacccgct gccactgaat cggtcaggga ccccagaagc caggttggat gttctgtaaa 60
   tgagcagctt aggaggctgt cctggtt 87
<210> 50
   <211> 86
   <212> DNA
   <213> Artificial Sequence
<220>
   <223> Description of Artificial Sequence: Synthetic DNA
<400> 50
   attcagtggc agcgggtctg ggacagattt cactctcacc atcagcagcc tgcaggctga 60
   agacgtcgca gtttattact gtcatc 86
<210> 51
   <211> 90
   <212> DNA
   <213> Artificial Sequence
<220>
   <223> Description of Artificial Sequence: Synthetic DNA
<400> 51
   gttttcccag tcacgaccgt acgtttgatc tccaccttgg tcccttggcc gaacgtgtac 60
   atactccact gatgacagta ataaactgcg 90
<210> 52
   <211> 379
   <212> DNA
   <213> Artificial Sequence
<220>
   <223> Description of Artificial Sequence:Synthetic DNA
<220>
   <221> CDS
   <222> (1)..(378)
<400> 52 .
   atg gat ttt cag gtg cag att ttc agc ttc ctg cta atc agt gcc tca    48
   Met Asp Phe Gln Val Gln Ile Phe Ser Phe Leu Leu Ile Ser Ala Ser
   -22     -20                 -15                 -10
   gtc ata atg tcc aga gga gac atc gtg atg acc cag tct cca gac tcc    96
   Val Ile Met Ser Arg Gly Asp Ile Val Met Thr Gln Ser Pro Asp Ser
        -5              -1   1               5                  10
   ctg gct gtg tct ctg ggc gag agg gcc acc atc aac tgc agt gcc agc   144
   Leu Ala Val Ser Leu Gly Glu Arg Ala Thr Ile Asn Cys Ser Ala Ser
                       15                  20                  25
   tcg agt gta agt tac atg cac tgg tac cag cag aaa cca gga cag cct   192
   Ser Ser Val Ser Tyr Met His Trp Tyr Gln Gln Lys Pro Gly Gln Pro
                   30                  35                  40
   cct aag ctg ctc att tac aga aca tcc aac ctg gct tct ggg gtc cct   240
   Pro Lys Leu Leu Ile Tyr Arg Thr Ser Asn Leu Ala Ser Gly Val Pro
               45                  50                  55
   gac cga ttc agt ggc agc ggg tct ggg aca gat ttc act ctc acc atc   288
   Asp Arg Phe Ser Gly Ser Gly Ser Gly Thr Asp Phe Thr Leu Thr Ile
        60                  65                  70
   agc agc ctg cag gct gaa gac gtc gca gtt tat tac tgt cat cag tgg   336
   Ser Ser Leu Gln Ala Glu Asp Val Ala Val Tyr Tyr Cys His Gln Trp
    75                  80                  85                  90
   agt atg tac acg ttc ggc caa ggg acc aag gtg gag atc aaa c         379
   Ser Met Tyr Thr Phe Gly Gln Gly Thr Lys Val Glu Ile Lys
                   95                 100                 104
<210> 53
   <211> 8
   <212> DNA
   <213> Artificial Sequence
<220>
   <223> Description of Artificial Sequence: Synthetic DNA
<400> 53
   ctctagag 8
<210> 54
   <211> 60
   <212> DNA
   <213> Artificial Sequence
<220>
   <223> Description of Artificial Sequence: Synthetic DNA
<400> 54
   cagtgttctt ggctgtgcaa aaagtggagg catttttcat aatagaaggt gcctacgtag 60
<210> 55
   <211> 67
   <212> DNA
   <213> Artificial Sequence
<220>
   <223> Description of Artificial Sequence: Synthetic DNA
<400> 55
   gatcctacgt aggcaccttc tattatgaaa aatgcctcca cttttgcaca gccaagaaca 60
   ctgcatg 67
<210> 56
   <211> 18
   <212> DNA
   <213> Artificial Sequence
<220>
   <223> Description of Artificial Sequence: Synthetic DNA
<400> 56
   GTATAATGAG CGGCCGCG 18
<210> 57
   <211> 22
   <212> DNA
   <213> Artificial Sequence
<220>
   <223> Description of Artificial Sequence: Synthetic DNA
<400> 57
   gatccgcggc cgctcattat ac 22
<210> 58
   <211> 56
   <212> DNA
   <213> Artificial Sequence
<220>
   <223> Description of Artificial Sequence: Synthetic DNA
<400> 58
   gaaggaaaca gaaggcgcca tctatatatt tattcgaggt accaatacaa tcatag 56
<210> 59
   <211> 30
   <212> DNA
   <213> Artificial Sequence
<220>
   <223> Description of Artificial Sequence: Synthetic DNA
<400> 59
   aaactgactt ggccggcgcc atttatgtct 30
<210> 60
   <211> 30
   <212> DNA
   <213> Artificial Sequence
<220>
   <223> Description of Artificial Sequence: Synthetic DNA
<400> 60
   cataaatcct ataggtacca acgacaacta 30
<210> 61
   <211> 87
   <212> DNA
   <213> Artificial Sequence
<220>
   <223> Description of Artificial Sequence: Synthetic DNA
<400> 61
   caggaaacag ctatgacgaa ttccaccatg gattttcaag tgcagatttt cagcttcctg 60
   ctaatcagtg cctcagtcat aatatcc 87
<210> 62
   <211> 93
   <212> DNA
   <213> Artificial Sequence
<220>
   <223> Description of Artificial Sequence: Synthetic DNA
<400> 62
   aagtgatggt gactctgtct cctacagatg cagacaggga ggatggagac tgggtcatct 60
   ggatatctcc tctggatatt atgactgagg cac 93
<210> 63
   <211> 8
   <212> DNA
   <213> Artificial Sequence
<220>
   <223> Description of Artificial Sequence: Synthetic DNA
<400> 63
<210> 64
   <211> 84
   <212> DNA
   <213> Artificial Sequence
<220>
   <223> Description of Artificial Sequence: Synthetic DNA
<400> 64
   atccactgcc gctgaacctt gatgggaccc cagaagccag gttggatgtt ctatagatca 60
   gaagcttagg ggctttccct ggtt 84
<210> 65
   <211> 94
   <212> DNA
   <213> Artificial Sequence
<220>
   <223> Description of Artificial Sequence: Synthetic DNA
<400> 65
   aaggttcagc ggcagtggat ctgggacaga tttcactctc accatcagca gcctgcagcc 60
   tgaagatttt gcaacttatt actgtcatca gtgg 94
<210> 66
   <211> 85
   <212> DNA
   <213> Artificial Sequence
<220>
   <223> Description of Artificial Sequence: Synthetic DNA
<400> 66
   gttttcccag tcacgaccgt acgtttgatc tctaccttgg tcccttggcc gaacgtgtac 60
   atactccact gatgacagta ataag 85
<210> 67
   <211> 379
   <212> DNA
   <213> Artificial Sequence
<220>
   <223> Description of Artificial Sequence:Synthetic DNA
<220>
   <221> CDS
   <222> (1)..(378)
<400> 67
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22     -20                 -15                 -10
   gtc ata ata tcc aga gga gat atc cag atg acc cag tct cca tcc tcc 96
   Val Ile Ile Ser Arg Gly Asp Ile Gln Met Thr Gln Ser Pro Ser Ser
       -5                  -1   1               5                10
   ctg tct gca tct gta gga gac aga gtc acc atc act tgt agt gcc agc 144
   Leu Ser Ala Ser Val Gly Asp Arg Val Thr Ile Thr Cys Ser Ala Ser
                       15                  20                  25
   tcg agt gta agt tac atg cac tgg tat cag cag aaa cca ggg aaa gcc 192
   Ser Ser Val Ser Tyr Met His Trp Tyr Gln Gln Lys Pro Gly Lys Ala
                   30                  35                  40
   cct aag ctt ctg atc tat aga aca tcc aac ctg gct tct ggg gtc cca 240
   Pro Lys Leu Leu Ile Tyr Arg Thr Ser Asn Leu Ala Ser Gly Val Pro
           45                  50                  55
   tca agg ttc agc ggc agt gga tct ggg aca gat ttc act ctc acc atc 288
   Ser Arg Phe Ser Gly Ser Gly Ser Gly Thr Asp Phe Thr Leu Thr Ile
       60                  35                  70
   agc agc ctg cag cct gaa gat ttt gca act tat tac tgt cat cag tgg 336
   Ser Ser Leu Gln Pro Glu Asp Phe Ala Thr Tyr Tyr Cys His Gln Trp
   75                  80                  85                  90
   agt atg tac acg ttc ggc caa ggg acc aag gta gag atc aaa c       379
   Ser Met Tyr Thr Phe Gly Gln Gly Thr Lys Val Glu Ile Lys
                   95                  100             104
<210> 68
   <211> 80
   <212> DNA
   <213> Artificial Sequence
<220>
   <223> Description of Artificial Sequence: Synthetic DNA
<400> 68
   caggaaacag ctatgactcc ggagctgagg tgaagaagcc tggggcctca gtgaaggtct 60
   cctgcaaggc ttctggatac                                             80
<210> 69
   <211> 80
   <212> DNA
   <213> Artificial Sequence
<220>
   <223> Description of Artificial Sequence: Synthetic DNA
<400> 69
   ccactcaagc ccttgtccag gggcctgtcg cacccagtgc atattgtaat taatgaaggt 60
   gtatccagaa gccttgcagg 80
<210> 70
   <211> 81
   <212> DNA
   <213> Artificial Sequence
<220>
   <223> Description of Artificial Sequence: Synthetic DNA
<400> 70
   ctggacaagg gcttgagtgg atgggagcta tttttccagg aaatggtttt acttcctaca 60
   atcagaagtt caagggcaga g 81
<210> 71
   <211> 79
   <212> DNA
   <213> Artificial Sequence
<220>
   <223> Description of Artificial Sequence: Synthetic DNA
<400> 71
   tctcaggctg cgcagctgca tgtaggctgt gctcgtggac ttgtcgacgg taatggtgac 60
   tctgcccttg aacttctga 79
<210> 72
   <211> 83
   <212> DNA
   <213> Artificial Sequence
<220>
   <223> Description of Artificial Sequence: Synthetic DNA
<400> 72
   tgcagctgcg cagcctgaga tctgaggaca cggccgtgta tttctgtgcg agagatggtg 60
   actattactt tgactactgg ggc 83
<210> 73
   <211> 81
   <212> DNA
   <213> Artificial Sequence
<220>
   <223> Description of Artificial Sequence: Synthetic DNA
<400> 73
   gttttcccag tcacgacggg cccttggtgg aggctgagga gacggtgacc agggttccct 60
   ggccccagta gtcaaagtaa t 81
<210> 74
   <211> 409
   <212> DNA
   <213> Artificial Sequence
<220>
   <223> Description of Artificial Sequence:Synthetic DNA
<220>
   <221> CDS
   <222> (1)..(408)
<400> 74
   atg gga ttc agc agg atc ttt ctc ttc ctc ctg tca gtg act aca ggt  48
   Met Gly Phe Ser Arg Ile Phe Leu Phe Leu Leu Ser Val Thr Thr Gly
   -19             -15                 -10                 -5
   gtc cac tcc cag gtg cag ctg gtg cag tcc gga gct gag gtg aag aag  96
   Val His Ser Gln Val Gln Leu Val Gln Ser Gly Ala Glu Val Lys Lys
            -1   1               5                  10
   cct ggg gcc tca gtg aag gtc tcc tgc aag gct tct gga tac acc ttc 144
   Pro Gly Ala Ser Val Lys Val Ser Cys Lys Ala Ser Gly Tyr Thr Phe
        15                  20                  25
   att aat tac aat atg cac tgg gtg cga cag gcc cct gga caa ggg ctt 192
   Ile Asn Tyr Asn Met His Trp Val Arg Gln Ala Pro Gly Gln Gly Leu
    30                  35                  40                  45
   gag tgg atg gga gct att ttt cca gga aat ggt ttt act tcc tac aat 240
   Glu Trp Met Gly Ala Ile Phe Pro Gly Asn Gly Phe Thr Ser Tyr Asn
                    50                  55                  60
   cag aag ttc aag ggc aga gtc acc att acc gtc gac aag tcc acg agc 288
   Gln Lys Phe Lys Gly Arg Val Thr Ile Thr Val Asp Lys Ser Thr Ser
                65                  70                  75
   aca gcc tac atg cag ctg cgc agc ctg aga tct gag gac acg gcc gtg 336
   Thr Ala Tyr Met Gln Leu Arg Ser Leu Arg Ser Glu Asp Thr Ala Val
            80                  85                  90
   tat ttc tgt gcg aga gat ggt gac tat tac ttt gac tac tgg ggc cag 384
   Tyr Phe Cys Ala Arg Asp Gly Asp Tyr Tyr Phe Asp Tyr Trp Gly Gln
        95                 100                 105
   gga acc ctg gtc acc gtc tcc tca g                                409
   Gly Thr Leu Val Thr Val Ser Ser
   110                 115     117
<210> 75
   <211> 87
   <212> DNA
   <213> Artificial Sequence
<220>
   <223> Description of Artificial Sequence: Synthetic DNA
<400> 75
   caggaaacag ctatgacgaa ttccaccatg gattttcaag tgcagatttt cagcttcctg  60
   ctaatcagtg cctcagtcat aatatcc                                        87
<210> 76
   <211> 93
   <212> DNA
   <213> Artificial Sequence
<220>
   <223> Description of Artificial Sequence: Synthetic DNA
<400> 76
   aagtgatggt gacctcctct cctacagatg cagacaggga ggatggagac tgggtcatct 60
   ggatatctcc tctggatatt atgactgagg cac 93
<210> 77
   <211> 85
   <212> DNA
   <213> Artificial Sequence
<220>
   <223> Description of Artificial Sequence: Synthetic DNA
<400> 77
   agaggaggtc accatcactt gtagtgccag ctcgagtgta agttacatgc actggtatca 60
   gcagaaacca gggaaagccc ctaag 85
<210> 78
   <211> 84
   <212> DNA
   <213> Artificial Sequence
<220>
   <223> Description of Artificial Sequence: Synthetic DNA
<400> 78
   atccactgcc gctgaacctt gatgggaccc cagaagccag gttggatgtt ctatagatca 60
   gaagcttagg ggctttccct ggtt 84
<210> 79
   <211> 94
   <212> DNA
   <213> Artificial Sequence
<220>
   <223> Description of Artificial Sequence: Synthetic DNA
<400> 79
   aaggttcagc ggcagtggat ctgggacatt ttatactctc accatcagca gcctgcagcc 60
   tgaagatttt gcaacttatt actgtcatca gtgg 94
<210> 80
   <211> 85
   <212> DNA
   <213> Artificial Sequence
<220>
   <223> Description of Artificial Sequence: Synthetic DNA
<400> 80
   gttttcccag tcacgaccgt acgtttgatc tctaccttgg tcccttggcc gaacgtgtac 60
   atactccact gatgacagta ataag 85
<210> 81
   <211> 379
   <212> DNA
   <213> Artificial Sequence
<220>
   <223> Description of Artificial Sequence:Synthetic DNA
<220>
   <221> CDS
   <222> (1)..(378)
<400> 81
   atg gat ttt caa gtg cag att ttc agc ttc ctg cta atc agt gcc tca  48
   Met Asp Phe Gln Val Gln Ile Phe Ser Phe Leu Leu Ile Ser Ala Ser
   -22     -20                 -15                 -10
   gtc ata ata tcc aga gga gat atc cag atg acc cag tct cca tcc tcc  96
   Val Ile Ile Ser Arg Gly Asp Ile Gln Met Thr Gln Ser Pro Ser Ser
       -5                  -1   1               5                  10
   ctg tct gca tct gta gga gag gag gtc acc atc act tgt agt gcc agc 144
   Leu Ser Ala Ser Val Gly Glu Glu Val Thr Ile Thr Cys Ser Ala Ser
                   15                  20                  25
   tcg agt gta agt tac atg cac tgg tat cag cag aaa cca ggg aaa gcc 192
   Ser Ser Val Ser Tyr Met His Trp Tyr Gln Gln Lys Pro Gly Lys Ala
               30                  35                  40
   cct aag ctt ctg atc tat aga aca tcc aac ctg gct tct ggg gtc cca 240
   Pro Lys Leu Leu Ile Tyr Arg Thr Ser Asn Leu Ala Ser Gly Val Pro
           45                  50                  55
   tca agg ttc agc ggc agt gga tct ggg aca ttt tat act ctc acc atc 288
   Ser Arg Phe Ser Gly Ser Gly Ser Gly Thr Phe Tyr Thr Leu Thr Ile
        60                  65                  70
   agc agc ctg cag cct gaa gat ttt gca act tat tac tgt cat cag tgg 336
   Ser Ser Leu Gln Pro Glu Asp Phe Ala Thr Tyr Tyr Cys His Gln Trp
    75                  80                  85                  90
   agt atg tac acg ttc ggc caa ggg acc aag gta gag atc aaa c       379
   Ser Met Tyr Thr Phe Gly Gln Gly Thr Lys Val Glu Ile Lys
                    95                 100
<210> 82
   <211> 138
   <212> PRT
   <213> Mus musculus
<220>
   <223>
<400> 82
   Met Glu Trp Asn Trp Val Val Leu Phe Leu Leu Ser Leu Thr Ala Gly
   -19             -15                 -10                  -5
   Val Tyr Ala Gln Gly Gln Met Gln Gln Ser Gly Ala Glu Leu Val Lys
            -1   1               5                  10
   Pro Gly Ala Ser Val Lys Leu Ser Cys Lys Pro Ser Gly Phe Thr Phe
        15                  20                  25
   Ser Ser Asn Tyr Ile Ser Trp Leu Lys Gln Lys Pro Gly Gln Ser Leu
    30                  35                  40                  45
   Glu Trp Ile Ala Trp Ile Tyr Ala Gly Thr Gly Asp Ala Ser Tyr Asn
                    50                  55                  60
   Gln Lys Phe Thr Ala Lys Ala His Val Thr Val Asp Thr Ser Ser Ser
                65                  70                  75
   Thr Ala Tyr Met Gln Leu Ser Ser Leu Thr Thr Glu Asp Ser Ala Ile
            80                  85                  90
   Tyr Tyr Cys Ala Arg His Gly Gly Asp Gly Tyr Trp Phe Ala Tyr Trp
        95                 100                 105
   Gly Gln Gly Thr Leu Val Thr Val Ser Ala
   110                 115             119
<210> 83
   <211> 128
   <212> PRT
   <213> Mus musculus
<220>
   <223>
<400> 83
   Met Asp Phe Gln Val Gln Ile Phe Ser Phe Leu Leu Ile Ser Ala Ser
   -22 -20 -15 -10
   Val Ile Ile Ser Arg Gly Gln Leu Val Leu Thr Gln Ser Pro Ala Ile
   -5 -1 1 5 10
   Met Ser Ala Ser Gln Gly Glu Lys Val Thr Met Thr Cys Ser Ala Ser
   15 20 25
   Ser Ser Val Ser Tyr Met His Trp Tyr Gln Gln Lys Ser Gly Thr Ser
   30 35 40
   Pro Lys Arg Trp Ile Tyr Asp Thr Ser Lys Leu Pro Ser Gly Val Pro
   45 50 55
   Ala Arg Phe Ser Gly Ser Gly Ser Gly Thr Ser Tyr Ser Leu Thr Ile
   60 65 70
   Ser Ser Met Glu Ala Glu Asp Ala Ala Thr Tyr Tyr Cys Gln Gln Trp
   75 80 85 90
   Ser Ser Asn Pro Pro Thr Phe Gly Ala Gly Thr Lys Leu Glu Leu Lys
   95 100 105 106
<210> 84
   <211> 136
   <212> PRT
   <213> Mus musculus
<220>
   <223>
<400> 84
   Met Gly Phe Ser Arg Ile Phe Leu Phe Leu Leu Ser Val Thr Thr Gly
   -19 -15 -10 -5
   Val His Ser Gln Ala Phe Leu Gln Gln Ser Gly Ala Glu Leu Val Arg
   -1 1 5 10
   Pro Gly Ala Ser Val Lys Met Ser Cys Lys Ala Ser Gly Tyr Thr Phe
   15 20 25
   Ile Asn Tyr Asn Met His Trp Val Lys Gln Thr Pro Arg Gln Gly Leu
   30 35 40 45
   Glu Trp Ile Gly Ala Ile Phe Pro Gly Asn Gly Phe Thr Ser Tyr Asn
   50 55 60
   Gln Lys Phe Lys Gly Lys Ala Thr Leu Thr Val Asp Lys Ser Ser Ser
   65 70 75
   Thr Val Tyr Met Gln Leu Arg Ser Leu Thr Ser Glu Asp Ser Ala Val
   80 85 90
   Tyr Phe Cys Ala Arg Asp Gly Asp Tyr Tyr Phe Asp Tyr Trp Gly Gln
   95 100 105
   Gly Thr Thr Leu Thr Val Ser Ser
   110 115 117
<210> 85
   <211> 126
   <212> PRT
   <213> Mus musculus
<220>
   <223>
<400> 85
   Met Asp Phe Gln Val Gln Ile Phe Ser Phe Leu Leu Ile Ser Ala Ser
   -22 -20 -15 -10
   Val Ile Met Ser Arg Gly Gln Ile Val Leu Thr Gln Ser Pro Ala Ile
   -5 -1 1 5 10
   Met Ser Ala Ser Leu Gly Glu Glu Ile Thr Leu Thr Cys Ser Ala Ser
   15 20 25
   Ser Ser Val Ser Tyr Met His Trp Tyr Gln Gln Lys Ser Gly Thr Ser
   30 35 40
   Pro Lys Leu Leu Ile Tyr Arg Thr Ser Asn Leu Ala Ser Gly Val Pro
   45 50 55
   Phe Arg Phe Ser Gly Ser Gly Ser Gly Thr Phe Tyr Ser Leu Thr Ile
   60 65 70
   Ser Ser Val Glu Ala Glu Asp Ala Ala Asp Tyr Tyr Cys His Gln Trp
   75 80 85 90
   Ser Met Tyr Thr Phe Gly Gly Gly Thr Lys Leu Glu Ile Lys
   95 100 104
<210> 86
   <211> 140
   <212> PRT
   <213> Artificial Sequence
<220>
   <223> Description of Artificial Sequence: Humanized antibody
<400> 86
<210> 87
   <211> 136
   <212> PRT
   <213> Artificial Sequence
<220>
   <223> Description of Artificial Sequence: Humanized antibody
<400> 87
<210> 88
   <211> 128
   <212> PRT
   <213> Artificial Sequence
<220>
   <223> Description of Artificial Sequence: Humanized antibody
<400> 88
   Met Asp Phe Gln Val Gln Ile Phe Ser Phe Leu Leu Ile Ser Ala Ser
   -22     -20                 -15                 -10
   Val Ile Ile Ser Arg Gly Asp Ile Gln Met Thr Gln Ser Pro Ser Ser
       -5              -1   1               5                  10
   Leu Ser Ala Ser Val Gly Asp Arg Val Thr Ile Thr Cys Ser Ala Ser
                   15                  20                  25
   Ser Ser Val Ser Tyr Met His Trp Tyr Gln Gln Lys Pro Gly Lys Ala
               30                  35                  40
   Pro Lys Leu Leu Ile Tyr Asp Thr Ser Lys Leu Pro Ser Gly Val Pro
           45                  50                  55
   Ser Arg Phe Ser Gly Ser Gly Ser Gly Thr Asp Phe Thr Leu Thr Ile
       60                  65                  70
   Ser Ser Leu Gln Pro Glu Asp Phe Ala Thr Tyr Tyr Cys Gln Gln Trp
   75                  80                  85                  90
   Ser Ser Asn Pro Pro Thr Phe Gly Gln Gly Thr Lys Val Glu Ile Lys
                   95                  100                 105 106
<210> 89
   <211> 126
   <212> PRT
   <213> Artificial Sequence
<220>
   <223> Description of Artificial Sequence: Humanized antibody
<400> 89
   Met Asp Phe Gln Val Gln Ile Phe Ser Phe Leu Leu Ile Ser Ala Ser
   -22     -20                 -15                 -10
   Val Ile Met Ser Arg Gly Asp Ile Val Met Thr Gln Ser Pro Asp Ser
       -5              -1   1               5                  10
   Leu Ala Val Ser Leu Gly Glu Arg Ala Thr Ile Asn Cys Ser Ala Ser
                   15                  20                  25
   Ser Ser Val Ser Tyr Met His Trp Tyr Gln Gln Lys Pro Gly Gln Pro
               30                  35                  40
   Pro Lys Leu Leu Ile Tyr Arg Thr Ser Asn Leu Ala Ser Gly Val Pro
           45                  50                  55
   Asp Arg Phe Ser Gly Ser Gly Ser Gly Thr Asp Phe Thr Leu Thr Ile
       60                  65                  70
   Ser Ser Leu Gln Ala Glu Asp Val Ala Val Tyr Tyr Cys His Gln Trp
   75 80 85 90
   Ser Met Tyr Thr Phe Gly Gln Gly Thr Lys Val Glu Ile Lys
   95 100 104
<210> 90
   <211> 126
   <212> PRT
   <213> Artificial Sequence
<220>
   <223> Description of Artificial Sequence: Humanized antibody
<400> 90
   Met Asp Phe Gln Val Gln Ile Phe Ser Phe Leu Leu Ile Ser Ala Ser
   -22 -20 -15 -10
   Val Ile Ile Ser Arg Gly Asp Ile Gln Met Thr Gln Ser Pro Ser Ser
   -5 -1 1 5 10
   Leu Ser Ala Ser Val Gly Asp Arg Val Thr Ile Thr Cys Ser Ala Ser
   15 20 25
   Ser Ser Val Ser Tyr Met His Trp Tyr Gln Gln Lys Pro Gly Lys Ala
   30 35 40
   Pro Lys Leu Leu Ile Tyr Arg Thr Ser Asn Leu Ala Ser Gly Val Pro
   45 50 55
   Ser Arg Phe Ser Gly Ser Gly Ser Gly Thr Asp Phe Thr Leu Thr Ile
   60 65 70
   Ser Ser Leu Gln Pro Glu Asp Phe Ala Thr Tyr Tyr Cys His Gln Trp
   75 80 85 90
   Ser Met Tyr Thr Phe Gly Gln Gly Thr Lys Val Glu Ile Lys
   95 100 104
<210> 91
   <211> 136
   <212> PRT
   <213> Artificial Sequence
<220>
   <223> Description of Artificial Sequence: Humanized antibody
<400> 91
   Met Gly Phe Ser Arg Ile Phe Leu Phe Leu Leu Ser Val Thr Thr Gly
   -19 -15 -10 -5
   Val His Ser Gln Val Gln Leu Val Gln Ser Gly Ala Glu Val Lys Lys
   -1 1 5 10
   Pro Gly Ala Ser Val Lys Val Ser Cys Lys Ala Ser Gly Tyr Thr Phe
   15 20 25
   Ile Asn Tyr Asn Met His Trp Val Arg Gln Ala Pro Gly Gln Gly Leu
   30 35 40 45
   Glu Trp Met Gly Ala Ile Phe Pro Gly Asn Gly Phe Thr Ser Tyr Asn
   50 55 60
   Gln Lys Phe Lys Gly Arg Val Thr Ile Thr Val Asp Lys Ser Thr Ser
   65 70 75
   Thr Ala Tyr Met Gln Leu Arg Ser Leu Arg Ser Glu Asp Thr Ala Val
   80 85 90
   Tyr Phe Cys Ala Arg Asp Gly Asp Tyr Tyr Phe Asp Tyr Trp Gly Gln
   95 100 105
   Gly Thr Leu Val Thr Val Ser Ser
   110 115 117
<210> 92
   <211> 126
   <212> PRT
   <213> Artificial Sequence
<220>
   <223> Description of Artificial Sequence: Humanized antibody
<400> 92
   Met Asp Phe Gln Val Gln Ile Phe Ser Phe Leu Leu Ile Ser Ala Ser
   -22 -20 -15 -10
   Val Ile Ile Ser Arg Gly Asp Ile Gln Met Thr Gln Ser Pro Ser Ser
   -5 -1 1 5 10
   Leu Ser Ala Ser Val Gly Glu Glu Val Thr Ile Thr Cys Ser Ala Ser
   15 20 25
   Ser Ser Val Ser Tyr Met His Trp Tyr Gln Gln Lys Pro Gly Lys Ala
   30 35 40
   Pro Lys Leu Leu Ile Tyr Arg Thr Ser Asn Leu Ala Ser Gly Val Pro
   45 50 55
   Ser Arg Phe Ser Gly Ser Gly Ser Gly Thr Phe Tyr Thr Leu Thr Ile
   60 65 70
   Ser Ser Leu Gln Pro Glu Asp Phe Ala Thr Tyr Tyr Cys His Gln Trp
   75 80 85 90
   Ser Met Tyr Thr Phe Gly Gln Gly Thr Lys Val Glu Ile Lys
   95 100 104